# EUROPEAN PATENT SPECIFICATION

(11) **EP 4 637 704 B1**
(45) Date of publication and mention of the grant of the patent: **18.11.2026**
(21) Application number: 23837310.4
(22) Date of filing: 21.12.2023
(51) Int. Cl.: A61K 8/898, A61Q 5/06, A61K 8/891, A61K 8/40, A61K 8/84

(54) **PROCESS FOR DYEING KERATIN HAIR FIBERS COMPRISING THE APPLICATION OF A (POLY)CARBODIIMIDE COMPOUND, A COMPOUND CONTAINING AT LEAST ONE CARBOXYLIC ACID GROUP, AN AMINO SILICONE AND A COLOURING AGENT**
VERFAHREN ZUM FÄRBEN VON KERATIN-HAARFASERN, UMFASSEND DURCH AUFBRINGEN EINER (POLY)CARBODIIMIDVERBINDUNG, EINER VERBINDUNG, DIE MINDESTENS EINE CARBONSÄUREGRUPPE, EIN AMINO-SILIKON UND EINEN FARBSTOFF ENTHÄLT.
PROCÉDÉ DE TEINTURE DES FIBRES CAPILLAIRES KÉRATINIQUES COMPRENANT L'APPLICATION D'UN COMPOSÉ (POLY)CARBODIIMIDE, D'UN COMPOSÉ CONTENANT AU MOINS UN GROUPE ACIDE CARBOXYLIQUE, D'UNE SILICONE AMINÉE ET D'UN AGENT COLORANT.

(30) Priority: 21.12.2022 FR 2214064
(43) Date of publication of application: 29.10.2025
(73) Proprietor: L'OREAL, 75008 Paris (FR)
(72) Inventor: DELOSTAL, Charlotte, 93400 SAINT OUEN (FR); DJAIDI, Victtoria, 93400 SAINT OUEN (FR); LIARD, Alexis, 93400 ST OUEN (FR)
(74) Representative: Casalonga
(86) International application number: PCT/EP2023/087285
(87) International publication number: WO 2024/133696

(56) References cited:
- WO-A1-2021/121882
- WO-A1-2022/189570
- WO-A1-2022/258494
- US-A1- 2019 001 163

## Description

The present invention relates to a process for dyeing keratin hair fibers, comprising the application to the keratin hair fibers of at least one composition C comprising at least one (poly)carbodiimide compound, at least one compound containing at least one carboxylic acid group, at least one amino silicone of formula (XIII), and at least one colouring agent chosen from pigments, direct dyes and mixtures thereof.

### Technical field

In the field of dyeing keratin hair fibers, in particular human keratin fibres, it is already known practice to dye keratin hair fibers via various techniques using direct dyes or pigments for non-permanent colouring, or dye precursors for permanent colouring. There are essentially three types of process for dyeing the keratin hair fibers:
a) "permanent" dyeing, the function of which is to afford a substantial modification to the natural colour and which uses oxidation dyes which penetrate into the keratin hair fibers fibre and forms the dye via an oxidative condensation process;
b) non-permanent, semi-permanent or direct dyeing, which does not use the oxidative condensation process and withstands four or five shampoo washes; it consists in dyeing keratin fibres with dye compositions containing direct dyes;
c) temporary dyeing, which gives rise to a modification of the natural colour of the keratin hair fibers that remains from one shampoo wash to the next, and which serves to enhance or correct a shade that has already been obtained. It may also be likened to a "makeup" process.

Another dyeing method consists in using pigments. Specifically, the use of pigment on the surface of keratin fibres generally makes it possible to obtain colourings that are visible on dark keratin hair fibers, since the surface pigment masks the natural colour of the fibre.

However, the colourings obtained via this dyeing method have the drawback of having poor resistance to shampoo washing and also to external agents such as sebum, perspiration, brushing and/or rubbing.

Furthermore, temporary keratin hair fibers dye compositions may lead to cosmetic properties that are not entirely satisfactory, more particularly keratin hair fibers conditioning properties, notably in terms of the suppleness and the smooth feel of the keratin hair fibers WO 2022/258494 and WO 2022/189570 disclose a process for colouring the hair, in particular the hair, comprising the application of a composition A and of a composition B to hair keratin fibres, in which: composition A comprises at least one (poly)carbodiimide compound, and composition B comprises at least one compound bearing a carboxylic acid group; composition A and/or composition B comprising at least one colouring agent chosen from pigments, direct dyes and mixtures thereof; and at least one non-silicone fatty substance; in which composition A and composition B are applied simultaneously or sequentially to the hair keratin fibres.

Composition A also contains amodimethicone-trideceth-5-trideceth-10 (Belsil ADM LOG 1).

The need thus remains for a process for dyeing keratin hair fibers which has the advantage of producing a uniform coloured coating on the keratin hair fibers with improved keratin hair fibers conditioning properties, notably in terms of the suppleness and the smooth feel of the keratin hair fibers, while at the same time forming a coating that is resistant to shampoo washing and to the various stresses to which the keratin hair fibers may be subjected, such as brushing and/or rubbing.

Thus, the aim of the present invention is to develop a process for dyeing keratin hair fibers which has the advantage of producing a uniform coloured coating on the keratin hair fibers with improved keratin hair fibers conditioning properties, notably in terms of the suppleness and the smooth feel of the keratin hair fibers, while at the same time forming a coating that is resistant to shampoo washing and to the various stresses to which the keratin hair fibers may be subjected, such as brushing and/or rubbing.

### Disclosure of the invention

One subject of the present invention is thus a process for dyeing keratin hair fibers comprising the application to the keratin hair fibers of at least one composition C comprising:
a) at least one (poly)carbodiimide compound;
b) at least one compound containing at least one carboxylic acid group;
c) at least one amino silicone of formula (XIII) as described below; and
d) at least one colouring agent chosen from pigments, direct dyes and mixtures thereof.

By virtue of the process for dyeing keratin hair fibers according to the invention, coloured coatings are obtained on the keratin hair fibers, which make it possible to obtain visible colouring on all keratin hair fibers types and to obtain excellent keratin hair fibers conditioning properties, notably in terms of the suppleness and the smooth feel of the keratin hair fibers.

The coloured coating obtained is resistant shampoo washing and also to the external stresses to which the keratin hair fibers may be subjected, such as blow-drying and perspiration.

For the purposes of the present invention, the term *"colouring that is resistant to shampoo washing"* means that the colouring obtained persists after at least one shampoo wash, preferably after three shampoo washes, more preferentially after five shampoo washes. The term *"keratin hair fibers"* particularly means the hair keratin fibers or the hair. In other words, the expressions "keratin hair fibers", "hair keratin fibers" and "hair" are equivalent in the continuation of the description.

For the purposes of the present invention, the term "keratin hair fibers" means head keratin hair fibers. This term does not correspond to bodily hairs, the eyebrows or the eyelashes. The term "*at least one"* means one or more.

Unless otherwise indicated, the limits of a range of values are included in that range, notably in the expressions "between" and "ranging from ... to ...".

The invention is not limited to the illustrated examples. The characteristics of the various examples may notably be combined within variants which are not illustrated.

For the purposes of the present invention and unless otherwise indicated:
- an *"alkyl"* radical denotes a linear or branched saturated radical containing, for example, from 1 to 20 carbon atoms;
- an *"aminoalkyl"* radical denotes an alkyl radical as defined previously, said alkyl radical comprising an NH₂ group;
- a *"hydroxyalkyl"* radical denotes an alkyl radical as defined previously, said alkyl radical comprising an OH group;
- an *"alkylene"* radical denotes a linear or branched divalent saturated C₂-C₄ hydrocarbon-based group such as methylene, ethylene or propylene;
- a *"cycloalkyl"* or "*alicycloalkyl"* radical denotes a cyclic saturated monocyclic or bicyclic, preferably monocyclic, hydrocarbon-based group comprising from 1 to 3 rings, preferably 2 rings, and comprising from 3 to 24 carbon atoms, in particular comprising from 3 to 20 carbon atoms, more particularly from 3 to 13 carbon atoms, even more particularly from 3 to 12 carbon atoms, preferably between 5 and 10 carbon atoms, such as cyclopropyl, cyclopentyl, cyclohexyl, cycloheptyl or norbornyl, in particular cyclopropyl, cyclopentyl or cyclohexyl, it being understood that the cycloalkyl radical may be substituted with one or more (C₁-C₄)alkyl groups such as methyl; preferably, the cycloalkyl radical is then an isobornyl group;
- a *"cycloalkylene"* radical denotes a divalent cycloalkyl group with *"cycloalkyl"* as defined previously, preferably of C₃-C₁₂;
- an *"aryl"* radical is a monocyclic, bicyclic or tricyclic, fused or non-fused, unsaturated and aromatic hydrocarbon-based cyclic radical, comprising from 6 to 14 carbon atoms, preferably between 6 and 12 carbon atoms; preferably, the aryl group comprises 1 ring of 6 carbon atoms such as phenyl, naphthyl, anthryl, phenanthryl and biphenyl, it being understood that the aryl radical may be substituted with one or more (C₁-C₄)alkyl groups such as methyl, preferably tolyl, xylyl, or methylnaphthyl; preferably, the aryl group represents phenyl;
- an *"arylene"* radical is a divalent aryl radical with *"aryl"* as defined previously; preferably, arylene represents phenylene;
- a *"heterocyclic"* radical denotes a saturated or unsaturated, non-aromatic or aromatic, monocyclic or polycyclic hydrocarbon-based radical, comprising one or more heteroatoms, preferably from 1 to 5 atoms chosen from O, S or N, including from 3 to 20 ring members, preferably between 5 and 10 ring members, such as imidazolyl, pyrrolyl and furanyl;
- a *"heterocycloalkylene"* radical is a divalent heterocyclic group with *"heterocyclic"* as defined previously;
- an *"aryloxy"* radical denotes an aryl-oxy radical with *"aryl"* as defined previously;
- an *"alkoxy"* radical denotes an alkyl-oxy radical with *"alkyl"* as defined previously;
- an *"acyloxy"* radical denotes an ester radical R-C(O)-O- with R being an alkyl group as defined previously;
- a "reactive" group is a group that is capable of forming a covalent bond with another identical or different group, by chemical reaction.

Unless otherwise indicated, when compounds are mentioned in the present patent application, this also includes the optical isomers thereof, the geometrical isomers thereof, the tautomers thereof or the salts thereof, alone or as a mixture.

### Detailed description of the invention

### Polycarbodiimide compound

Composition C used in the context of the process according to the invention comprises at least one (poly)carbodiimide compound.

The composition may comprise at least two different (poly)carbodiimide compounds, present as a mixture in the composition.

The term *"(poly)carbodiimide compound"* means a compound comprising one or more carbodiimide groups, preferably at least two carbodiimide groups, more preferentially at least three carbodiimide groups; in particular, the number of carbodiimide groups does not exceed 200, preferably 150, more preferentially 100.

The term *"carbodiimide group"* means a divalent linear triatomic fraction of general formula -(N=C=N)-.

The (poly)carbodiimide compound(s) according to the invention may optionally comprise in their structure one or more reactive groups different from carbodiimide groups, chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

The reactive group(s) other than the carbodiimide groups may be side or end groups. Preferably, the (poly)carbodiimide compound(s) comprise one or more end groups different from carbodiimide groups, preferably one or more end groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

According to a particular embodiment, the (poly)carbodiimide compound is chosen from the compounds of formula (I) below: 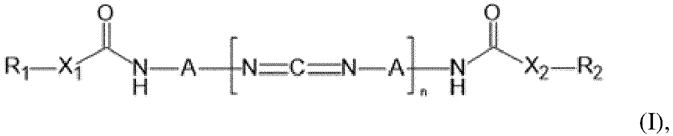 in which:
- X₁ and X₂ independently represent an oxygen atom O, a sulfur atom S or an NH group;
- R₁ and R₂ independently represent a group chosen from a hydrocarbon-based radical, preferably alkyl, optionally interrupted with one or more heteroatoms, a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups, and a hydrocarbon-based radical, preferably alkyl, optionally interrupted with one or more heteroatoms and with one or more groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- n denotes an integer ranging from 1 to 1000; and
- A is a monomer chosen from the compounds below:

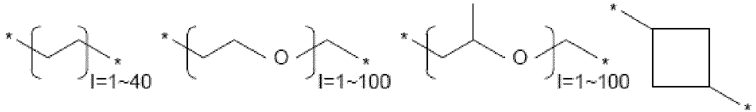

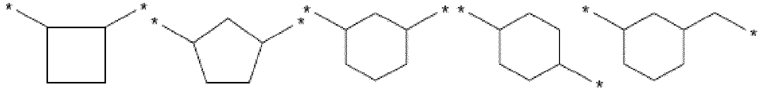

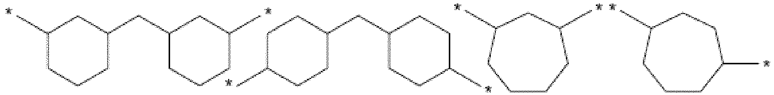

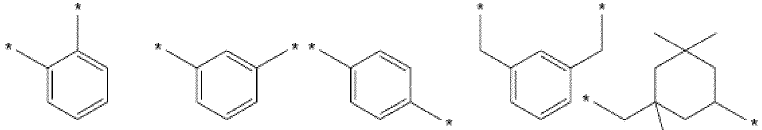

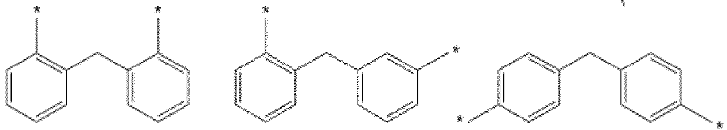

According to another embodiment, the (poly)carbodiimide compound is chosen from the compounds of formula (I') below: 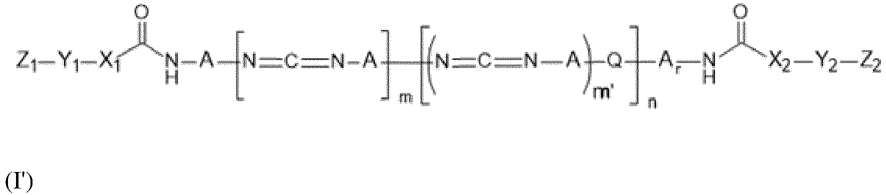 in which:
- X₁ and X₂ independently represent an oxygen atom O, a sulfur atom S or an NH group;
- Y₁ and Y₂ independently represent a divalent organic radical chosen from a saturated C₁ to C₃₆ aliphatic group or a C₆ to C₂₄ aromatic or alkylaromatic group, the aliphatic or aromatic group optionally comprising one or more non-pendent heteroatoms, such as a nitrogen atom, an oxygen atom, a sulfur atom, or combinations thereof;
- Z₁ and Z₂ independently represent a reactive end group or an inert end group;
- as inert end group, Z₁ and Z₂ may represent, independently, a saturated, linear or branched or cyclic C₁ to C₅₀ aliphatic group, or a C₆ to C₁₈ aromatic group, said aliphatic and aromatic groups optionally comprising from 1 to 10 heteroatoms chosen from nitrogen, oxygen, sulfur and combinations thereof, and the aliphatic or aromatic group may be partially or totally fluorinated; in this variant, Z₁ and Z₂ comprise a bonding group CG connecting Z₁ to Y₁ and Z₂ to Y₂, the group CG possibly being a single covalent bond, a saturated C-C bond, an unsaturated covalent C-C bond, an amide group, an ester group, a carbonate group, a thioester group, an ether group, a urethane group, a thiourethane group or a urea group;
- as reactive end group, Z₁ and Z₂ may be chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- Q represents an organopolymer or an organooligomer comprising repeating units of saturated, linear or branched or cyclic aliphatic groups, or of aromatic groups or alkylaromatic groups, coupled via carbonate, ester, ether, amide, urethane or urea repeating bonds or combinations thereof;
- A represents a divalent aliphatic, aromatic, alkylaromatic or linear, saturated, branched or cyclic radical containing from 2 to 30 carbon atoms, which may optionally comprise one or more non-pendent heteroatoms such as a nitrogen atom, an oxygen atom, a sulfur atom, or combinations thereof, in the aliphatic chain or the aromatic chain;
- r denotes an integer equal to 0 or 1;
- m denotes an integer ranging from 0 to 1000, preferably equal to 0 or 1;
- m' denotes an integer ranging from 0 to 1000, preferably equal to 0 or 1;
- n denotes an integer ranging from 0 to 1000, preferably equal to 0 or 1, with m + (m'*n) ≥ 2.

Preferably, Z₁ and Z₂ independently represent a reactive end group; more preferentially, Z₁ and Z₂ independently represent a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

Such (poly)carbodiimide compounds are sold, for example, by the company Stahl B.V. under the name Permutex XR, or under the name RelcaLink10 or under the name Picassian XL and by the company Nisshinbo under the name Carbodilite with the series V-02, V-02-L2, SV-02, E-02, V-10, SW-12G, E-03A, E-04DG-T, E-05, V-04, V-02B, V-04PF, V-05.

Preferably, the (poly)carbodiimide compound(s) are chosen from the compounds of formula (II) below: 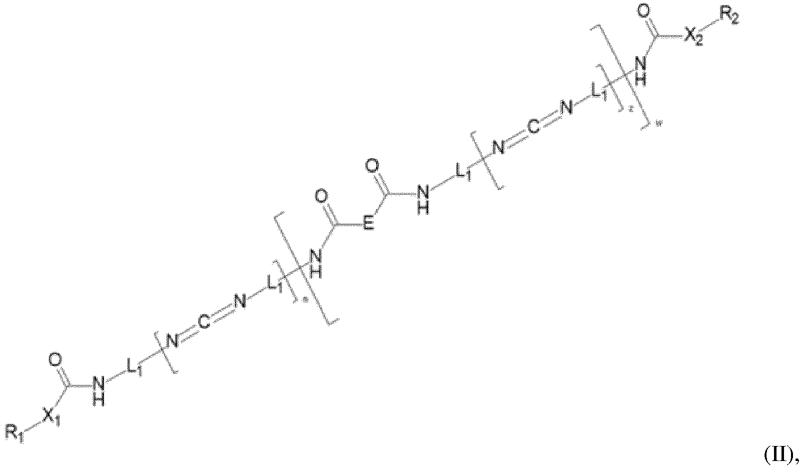 in which:
- X₁ and X₂ independently represent an oxygen atom O, a sulfur atom S or an NH group;
- R₁ and R₂ independently represent a hydrocarbon-based radical optionally interrupted with one or more heteroatoms;
- n and z denote an integer ranging from 1 to 20, with n+z ≥ 2 and w denotes an integer ranging from 1 to 3;
- L₁ independently represents a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:
   -O-R₃-O-; -S-R₄-S-; -R₅-N(R₆)-R₄-N(R₆)-R₅-;
   in which R₃ and R₄ independently represent a divalent hydrocarbon-based radical optionally interrupted with one or more heteroatoms;
   -
   - R₅ independently represents a covalent bond or a saturated divalent hydrocarbon-based radical, optionally interrupted with one or more heteroatoms;
   - R₆ independently represents a hydrogen atom or a hydrocarbon-based radical, optionally interrupted with one or more heteroatoms.

The term *"hydrocarbon-based radical"* means a saturated or unsaturated, linear or branched radical containing from 1 to 300 carbon atoms, preferably from 1 to 250 carbon atoms, more preferentially from 1 to 200 carbon atoms. Preferably, the hydrocarbon-based radical is a saturated linear radical.

The hydrocarbon-based radical may comprise one or more cyclic groups.

The hydrocarbon-based radical may be interrupted with one or more heteroatoms, in particular chosen from O, S or N and/or substituted with one or more cations, anions or zwitterions or cationic groups such as ammonium, anionic groups such as carboxylate, or zwitterionic groups, and/or comprising a metal ion which may be incorporated in the form of a salt.

The term "*heteroatom(s)"* means an oxygen O, sulfur S or nitrogen N atom, and also halogen atoms such as Cl, F, Br and I. If the heteroatom is included in the chain of the hydrocarbon-based radical, the heteroatom is preferably chosen from oxygen O, sulfur S or nitrogen N atoms.

Preferably, X₁ and X₂ independently represent an oxygen atom.

Preferably, R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof.

In a preferred embodiment, R₁ and R₂ are independently chosen from groups (i) to (iv) below:
(i) the compound of formula (III) below:

   R₇-O-C(O)-C(R₈)(H)- (III),

   in which R₇ represents a C₁-C₃ alkyl group and R₈ represents a hydrogen atom or a C₁-C₃ alkyl group; preferably, R₇ is a methyl and R₈ is a hydrogen atom or a methyl.
(ii) the compound of formula (IV) below:

   R₉-[O-CH₂-C(H)(R₁₀)]ₚ- (IV),

   in which R₉ represents a C₁-C₄ alkyl group, R₁₀ represents a hydrogen atom or a C₁-C₄ alkyl group and p denotes an integer ranging from 1 to 3; preferably, R₉ is a methyl, ethyl or butyl, R₁₀ is a hydrogen atom or a methyl and p is equal to 1.
(iii) the compound of formula (V) below:

   (R₁₁)₂N-CH₂-C(H)(R₁₂)- (V),

   in which R₁₁ represents a C₁-C₄ alkyl group and R₁₂ represents a hydrogen atom or a C₁-C₄ alkyl group; preferably, R₁₁ is a methyl, ethyl or butyl and R₁₂ is a hydrogen atom or a methyl.
(iv) the compound of formula (VI) below:

   R₁₃-[O-CH₂-C(H)(R₁₄)]_{q}- (VI),

   in which R₁₃ represents a C₁-C₄ alkyl group or a phenyl, R₁₄ represents a hydrogen atom or a C₁-C₄ alkyl group and q denotes an integer ranging from 4 to 30; preferably, R₁₃ is a methyl, ethyl or butyl and R₁₄ is a hydrogen atom or a methyl.

Preferably, R₁ and R₂ independently represent a compound of formula (VI) in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30.

According to an alternative embodiment, R₁ and R₂ are different and one of the radicals R₁ or R₂ represents a compound of formula (IV) as described above and the other radical R₁ or R₂ represents a compound of formula (VI) as described above.

Preferably, in formula (IV), R₉ is a methyl, ethyl or butyl and R₁₀ is a hydrogen atom or a methyl and p is equal to 1.

Preferably, in formula (VI), R₁₃ is a methyl, ethyl or butyl and R₁₄ is a hydrogen atom or a methyl and q denotes an integer ranging from 4 to 30.

According to another alternative embodiment, R₁ and R₂ are identical and represent a compound of formula (VI) in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30.

Preferably, n denotes an integer ranging from 1 to 20, more preferentially from 2 to 20.

Preferably, z denotes an integer ranging from 1 to 20, more preferentially from 2 to 20.

Preferably, w is equal to 1.

Preferably, w is equal to 1, n+z denotes an integer ranging from 4 to 10.

Preferably, L₁ is chosen from a C₁-C₁₈ divalent aliphatic hydrocarbon-based radical such as methylene, ethylene and propylene, a C₃-C₁₅ cycloalkylene radical such as cyclopentylene, cycloheptylene and cyclohexylene, a C₃-C₁₂ heterocycloalkylene group such as imidazolene, pyrrolene and furanylene, or a C₆-C₁₄ arylene group such as phenylene, and mixtures thereof.

For example, L₁ may be chosen from a radical derived from tolylene diisocyanate, hexamethylene diisocyanate, xylylene diisocyanate, 2,2,4-trimethylhexamethylene diisocyanate, 1,12-dodecane diisocyanate, norbornane diisocyanate, 2,4-bis(8-isocyanatooctyl)-1,3-dioctylcyclobutane, 4,4'-dicyclohexylmethane diisocyanate, tetramethylxylylene diisocyanate, isophorone diisocyanate, 1,5-napththylene diisocyanate, 4,4'-diphenylmethane diisocyanate, 4,4'-diphenyldimethylmethane diisocyanate and phenylene diisocyanate, and mixtures thereof.

Preferably, L₁ is chosen from a C₃-C₁₅ cycloalkylene radical or a C₆-C₁₄ arylene group, and mixtures thereof, such as the compounds of formula (VII) below:

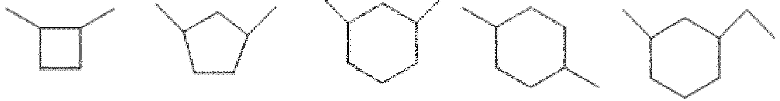

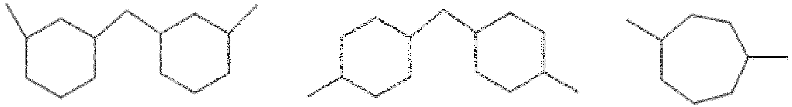

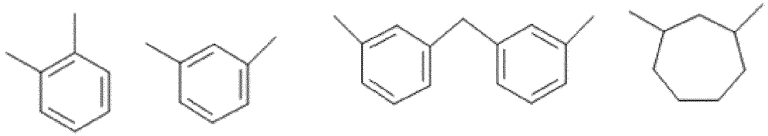

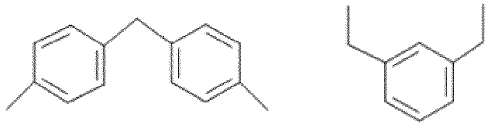

Preferably, L₁ is 4,4-dicyclohexylenemethane corresponding to formula (VIII) below:

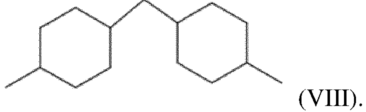

According to another embodiment, when L1 is a C₆-C₁₄ arylene group, L₁ is not the m-tetramethylxylylene radical represented by formula (IX) below:

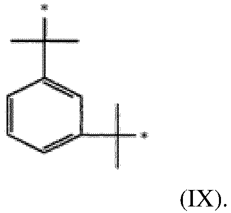

As indicated previously, E independently represents a group chosen from:
- O-R₃-O-; -S-R₄-S-; -R₅-N(R₆)-R₄-N(R₆)-R₅-;
   in which R₃ and R₄ independently represent a divalent hydrocarbon-based radical optionally interrupted with one or more heteroatoms;
- R₅ independently represents a covalent bond or a saturated divalent hydrocarbon-based radical, optionally interrupted with one or more heteroatoms; and
- R₆ independently represents a hydrogen atom or a hydrocarbon-based radical, optionally interrupted with one or more heteroatoms.

Preferably, R₃ and R₄ are independently chosen from a C₆-C₁₄ arylene radical such as phenylene, a C₃-C₁₂ cycloalkylene radical such as cyclopropylene and cyclobutylene, a linear or branched C₁-C₁₈ alkylene radical such as methylene and ethylene, optionally interrupted with one or more heteroatoms, and mixtures thereof.

More preferentially, R₃ and R₄ are independently chosen from a linear or branched C₁-C₁₈ alkylene radical such as methylene, butylene, propylene or ethylene, optionally interrupted with one or more heteroatoms.

Preferably, when R₅ is not a covalent bond, R₅ is chosen from a C₆-C₁₄ arylene radical such as phenylene, a C₃-C₁₂ cycloalkylene radical such as cyclopropylene and cyclobutylene, a linear or branched C₁-C₁₈ alkylene radical such as methylene and ethylene, optionally interrupted with one or more heteroatoms, and mixtures thereof.

Preferably, R₆ is chosen from a C₆-C₁₄ arylene radical such as phenylene, a C₃-C₁₂ cycloalkylene radical such as cyclopropylene and cyclobutylene, a linear or branched C₁-C₁₈ alkylene radical such as methylene and ethylene, optionally interrupted with one or more heteroatoms, and mixtures thereof.

Preferably, E represents a group -O-R₃-O- in which R₃ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

More preferentially, E represents a group -O-R₃-O- in which R₃ represents a linear or branched C₁-C₁₈ alkylene radical such as methylene, butylene, propylene or ethylene, optionally interrupted with one or more heteroatoms.

According to a particular embodiment, the (poly)carbodiimide compound is a copolymer derived from α-methylstyryl isocyanates of formula (X) below: 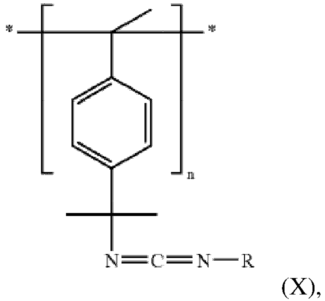
in which R independently represents an alkyl group containing from 1 to 24 carbon atoms, a cycloalkyl group containing from 3 to 24 carbon atoms or an aryl group containing from 6 to 24 carbon atoms, and
n denotes an integer ranging from 2 to 100.

In this embodiment, the term "alkyl group" is as defined previously.

In this embodiment, the term "cycloalkyl group" is as defined previously.

In this embodiment, n may denote an integer ranging from 2 to 50, preferably from 3 to 30 and even more preferentially from 5 to 10.

According to another particular embodiment, the (poly)carbodiimide compound is a compound of formula (XI) below: 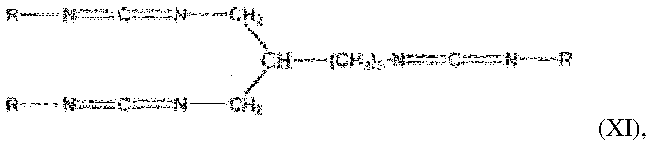 in which R independently represents an alkyl group containing from 1 to 24 carbon atoms, a cycloalkyl group containing from 3 to 24 carbon atoms or an aryl group containing from 6 to 24 carbon atoms.

The "alkyl group", the "cycloalkyl group" and the "aryl group" are as defined previously.

According to a preferred embodiment, the (poly)carbodiimide compound is chosen from the compounds of formula (I) or of formula (II) in which:
- X₁ and X₂ independently represent an oxygen atom;
- R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof, preferably monoalkyl ethers of (poly)alkylene glycol, in which a hydroxyl group has been removed, more preferentially the compound of formula (VI) as described previously in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom, and q denotes an integer ranging from 4 to 30;
- n and z, when they are present, denote an integer ranging from 1 to 20, with n+z ≥ 2 and w is equal to 1;
- L₁, when it is present, is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof, preferably a C₃-C₁₅ cycloalkylene radical;
- A, when it is present, is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof, preferably a C₃-C₁₅ cycloalkylene radical;
- E, when it is present, independently represents a group chosen from:
   - O-R₃-O-; -S-R₄-S-; -R₅-N(R₆)-R₄-N(R₆)-R₅-;
      in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
   - when R₅ is not a covalent bond, R₅, when it is present, is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
   - R₆, when it is present, is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

Preferably, the (poly)carbodiimide compound is chosen from the compounds of formula (II) in which:
- X₁ and X₂ independently represent an oxygen atom;
- R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof;
- n and z denote an integer ranging from 1 to 20, with n+z ≥ 2 and w is equal to 1;
- L₁ is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:
   - O-R₃-O-; -S-R₄-S-; -R₅-N(R₆)-R₄-N(R₆)-R₅-;
      in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
   - when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
   - R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

More preferentially, the (poly)carbodiimide compound is chosen from the compounds of formula (II) in which:
- X₁ and X₂ independently represent an oxygen atom;
- R₁ and R₂ are, independently, monoalkyl ethers of (poly)alkylene glycol, in which a hydroxyl group has been removed;
- n and z denote an integer ranging from 1 to 20, with n+z ≥ 2 and w is equal to 1;
- L₁ is a C₃-C₁₅ cycloalkylene radical;
- E independently represents a group chosen from:
   - O-R₃-O-; -S-R₄-S-; -R₅-N(R₆)-R₄-N(R₆)-R₅-;
      in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
   - when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
   - R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

Even more preferentially, the (poly)carbodiimide compound is chosen from the compounds of formula (II) in which:
- X₁ and X₂ independently represent an oxygen atom;
- R₁ and R₂ independently represent the compound of formula (VI) below:

   R₁₃-[O-CH₂-C(H)(R₁₄)]_{q}- (VI),

   in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane; and
- E represents a group -O-R₃-O- in which R₃ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

Even more preferentially, the (poly)carbodiimide compound is chosen from the compounds of formula (II) in which:
- X₁ and X₂ independently represent an oxygen atom;
- R₁ and R₂ independently represent the compound of formula (VI) below:

   R₁₃-[O-CH₂-C(H)(R₁₄)]_{q}- (VI)

   in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane, preferably 4,4-dicyclohexylenemethane; and
- E represents a group -O-R₃-O- in which R₃ represents a linear or branched C₁-C₁₈ alkylene radical such as methylene, propylene, butylene or ethylene, optionally interrupted with one or more heteroatoms.

According to a preferred embodiment, the (poly)carbodiimide compound is a compound of formula (XII) below: 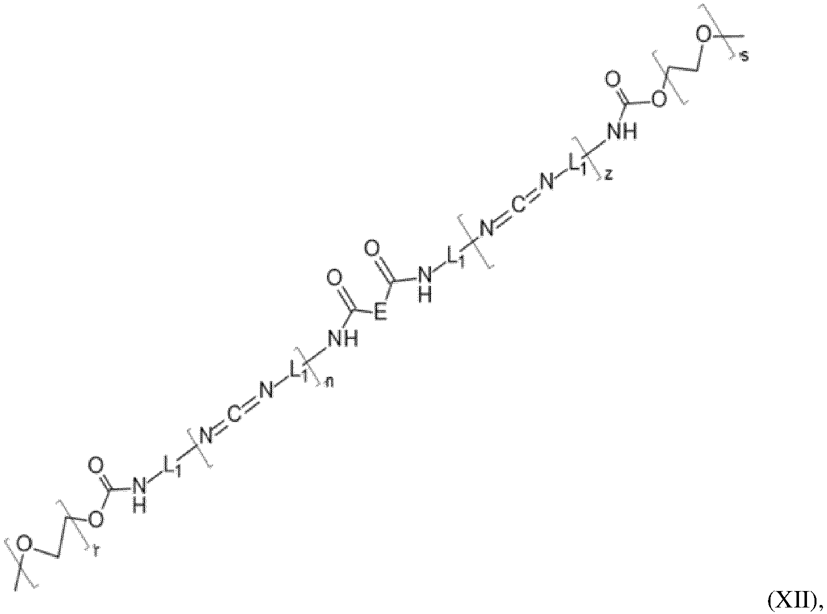 in which L1 is 4,4-dicyclohexylenemethane, n and z denote an integer ranging from 1 to 20, with n+z ranging from 4 to 10, E represents a group -O-R₃-O- in which R₃ represents a linear or branched C₁-C₁₈ alkylene radical such as methylene, propylene, butylene or ethylene, optionally interrupted with one or more heteroatoms, and r and s denote an integer ranging from 4 to 30.

Advantageously, the total amount of the (poly)carbodiimide compound(s) ranges from 0.01% to 20% by weight, preferably from 0.1% to 15% by weight, more preferentially from 0.2% to 10% by weight, even more preferentially from 0.5% to 8% and better still from 1% to 6% by weight, relative to the total weight of composition C.

### Compound containing at least one carboxylic acid group

Composition C used in the context of the dyeing process according to the invention comprises (ii) at least one compound containing at least one carboxylic acid group. Preferably, the compound(s) containing at least one carboxylic acid group are chosen from silicone compounds comprising at least one carboxylic group, polyurethanes, acrylic polymers and mixtures thereof.

### Polyurethanes and acrylic polymers:

According to a preferred embodiment, composition C comprises at least one compound containing at least one carboxylic acid group, chosen from polyurethanes, acrylic polymers and mixtures thereof.

Preferably, the compound(s) containing at least one carboxylic acid group are in the form of aqueous dispersions of particles of polymer(s) chosen from polyurethanes, acrylic polymers and mixtures thereof.

Preferably, composition C comprises at least one compound containing at least one carboxylic acid group in the form of aqueous dispersions of particles of polyurethanes, acrylic polymers and mixtures thereof.

The dispersion(s) may be simple dispersions in the aqueous medium of the cosmetic composition. As a particular case of dispersions, mention may be made of latices.

The aqueous dispersion(s) of polymer particles may be chosen from aqueous dispersions of polyurethane particles.

More particularly, the polyurethane(s) present in the aqueous dispersions used in the present invention result from the reaction of:
- a prepolymer of formula (A) below: 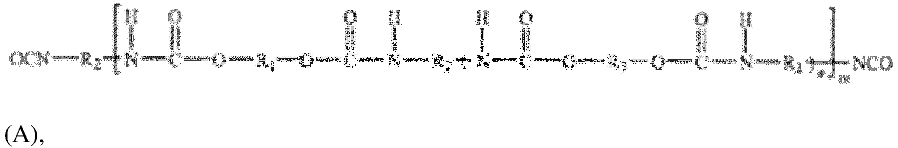 in which:
   - R₁ represents a divalent radical of a dihydroxylated compound;
   - R₂ represents a radical of an aliphatic or cycloaliphatic polyisocyanate;
   - R₃ represents a radical of a low molecular weight diol, optionally substituted with one or more ionic groups;
   - n represents an integer ranging from 1 to 5, and
   - m is greater than 1;
   - at least one chain extender according to formula (B) below:

      H₂N-R₄-NH₂ (B),

      in which R₄ represents an alkylene or alkylene oxide radical which is not substituted with one or more ionic or potentially ionic groups; and
   - at least one chain extender according to formula (C) below:

      H₂N-R₅-NH₂ (C),

      in which R₅ represents an alkylene radical substituted with one or more ionic or potentially ionic groups.

Among the dihydroxylated compounds that may be used according to the present invention, mention may be made notably of the compounds containing two hydroxyl groups and having a number-average molecular weight from about 700 to about 16 000, and preferably from about 750 to about 5000. As examples of dihydroxylated compounds of high molecular weight, mention may be made of polyol polyesters, polyol polyethers, polyhydroxylated polycarbonates, polyhydroxylated polyacetates, polyhydroxylated polyacrylates, polyhydroxylated amide polyesters, polyhydroxylated polyalkadienes, polyhydroxylated polythioethers, and mixtures thereof. Preferably, the hydroxylated compounds are chosen from polyol polyesters, polyol polyethers, polyhydroxylated polycarbonates, and mixtures thereof.

The polyisocyanates that may be used according to the present invention are notably chosen from organic diisocyanates with a molecular weight from about 112 to 1000, and preferably from about 140 to 400.

Preferably, the polyisocyanates are chosen from diisocyanates and more particularly from those represented by the general formula R₂(NCO)₂, in which R₂ represents a divalent aliphatic hydrocarbon-based group containing from 4 to 18 carbon atoms, a divalent cycloaliphatic hydrocarbon-based group containing from 5 to 15 carbon atoms, a divalent araliphatic hydrocarbon-based group containing from 7 to 15 carbon atoms or a divalent aromatic hydrocarbon-based group containing from 6 to 15 carbon atoms.

Preferably, R₂ represents an organic diisocyanate. As examples of organic diisocyanates, the following may notably be chosen: tetramethylene diisocyanate, 1,6-hexamethylene diisocyanate, dodecamethylene diisocyanate, 1,3-diisocyanatocyclohexane, 1,4-diisocyanatocyclohexane, 3-isocyanatomethyl-3,5,5-trimethylcyclohexane isocyanate (isophorone diisocyanate or IPDI), bis(4-isocyanatocyclohexyl)methane, 1,3-bis(isocyanatomethyl)cyclohexane, 1,4-bis(isocyanatomethyl)cyclohexane, bis(4-isocyanato-3-methylcyclohexyl)methane, isomers of toluene diisocyanate (TDI) such as toluene 2,4-diisocyanate, toluene 2,6-diisocyanate and mixtures thereof, hydrogenated toluene diisocyanate, diphenylmethane 4,4'-diisocyanate and mixtures with its isomers diphenylmethane 2,4-diisocyanate and optionally diphenylmethane 2,2'-diisocyanate, naphthalene 1,5-diisocyanate, and mixtures thereof.

Preferably, the diisocyanates are aliphatic and cycloaliphatic diisocyanates, and are more preferentially chosen from 1,6-hexamethylene diisocyanate, 3-isocyanatomethyl-3,5,5-trimethylcyclohexane isocyanate, and mixtures thereof.

According to the present invention, the term "low molecular weight diol" refers to a diol with a molecular weight from about 62 to 700, and preferably from 62 to 200. These diols may comprise aliphatic, alicyclic or aromatic groups. Preferably, they comprise only aliphatic groups.

Preferably, R₃ represents a low molecular weight diol containing more than 20 carbon atoms, more preferentially chosen from ethylene glycol, diethylene glycol, 1,2-propanediol, 1,3-propanediol, 1,4-butanediol, 1,3-butylene glycol, neopentyl glycol, butylethylpropanediol, cyclohexanediol, 1,4-cyclohexanedimethanol, 1,6-hexanediol, bisphenol A (2,2-bis(4-hydroxyphenyl)propane), hydrogenated bisphenol A (2,2-bis(4-hydroxycyclohexyl)propane), and mixtures thereof.

The low molecular weight diols may optionally comprise ionic or potentially ionic groups. Examples of low molecular weight diols containing ionic or potentially ionic groups are notably described in patent US 3 412 054. Such compounds are preferably chosen from dimethylolbutanoic acid, dimethylolpropionic acid, polycaprolactone diols containing a carboxyl group, and mixtures thereof.

If low molecular weight diols containing ionic or potentially ionic groups are used, they are preferably used in an amount such that less than 0.30 meq of COOH per gram of polyurethane is present in the polyurethane dispersion.

The prepolymer is extended by means of two families of chain extenders. The first family of chain extenders corresponds to the compounds of general formula (B).

The chain extenders of formula (B) are preferably chosen from alkylenediamines, such as hydrazine, ethylenediamine, propylenediamine, 1,4-butylenediamine, piperazine; alkylenediamine oxides, such as 3-{2-[2-(3-aminopropoxy)ethoxy]ethoxy}propylamine (also known as dipropylamine diethylene glycol or DPA-DEG available from Tomah Products, Milton, Wis.), 2-methyl-1,5-pentanediamine (Dytec A from DuPont), hexanediamine, isophorone diamine, 4,4-methylenedi(cyclohexylamine), ether amines of the DPA series, available from Tomah Products, Milton, Wis., such as dipropylamine propylene glycol, dipropylamine dipropylene glycol, dipropylamine tripropylene glycol, dipropylamine poly(propylene glycol), dipropylamine ethylene glycol, dipropylamine poly(ethylene glycol), dipropylamine 1,3-propanediol, dipropylamine 2-methyl-1,3-propanediol, dipropylamine 1,4-butanediol, dipropylamine 1,3-butanediol, dipropylamine 1,6-hexanediol and dipropylamine cyclohexane-1,4-dimethanol; and mixtures thereof.

The second family of chain extenders corresponds to the compounds of general formula (C). Such compounds preferably have an ionic or potentially ionic group and two groups that can react with isocyanate groups. Such compounds may optionally comprise two groups that react with isocyanate groups and one group which is ionic or capable of forming an ionic group.

The ionic or potentially ionic group may preferably be chosen from ternary or quaternary ammonium groups or groups that can be converted into such groups, a carboxyl group, a carboxylate group, a sulfonic acid group and a sulfonate group. The at least partial conversion of groups that can be converted into a ternary or quaternary ammonium group salt may be performed before or during the mixing with water.

The chain extenders of formula (C) are preferably chosen from diaminosulfonates, for instance the sodium salt of N-(2-aminoethyl)-2-aminoethanesulfonic acid (ASA), the sodium salt of N-(2-aminoethyl)-2-aminopropionic acid, and mixtures thereof.

The polyurethane that may be used according to the present invention may optionally also comprise compounds which are located, respectively, at the chain ends and which terminate said chains (chain terminators). Such compounds are notably described in patents US 7 445 770 and/or US 7 452 770.

Preferably, the aqueous dispersion of polyurethane particles has a viscosity of less than 2000 mPa.s at 23°C, more preferentially less than 1500, and better still less than 1000. Even more preferably, the aqueous polyurethane dispersion has a glass transition temperature of less than 0°C.

Preferably also, the aqueous polyurethane dispersion has a polyurethane (or active material, or solids) content, on the basis of the weight of the dispersion, of from 20% to 60% by weight, more preferentially from 25% to 55% by weight and better still from 30% to 50% by weight. This is intended to mean that the polyurethane content (dry matter) of the aqueous dispersion is preferably from 20% to 60% by weight, more preferentially from 25% to 55% by weight and better still from 30% to 50% by weight, relative to the total weight of the dispersion.

Preferably also, the aqueous dispersion of polyurethane particles has a glass transition temperature (Tg) of less than or equal to -25°C, preferably less than -35°C and more preferentially less than -40°C.

The polyurethane particles may have a mean diameter ranging up to about 1000 nm, for example from about 50 nm to about 800 nm, better still from about 100 nm to about 500 nm. These particle sizes may be measured with a laser particle size analyser (for example Brookhaven BI90).

As non-limiting examples of aqueous polyurethane dispersions, mention may be made of those sold under the name Baycusan^{®} by Bayer, for instance Baycusan^{®} C1000 (INCI name: polyurethane-34), Baycusan^{®} C1001 (INCI name: polyurethane-34), Baycusan^{®} C1003 (INCI name: polyurethane-32), Baycusan^{®} C1004 (INCI name: polyurethane-35) and Baycusan^{®} C1008 (INCI name: polyurethane-48).

Mention may also be made of the aqueous polyurethane dispersions of isophthalic acid/adipic acid copolymer/hexylene glycol/neopentyl glycol/dimethylol acid/isophorone diisocyanate (INCI name: Polyurethane-1, such as Luviset^{®} PUR, BASF), the polyurethane of polycarbonate, polyurethane and aliphatic polyurethane of aliphatic polyester (such as the Neorez^{®} or DSM series, such as Neorez^{®} R989 and Neorez^{®} R-2202).

According to a preferred embodiment, the aqueous dispersion of polyurethane particles may be chosen from aqueous dispersions of particles of compounds having the INCI name polyurethane-35 or compounds having the INCI name polyurethane-34.

Preferably, the compound(s) containing at least one carboxylic acid group are in the form of aqueous dispersions of particles of acrylic polymers, more preferentially in the form of aqueous dispersions of film-forming acrylic polymer particles.

For the purposes of the invention, the term *"polymer"* means a compound corresponding to the repetition of one or more units (these units being derived from compounds known as monomers). This or these unit(s) are repeated at least twice and preferably at least three times.

The term *"film-forming polymer"* refers to a polymer that is capable of forming, by itself or in the presence of an auxiliary film-forming agent, a macroscopically continuous film on a support, notably on keratin hair fibers keratin materials, and preferably a cohesive film.

For the purposes of the present invention, the term *"acrylic polymer"* means a polymer synthesized from at least one monomer chosen from (meth)acrylic acid and/or (meth)acrylic acid ester and/or (meth)acrylic acid amide.

The unit(s) derived from the (meth)acrylic acid monomers of the polymer may optionally be in the form of salt(s), notably of alkali metal, alkaline-earth metal or ammonium salt(s), or organic base salt(s).

The (meth)acrylic acid esters (also known as (meth)acrylates) are advantageously chosen from alkyl (meth)acrylates, in particular C₁ to C₃₀, preferably C₁ to C₂₀ and better still C₁ to C₁₀ alkyl (meth)acrylates, aryl (meth)acrylates, in particular C₆ to C₁₀ aryl (meth)acrylates, and hydroxyalkyl (meth)acrylates, in particular C₂ to C₆ hydroxyalkyl (meth)acrylates.

Among the alkyl (meth)acrylates that may be mentioned are methyl (meth)acrylate, ethyl (meth)acrylate, butyl (meth)acrylate, isobutyl (meth)acrylate, 2-ethylhexyl (meth)acrylate, lauryl (meth)acrylate and cyclohexyl (meth)acrylate.

Among the hydroxyalkyl (meth)acrylates that may be mentioned are hydroxyethyl acrylate, 2-hydroxypropyl acrylate, hydroxyethyl methacrylate and 2-hydroxypropyl methacrylate.

Among the aryl (meth)acrylates that may be mentioned are benzyl acrylate and phenyl acrylate.

The (meth)acrylic acid esters that are particularly preferred are alkyl, preferably C₁ to C₃₀, more preferentially C₁ to C₂₀, better still C₁ to C₁₀, and even more particularly C₁ to C₄, alkyl (meth)acrylates.

According to the present invention, the alkyl group of the esters may be fluorinated, or even perfluorinated, i.e. some or all of the hydrogen atoms of the alkyl group are replaced with fluorine atoms.

As (meth)acrylic acid amides, examples that may be mentioned include (meth)acrylamides and also N-alkyl(meth)acrylamides, in particular N-(C₂ to C₁₂ alkyl)(meth)acrylamides. Among the N-alkyl(meth)acrylamides that may be mentioned are N-ethylacrylamide, N-t-butylacrylamide, N-t-octylacrylamide and N-undecylacrylamide.

The acrylic polymer according to the invention may be a homopolymer or a copolymer, advantageously a copolymer, better still a copolymer of (meth)acrylic acid and of (meth)acrylic acid esters.

Preferably, the acrylic polymer(s) according to the invention comprise one or more units derived from the following monomers:
a) (meth)acrylic acid; and
b) C₁ to C₃₀, more preferentially C₁ to C₂₀, better still C₁ to C₁₀, and even more particularly C₁ to C₄, alkyl (meth)acrylate.

Preferably, the aqueous dispersion of acrylic polymer particles does not comprise any surfactant.

The term *"surfactant"* refers to any agent that is capable of modifying the surface tension between two surfaces.

Among the acrylic polymers according to the invention, mention may be made of copolymers of (meth)acrylic acid and of methyl or ethyl (meth)acrylate, in particular copolymers of methacrylic acid and of ethyl acrylate such as the compound sold under the trade name Luvimer MAE by the company BASF, or the compound Polyacrylate-2 Crosspolymer sold under the trade name Fixate Superhold Polymer by the company Lubrizol, or the compound Acrylate Copolymer sold under the trade name Daitosol 3000VP3 by the company Daito Kasei Kogyo, or the compound Acrylate Polymer sold under the trade name Daitosol 3000 SLPN-PE1 or Daitosol 3000SLPN-SD by the company Daito Kasei Kogyo.

The acrylic polymer may optionally comprise one or more additional monomers, other than the (meth)acrylic acid and/or (meth)acrylic acid ester and/or (meth)acrylic acid amide monomers.

As additional monomer, mention will be made, for example, of styrene monomers, in particular styrene and α-methylstyrene, and preferably styrene.

In particular, the acrylic polymer may be a styrene/(meth)acrylate copolymer and notably a polymer chosen from copolymers resulting from the polymerization of at least one styrene monomer and at least one C₁ to C₂₀, preferably C₁ to C₁₀, alkyl (meth)acrylate monomer.

The C₁ to C₁₀ alkyl (meth)acrylate monomer may be chosen from methyl acrylate, ethyl acrylate, propyl acrylate, butyl acrylate, hexyl acrylate, octyl acrylate and 2-ethylhexyl acrylate.

As acrylic polymer, mention may be made of the styrene/(meth)acrylate copolymers sold under the name Joncryl 77 by the company BASF, under the name Yodosol GH41F by the company Akzo Nobel and under the name Syntran 5760 CG by the company Interpolymer.

Preferably, composition C comprises at least one aqueous dispersion of acrylic polymer particles.

More preferentially, composition C comprises at least one aqueous dispersion of acrylic polymer particles comprising one or more units derived from the following monomers:
a) (meth)acrylic acid; and
b) C₁ to C₃₀, more preferentially C₁ to C₂₀, better still C₁ to C₁₀, and even more particularly C₁ to C₄, alkyl (meth)acrylate.

Preferably, the aqueous dispersion of acrylic polymer particles has an acrylic polymer (or active material, or solids) content, on the basis of the weight of the dispersion, of from 20% to 60% by weight, more preferentially from 22% to 55% by weight and better still from 25% to 50% by weight.

### Silicone acrylic copolymer

According to a particular embodiment, the silicone compound(s) comprising at least one carboxylic group are chosen from silicone acrylic copolymers.

Thus, according to a particular embodiment, the compound(s) containing at least one carboxylic function are chosen from silicone acrylic copolymers.

Preferably, said silicone acrylic copolymer(s) comprise:
- at least one acrylic or methacrylic or crotonic unit; and
- at least one polydimethylsiloxane (PDMS) unit.

For the purposes of the present invention, the term *"polydimethylsiloxanes"* (also abbreviated as PDMSs) denotes, in accordance with what is generally accepted, any organosilicon polymer or oligomer of linear structure, of variable molecular weight, obtained by polymerization and/or polycondensation of suitably functionalized silanes, and constituted essentially of a repetition of main units in which the silicon atoms are linked together via oxygen atoms (siloxane bond Si-O-Si), including methyl radicals directly linked via a carbon atom to said silicon atoms.

The PDMS chains that may be used to obtain the copolymer used according to the invention include at least one polymerizable radical group, preferably located on at least one of the ends of the chain, i.e. the PDMS may contain, for example, a polymerizable radical group on each of the two ends of the chain or a polymerizable radical group on one end of the chain and a trimethylsilyl end group on the other end of the chain.

The term *"polymerizable radical group"* means a radical that is capable of polymerizing with other polymerizable radical groups or monomers. Preferably, the polydimethylsiloxane unit comprises at least one polymerizable radical group.

Preferably, the polymerizable radical group comprises at least one vinyl group.

Preferably, the polydimethylsiloxane (PDMS) unit comprises at least one polymerizable radical group comprising at least one vinyl group, preferably at least two polymerizable radical groups comprising at least one vinyl group, preferably located on at least one of the chain ends.

As indicated previously, said silicone acrylic copolymer(s) comprise(s) at least one acrylic or methacrylic or crotonic unit, i.e. at least one unit comprising a carboxylic group.

The term *"carboxylic group"* means a COOH or COO- functional group, the counterion of the COO- group possibly being chosen from alkali metals, alkaline-earth metals and quaternary ammoniums.

Preferably, said silicone acrylic copolymer(s) comprise:
- at least one acrylic or methacrylic or crotonic unit, and at least one acrylic ester or methacrylic ester or vinyl ester unit; and
- at least one polydimethylsiloxane (PDMS) unit.

More preferentially, the composition comprises one or more silicone acrylic copolymers comprising:
- at least one crotonic unit and at least one unit chosen from an alkyl crotonate unit, the alkyl radical being a linear or branched, saturated radical containing from 1 to 20 carbon atoms; a vinyl acetate unit; a vinyl alkyl ester unit, the alkyl radical being a linear or branched, saturated radical containing from 2 to 20 carbon atoms; and mixtures thereof; and
- at least one polydimethylsiloxane (PDMS) unit.

The term *"crotonic unit"* means a unit derived from a crotonic acid monomer or a salt thereof.

The term *"alkyl crotonate unit"* means a unit derived from a crotonic acid ester monomer containing a saturated, linear or branched alkyl radical containing from 1 to 20 carbon atoms.

The term *"vinyl alkyl ester unit"* means a unit derived from a vinyl ester monomer containing a saturated, linear or branched alkyl radical containing from 2 to 20 carbon atoms.

The term *"vinyl acetate unit"* means a unit derived from a vinyl acetate monomer.

According to a preferred embodiment, said silicone acrylic copolymer(s) comprise:
- at least one crotonic unit, at least one vinyl acetate unit and at least one vinyl alkyl ester unit, the alkyl radical being a saturated, linear or branched radical containing from 2 to 20 carbon atoms, preferably from 2 to 18 carbon atoms, and
- at least one polydimethylsiloxane (PDMS) unit.

According to a particularly preferred embodiment, said silicone acrylic copolymer(s) comprise:
- at least one crotonic unit, at least one vinyl acetate unit and at least one vinyl alkyl ester unit, the alkyl radical being a linear or branched, saturated radical containing from 6 to 16 carbon atoms, and
- at least one polydimethylsiloxane (PDMS) unit comprising at least one polymerizable radical group comprising at least one vinyl group.

More preferentially, the composition comprises one or more silicone acrylic copolymers comprising:
- at least one crotonic unit, at least one vinyl acetate unit and at least one vinyl alkyl ester unit, the alkyl radical being a saturated, linear or branched radical containing from 2 to 20 carbon atoms, preferably from 2 to 18 carbon atoms; and
- at least one polydimethylsiloxane (PDMS) unit including at least one polymerizable radical group comprising at least one vinyl group.

Even more preferentially, the composition comprises one or more silicone acrylic copolymers comprising:
- at least one crotonic unit, at least one vinyl acetate unit and at least one vinyl alkyl ester unit, the alkyl radical being a saturated, linear or branched radical containing from 6 to 16 carbon atoms; and
- at least one polydimethylsiloxane (PDMS) unit including at least one polymerizable radical group comprising at least one vinyl group.

Among the silicone acrylic copolymers that may be used in the context according to the invention, mention may be made of the compound sold by the company Wacker Chemie AG under the trade name Belsil^{®} P1101, having the INCI name Crotonic Acid/Vinyl C8-12 Isoalkyl Esters/VA/Bis-Vinyldimethicone Crosspolymer.

The total amount of the compound(s) containing at least one carboxylic acid group preferably ranges from 0.1% to 30% by weight, more preferentially from 0.5% to 20% by weight, better still from 0.5% to 15% by weight, and even more preferentially from 1% to 10% by weight, relative to the total weight of composition C.

The total amount of the aqueous dispersion(s) of polymer particles chosen from polyurethanes, acrylic polymers, and mixtures thereof preferably ranges from 0.1% to 50% by weight, more preferentially from 0.5% to 40% by weight, better still from 0.5% to 30% by weight, and even more preferentially from 1% to 20% by weight, relative to the total weight of composition C.

According to a particular embodiment, the total amount of the aqueous dispersion(s) of acrylic polymer particle(s) preferably ranges from 0.1% to 50% by weight, more preferentially from 0.5% to 40% by weight, better still from 0.5% to 30% by weight, and even more preferentially from 1% to 20% by weight, relative to the total weight of composition C.

Advantageously, the total amount of silicone acrylic copolymer(s) ranges from 0.1% to 30% by weight, preferably from 0.5% to 20% by weight, more preferentially from 1% to 15% by weight and better still from 2% to 10% by weight relative to the total weight of composition C.

### Amino silicone of formula (XIII)

Composition C used in the context of the dyeing process according to the invention comprises at least one amino silicone of formula (XIII) below: 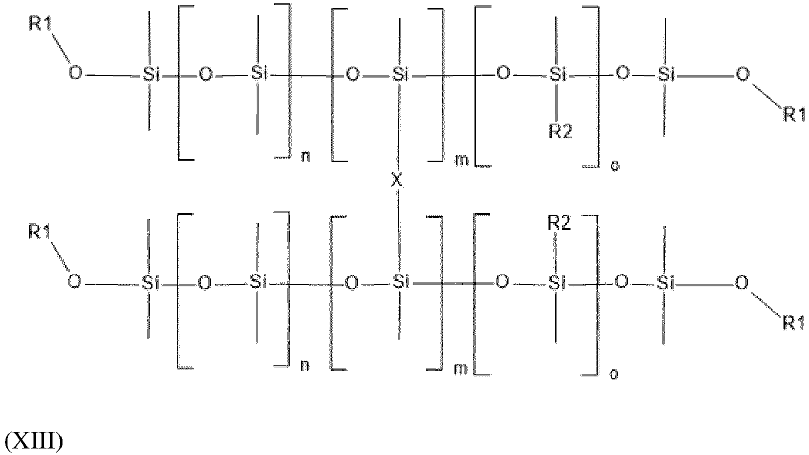 in which:
- **R1** independently represents a hydrogen atom or an alkyl group containing from 1 to 10 carbon atoms, preferably from 1 to 4 carbon atoms;
- **R2** independently represents a monovalent radical of formula -C_{q}H_{2q}L in which q is a number ranging from 2 to 8 and L is an optionally quaternized amine group chosen from the following groups:
   - N(R3)₂; -N+(R3)₃ A⁻; -NR3-Y-N(R3)₂ and -NR-Y-N+(R3)₃ A⁻,
      in which R3, which may be identical or different, represents a hydrogen atom, a phenyl group, a benzyl group or a saturated monovalent hydrocarbon-based radical, for example a C₁-C₂₀ alkyl group; Y denotes a linear or branched group of formula CᵣH₂ᵣ, r being an integer ranging from 2 to 6, preferably from 2 to 4; and A⁻ represents a cosmetically acceptable anion, notably a halide such as fluoride, chloride, bromide or iodide;
   - X represents a radical corresponding to formula (XIV) below: 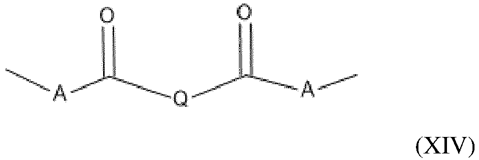 in which:
      - A independently represents a linear or branched alkylene group containing from 1 to 10 carbon atoms, which may be interrupted with at least one heteroatom chosen from O, S, NH or a carbonyl group (CO), preferably NH; and
      - Q represents an alkylene group containing from 1 to 20 carbon atoms, said alkylene group being optionally substituted with at least one alkoxy group containing from 1 to 10 carbon atoms, a hydroxyl group or an aminoalkyl group containing from 1 to 20 carbon atoms, and
      - n denotes an integer ranging from 0 to 500, m denotes an integer ranging from 1 to 500 and **o** denotes an integer ranging from 0 to 500 with n+m+o ranging from 250 to 500.

The term *"amino silicone"* denotes any silicone including at least one primary, secondary or tertiary amine or a quaternary ammonium group.

The weight-average molecular masses of these amino silicones may be measured by gel permeation chromatography (GPC) at room temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

According to a preferred embodiment, the amino silicone(s) of formula (XIII) are such that:
- **R1** independently represents a hydrogen atom or an alkyl group containing from 1 to 10 carbon atoms, preferably from 1 to 4 carbon atoms;
- R2 independently represents a monovalent radical of formula -C_{q}H_{2q}L in which q is a number ranging from 2 to 8 and L is an optionally quaternized amine group chosen from the following groups:
   - N(R3)₂;
   - N(R3)-CH₂-CH₂-N(R3)₂;
      in which R3, which may be identical or different, represents a hydrogen atom, a phenyl group, a benzyl group or a saturated monovalent hydrocarbon-based radical, for example a C₁-C₂₀ alkyl group;
   - X represents a radical corresponding to formula (XIV) below: 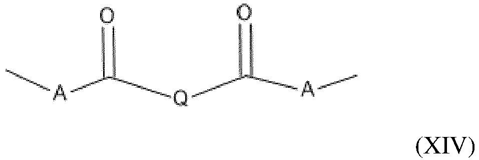 in which:
      - A independently represents a linear or branched alkylene group containing from 1 to 6 carbon atoms, which is interrupted with at least one heteroatom chosen from O, S, NH or a carbonyl group (CO), preferably NH; and
      - Q represents an alkylene group containing from 1 to 10 carbon atoms, said alkylene group being optionally substituted with at least one alkoxy group containing from 1 to 10 carbon atoms, a hydroxyl group or an aminoalkyl group containing from 1 to 20 carbon atoms, and
      - n denotes an integer ranging from 0 to 500, m denotes an integer ranging from 1 to 500 and **o** denotes an integer ranging from 0 to 500 with n+m+o ranging from 250 to 500.

According to a more preferred embodiment, the amino silicone(s) of formula (XIII) are such that:
- **R1** independently represents a hydrogen atom or an alkyl group containing from 1 to 10 carbon atoms, preferably from 1 to 4 carbon atoms;
- **R2** independently represents a monovalent radical of formula -C_{q}H_{2q}L in which q is a number ranging from 2 to 8 and L is an amine group having the following formula: -N(R3)-CH₂-CH₂-N(R3)₂;
   in which **R3** represents a hydrogen atom,
- X represents a radical corresponding to formula (XV) below: 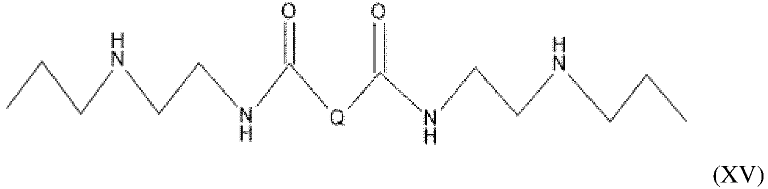 in which:
   - Q represents an alkylene group containing from 1 to 10 carbon atoms, said alkylene group being optionally substituted with at least one alkoxy group containing from 1 to 10 carbon atoms, a hydroxyl group or an aminoalkyl group containing from 1 to 20 carbon atoms, and
   - n denotes an integer ranging from 0 to 500, m denotes an integer ranging from 1 to 500 and **o** denotes an integer ranging from 0 to 500 with n+m+o ranging from 250 to 500.

According to a more preferred embodiment, the amino silicone is of formula (XVI) below: 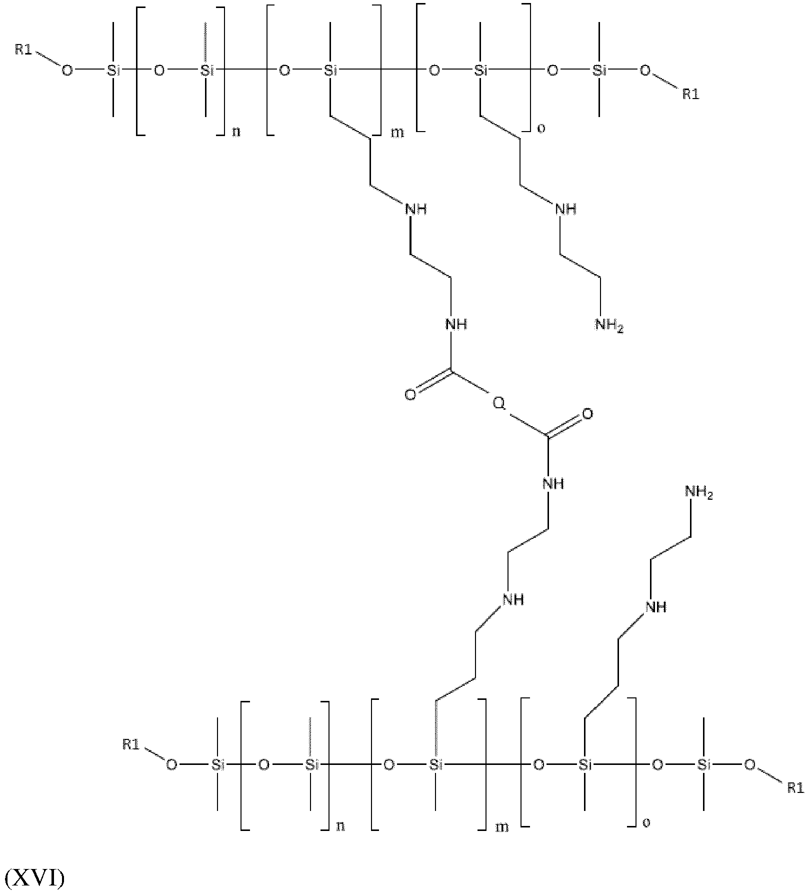 in which:
- **R1** independently represents a hydrogen atom or an alkyl group containing from 1 to 10 carbon atoms, preferably from 1 to 4 carbon atoms;
- Q represents an alkylene group containing from 1 to 10 carbon atoms, said alkylene group being optionally substituted with at least one alkoxy group containing from 1 to 10 carbon atoms, a hydroxyl group or an aminoalkyl group containing from 1 to 20 carbon atoms, and
- n denotes an integer ranging from 0 to 500, m denotes an integer ranging from 1 to 500 and **o** denotes an integer ranging from 0 to 500 with n+m+o ranging from 250 to 500.

According to a particular embodiment, the amino silicone(s) of formula (XIII) are present in emulsion or microemulsion with surfactants, preferably with nonionic surfactants such as trideceth-10.

Among the amino silicones of formula (XIII) that may be used in composition C, mention may be made of the amino silicone having the INCI name Bis-Hydroxy/Methoxy/Methyl Amodimethicone Crosspolymer.

Mention may also be made of the commercial product Belsil^{®} DADM 3240 E sold by the company Wacker.

The amino silicone(s) of formula (XIII) may be present in a total amount ranging from 0.01% to 20%, preferably from 0.05% to 15%, more preferentially from 0.1% to 10% and even more preferentially from 0.25% to 8% by weight relative to the total weight of composition C.

### Block silicone copolymer:

Composition C used in the context of the dyeing process according to the invention may comprise one or more block silicone copolymers different from the silicone compounds described previously.

Preferably, the block silicone copolymer used in the composition according to the invention is a linear block copolymer, i.e. a non-crosslinked copolymer obtained by chain extension and not by crosslinking. The term "block copolymer" denotes a polymer comprising at least two different blocks. Each block of the polymer is derived from one type of monomer or from several different types of monomer. This means that each block may consist of a homopolymer or a copolymer; this copolymer constituting the block may in turn be random or alternating. It should also be noted that when the copolymer is "linear", the polymer structure is not branched, star-branched or grafted.

The block silicone copolymer, which is preferably linear, is advantageously in the form of particles dispersed in an aqueous medium.

The block copolymers are obtained by chain extension.

The aqueous dispersions of block silicone copolymer particles used according to the invention may be chosen notably from those described in EP-A-874017.

According to said document, the silicone copolymers constituting these particles may notably be obtained by chain extension reaction, in the presence of a catalyst, from at least:
- (a) a polysiloxane (i) containing at least one reactive group and preferably one or two reactive groups per molecule; and
- (b) an organosilicon compound (ii) which reacts with the polysiloxane (i) by chain extension reaction.

In particular, the polysiloxane (i) is chosen from the compounds of formula (XVII): 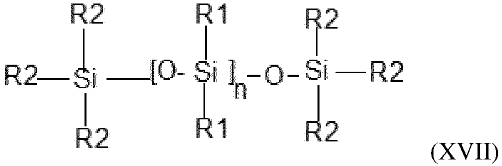 in which R1 and R2, independently of each other, represent a hydrocarbon-based group containing from 1 to 20 carbon atoms and preferably from 1 to 10 carbon atoms, such as methyl, ethyl, propyl or butyl, or an aryl group such as phenyl, or a reactive group, n is an integer greater than 1, on condition that there are on average between one and two reactive groups per polymer.

The term "reactive group" means any group that is capable of reacting with the organosilicon compound (ii) to form a block copolymer.

As reactive groups, mention may be made of hydrogen; aliphatically unsaturated groups and notably vinyl, allyl or hexanyl; the hydroxyl group; alkoxy groups such as methoxy, ethoxy or propoxy; alkoxy-alkoxy groups; the acetoxy group; amine groups, and mixtures thereof. Preferably, more than 90% and better still more than 98% of the reactive groups are at the end of the chain, i.e. the radicals R2 generally constitute more than 90% and even 98% of the reactive groups.

Preferably, n may notably be an integer ranging from 2 to 100, preferably from 10 to 30, and better still from 15 to 25.

The polysiloxanes of formula (XVII) are preferably linear polymers, i.e. including few branches, and generally less than 2 mol% of siloxane units. Moreover, the groups R1 and R2 may optionally be substituted with amine groups, epoxy groups, or groups including sulfur, silicon or oxygen.

Preferably, at least 80% of the groups R1 are alkyl groups and better still methyl groups. Preferably, the reactive group R2 is an aliphatically unsaturated group, notably vinyl. As polysiloxanes (i), mention may notably be made of dimethylvinylsiloxy-polydimethylsiloxane, a compound of formula (XVII) in which the radicals R1 are methyl radicals and, at the end of the chain, the radical R2 is a vinyl radical while the other two radicals R2 are methyl radicals.

The organosilicon compound (ii) may be chosen from the polysiloxanes of formula (XVII) or compounds acting as chain-extending agents.

If it is a compound of formulation (XVII), the polysiloxane (i) will include a first reactive group and the organosilicon compound (ii) will include a second reactive group which will react with the first.

If it is a chain-extending agent, it may be a silane, a siloxane (disiloxane or trisiloxane) or a silazane.

Preferably, the organosilicon compound (ii) is a liquid organohydrogenopolysiloxane of formula (XVIII): 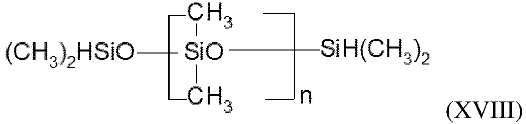 where n is an integer greater than 1 and preferably greater than 10, for example ranging from 2 to 100, preferably from 10 to 30 and better still from 15 to 25. According to a particular embodiment of the invention, n is equal to 20.

The block silicone copolymers used according to the invention are advantageously free of oxyalkylene group(s), notably free of oxyethylene and/or oxypropylene group(s).

The catalyst for the reaction between the polysiloxane and the organosilicon compound may be chosen from metals and notably from platinum, rhodium, tin, titanium, copper and lead. It is preferably platinum or rhodium.

The block silicone copolymer(s) used according to the invention are generally in the form of an aqueous dispersion of particles, which may notably be obtained, for example, by mixing (a) water, (b) at least one emulsifier, (c) the polysiloxane (i), (d) the organosilicon compound (ii) and (e) a catalyst. Preferably, one of the constituents (c), (d) or (e) is added last to the mixture, so that the chain extension reaction only begins in the dispersion.

As emulsifiers that may be used in the preparation process described above to obtain the aqueous dispersion of particles, mention may be made of nonionic or ionic (anionic, cationic or amphoteric) emulsifiers. They are preferably nonionic emulsifiers, which may be chosen from polyalkylene glycol fatty alcohol ethers, including from 8 to 30 carbon atoms and preferably from 10 to 22 carbon atoms; polyoxyalkylenated and notably polyoxyethylenated sorbitan alkyl esters, where the alkyl radical includes from 8 to 30 carbon atoms and preferably from 10 to 22 carbon atoms; polyoxyalkylenated and notably polyoxyethylenated alkyl esters, in which the alkyl radical includes from 8 to 30 carbon atoms and preferably from 10 to 22 carbon atoms; polyethylene glycols; polypropylene glycols; diethylene glycols; and mixtures thereof. The amount of emulsifier(s) is generally from 1% to 30% by weight relative to the total weight of the reaction mixture.

The emulsifier used to obtain the aqueous dispersion of particles is preferably chosen from polyethylene glycol fatty alcohol ethers and mixtures thereof, and notably polyethylene glycol alcohol ethers including 12 or 13 carbon atoms and from 2 to 100 oxyethylene units and preferably from 3 to 50 oxyethylene units, and mixtures thereof. Examples that may be mentioned include C₁₂-C₁₃ Pareth-3, C₁₂-C₁₃ Pareth-23 and mixtures thereof.

According to a particular embodiment of the invention, the block silicone copolymers are obtained from dimethylvinylsiloxy-polydimethylsiloxane (or divinyl dimethicone) as compound (i), and the compound of formula (XVIII) with preferably n = 20, as compound (ii), preferably in the presence of a platinum-type catalyst, and preferably in the form of a particle dispersion obtained in the presence of C₁₂-C₁₃ Pareth-3 and C₁₂-C₁₃ Pareth-23 as emulsifiers.

As block silicone copolymer particles, use may notably be made of the product sold under the name HMW 2220 Nonionic Emulsion by the company Dow Corning (CTFA name: divinyl dimethicone/dimethicone copolymer/C12-C13 Pareth-3/C12-C13 Pareth-23), which is an aqueous divinyl dimethicone/dimethicone copolymer dispersion containing C₁₂-C₁₃ Pareth-3 and C₁₂-C₁₃ Pareth-23.

The block silicone copolymer(s) may be present in a total amount ranging from 0.01% to 15%, preferably from 0.05% to 10%, more preferentially from 0.1% to 8% and even more preferentially from 0.25% to 5% by weight relative to the total weight of composition C.

### Colouring agent

Composition C used in the context of the process according to the invention comprises one or more colouring agents chosen from pigments, direct dyes and mixtures thereof.

Preferably, composition C used in the context of the process according to the invention comprises one or more pigments.

The term *"pigment"* refers to any pigment that gives colour to keratin materials. Their solubility in water at 25°C and at atmospheric pressure (760 mmHg) is less than 0.05% by weight, and preferably less than 0.01%.

The pigments that may be used are notably chosen from the organic and/or mineral pigments known in the art, notably those described in Kirk-Othmer's Encyclopedia of Chemical Technology and in Ullmann's Encyclopedia of Industrial Chemistry*.*

They may be natural, of natural origin, or non-natural.

These pigments may be in pigment powder or paste form. They may be coated or uncoated.

The pigments may be chosen, for example, from mineral pigments, organic pigments, lakes, pigments with special effects such as nacres or glitter flakes, and mixtures thereof. The pigment may be a mineral pigment. The term "mineral pigment" refers to any pigment that satisfies the definition in Ullmann's encyclopedia in the chapter on inorganic pigments. Among the mineral pigments that are useful in the present invention, mention may be made of iron oxides, chromium oxides, manganese violet, ultramarine blue, chromium hydrate, ferric blue and titanium oxide.

The pigment may be an organic pigment. The term "organic pigment" refers to any pigment that satisfies the definition in Ullmann's encyclopedia in the chapter on organic pigments.

The organic pigment may notably be chosen from nitroso, nitro, azo, xanthene, pyrene, quinoline, anthraquinone, triphenylmethane, fluorane, phthalocyanine, metal-complex, isoindolinone, isoindoline, quinacridone, perinone, perylene, diketopyrrolopyrrole, indigo, thioindigo, dioxazine, triphenylmethane and quinophthalone compounds.

In particular, the white or coloured organic pigments may be chosen from carmine, carbon black, aniline black, azo yellow, quinacridone, phthalocyanine blue, the blue pigments codified in the Color Index under the references CI 42090, 69800, 69825, 74100, 74160, the yellow pigments codified in the Color Index under the references CI 11680, 11710, 19140, 20040, 21100, 21108, 47000, 47005, the green pigments codified in the Color Index under the references CI 61565, 61570, 74260, the orange pigments codified in the Color Index under the references CI 11725, 45370, 71105, the red pigments codified in the Color Index under the references CI 12085, 12120, 12370, 12420, 12490, 14700, 15525, 15580, 15620, 15630, 15800, 15850, 15865, 15880, 26100, 45380, 45410, 58000, 73360, 73915, 75470, the pigments obtained by oxidative polymerization of indole or phenol derivatives as described in patent FR 2 679 771.

Examples that may also be mentioned include pigment pastes of organic pigments, such as the products sold by the company Hoechst under the names:
- Cosmenyl Yellow 10G: Yellow 3 pigment (CI 11710);
- Cosmenyl Yellow G: Yellow 1 pigment (CI 11680);
- Cosmenyl Orange GR: Orange 43 pigment (CI 71105);
- Cosmenyl Red R: Red 4 pigment (CI 12085);
- Cosmenyl Carmine FB: Red 5 pigment (CI 12490);
- Cosmenyl Violet RL: Violet 23 pigment (CI 51319);
- Cosmenyl Blue A2R: Blue 15.1 pigment (CI 74160);
- Cosmenyl Green GG: Green 7 pigment (CI 74260);
- Cosmenyl Black R: Black 7 pigment (CI 77266).

The pigments in accordance with the invention may also be in the form of composite pigments, as described in patent EP 1 184 426. These composite pigments may notably be composed of particles including an inorganic core, at least one binder for attaching the organic pigments to the core, and at least one organic pigment which at least partially covers the core.

The organic pigment may also be a lake. The term "lake" refers to dyes adsorbed onto insoluble particles, the assembly thus obtained remaining insoluble during use.

The mineral substrates onto which the dyes are adsorbed are, for example, alumina, silica, calcium sodium borosilicate or calcium aluminium borosilicate and aluminium.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 10 (CI 77 002), D&C Green 3 (CI 42 053), D&C Blue 1 (CI 42 090).

An example of a lake that may be mentioned is the product known under the following name: D&C Red 7 (CI 15 850:1).

The pigment may also be a pigment with special effects. The term "pigments with special effects" means pigments that generally create a coloured appearance (characterized by a certain shade, a certain vivacity and a certain level of luminance) that is non-uniform and that changes as a function of the conditions of observation (light, temperature, angles of observation, etc.). They thereby differ from coloured pigments, which afford a standard uniform opaque, semi-transparent or transparent shade.

Several types of special-effect pigments exist: those with a low refractive index, such as fluorescent or photochromic pigments, and those with a higher refractive index, such as nacres, interference pigments or glitter flakes.

Examples of pigments with special effects that may be mentioned include nacreous pigments such as mica covered with titanium or with bismuth oxychloride, coloured nacreous pigments such as mica covered with titanium and with iron oxides, mica covered with iron oxide, mica covered with titanium and notably with ferric blue or with chromium oxide, mica covered with titanium and with an organic pigment as defined previously, and also nacreous pigments based on bismuth oxychloride. Nacreous pigments that may be mentioned include the nacres Cellini sold by BASF (mica-TiO₂-lake), Prestige sold by Eckart (mica-TiO₂), Prestige Bronze sold by Eckart (mica-Fe₂O₃) and Colorona sold by Merck (mica-TiO₂-Fe₂O₃).

Mention may also be made of the gold-coloured nacres sold notably by the company BASF under the name Brilliant gold 212G (Timica), Gold 222C (Cloisonne), Sparkle gold (Timica), Gold 4504 (Chromalite) and Monarch gold 233X (Cloisonne); the bronze nacres sold notably by the company Merck under the name Bronze fine (17384) (Colorona) and Bronze (17353) (Colorona) and by the company BASF under the name Super bronze (Cloisonne); the orange nacres sold notably by the company BASF under the name Orange 363C (Cloisonne) and Orange MCR 101 (Cosmica) and by the company Merck under the name Passion orange (Colorona) and Matte orange (17449) (Microna); the brown nacres sold notably by the company BASF under the name Nu-antique copper 340XB (Cloisonne) and Brown CL4509 (Chromalite); the nacres with a copper tint sold notably by the company BASF under the name Copper 340A (Timica); the nacres with a red tint sold notably by the company Merck under the name Sienna fine (17386) (Colorona); the nacres with a yellow tint sold notably by the company BASF under the name Yellow (4502) (Chromalite); the red nacres with a gold tint sold notably by the company BASF under the name Sunstone G012 (Gemtone); the pink nacres sold notably by the company BASF under the name Tan opale G005 (Gemtone); the black nacres with a gold tint sold notably by the company BASF under the name Nu antique bronze 240 AB (Timica), the blue nacres sold notably by the company Merck under the name Matte blue (17433) (Microna), the white nacres with a silvery tint sold notably by the company Merck under the name Xirona Silver, and the golden-green pink-orange nacres sold notably by the company Merck under the name Indian summer (Xirona), and mixtures thereof.

Still as examples of nacres, mention may also be made of particles including a borosilicate substrate coated with titanium oxide.

Particles comprising a glass substrate coated with titanium oxide are notably sold under the name Metashine MC1080RY by the company Toyal.

Finally, examples of nacres that may also be mentioned include polyethylene terephthalate glitter flakes, notably those sold by the company Meadowbrook Inventions under the name Silver 1P 0.004X0.004 (silver glitter flakes). It is also possible to envisage multilayer pigments based on synthetic substrates, such as alumina, silica, calcium sodium borosilicate, calcium aluminium borosilicate and aluminium.

The pigments with special effects may also be chosen from reflective particles, i.e. notably from particles whose size, structure, notably the thickness of the layer(s) of which they are made and their physical and chemical nature, and surface state, allow them to reflect incident light. This reflection may, where appropriate, have an intensity sufficient to create at the surface of the composition or of the mixture, when it is applied to the support to be made up, highlight points that are visible to the naked eye, i.e. brighter points that contrast with their environment, making them appear to sparkle.

The reflective particles may be selected so as not to significantly alter the colouring effect generated by the colouring agents with which they are combined, and more particularly so as to optimize this effect in terms of colour rendition. They may more particularly have a yellow, pink, red, bronze, orange, brown, gold and/or coppery colour or tint.

These particles may have varied forms and may notably be in platelet or globular form, in particular in spherical form.

The reflective particles, whatever their form, may or may not have a multilayer structure and, in the case of a multilayer structure, may have, for example, at least one layer of uniform thickness, notably of a reflective material.

When the reflective particles do not have a multilayer structure, they may be composed, for example, of metal oxides, notably titanium or iron oxides obtained synthetically.

When the reflective particles have a multilayer structure, they may include, for example, a natural or synthetic substrate, notably a synthetic substrate at least partially coated with at least one layer of a reflective material, notably of at least one metal or metallic material. The substrate may be made of one or more organic and/or mineral materials.

More particularly, it may be chosen from glasses, ceramics, graphite, metal oxides, aluminas, silicas, silicates, notably aluminosilicates and borosilicates, and synthetic mica, and mixtures thereof, this list not being limiting.

The reflective material may include a layer of metal or of a metallic material.

Reflective particles are notably described in JP-A-09188830, JP-A-10158450, JP-A-10158541, JP-A-07258460 and JP-A-05017710.

Again as an example of reflective particles including a mineral substrate coated with a layer of metal, mention may also be made of particles including a silver-coated borosilicate substrate.

Particles with a silver-coated glass substrate, in the form of platelets, are sold under the name Microglass Metashine REFSX 2025 PS by the company Toyal. Particles with a glass substrate coated with a nickel/chromium/molybdenum alloy are sold under the names Crystal Star GF 550 and GF 2525 by this same company.

Use may also be made of particles comprising a metal substrate, such as silver, aluminium, iron, chromium, nickel, molybdenum, gold, copper, zinc, tin, magnesium, steel, bronze or titanium, said substrate being coated with at least one layer of at least one metal oxide, such as titanium oxide, aluminium oxide, iron oxide, cerium oxide, chromium oxide, silicon oxides and mixtures thereof.

Examples that may be mentioned include aluminium powder, bronze powder or copper powder coated with SiO₂ sold under the name Visionaire by the company Eckart.

Mention may also be made of interference pigments which are not attached to a substrate, such as liquid crystals (Helicones HC from Wacker) or interference holographic glitter flakes (Geometric Pigments or Spectra f/x from Spectratek). Special-effect pigments also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

The variety of pigments that may be used in the present invention makes it possible to obtain a wide range of colours, and also particular optical effects such as metallic effects or interference effects.

The size of the pigment used in the composition according to the present invention is generally between 10 nm and 200 µm, preferably between 20 nm and 80 µm and more preferentially between 30 nm and 50 µm.

The pigments may be dispersed in the composition by means of a dispersant.

The dispersant serves to protect the dispersed particles against their agglomeration or flocculation. This dispersant may be a surfactant, an oligomer, a polymer or a mixture of several thereof, bearing one or more functionalities with strong affinity for the surface of the particles to be dispersed. In particular, they may become physically or chemically attached to the surface of the pigments. These dispersants also contain at least one functional group that is compatible with or soluble in the continuous medium. In particular, esters of 12-hydroxystearic acid in particular and of C8 to C20 fatty acid and of polyols such as glycerol or diglycerol are used, such as poly(12-hydroxystearic acid) stearate with a molecular weight of approximately 750 g/mol, such as the product sold under the name Solsperse 21 000 by the company Avecia, polyglyceryl-2 dipolyhydroxystearate (CTFA name) sold under the reference Dehymyls PGPH by the company Henkel, or else polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the composition may be surface-treated with an organic agent.

Thus, the pigments surface-treated beforehand that are useful in the context of the invention are pigments which have been totally or partially subjected to a surface treatment of chemical, electronic, electrochemical, mechanochemical or mechanical nature with an organic agent, such as those described notably in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or aluminium salts of fatty acids, for example aluminium stearate or laurate; metal alkoxides; polyethylene; (meth)acrylic polymers, for example polymethyl methacrylates; polymers and copolymers containing acrylate units; alkanolamines; silicone compounds, for example silicones, notably polydimethylsiloxanes; organofluorine compounds, for example perfluoroalkyl ethers; fluorosilicone compounds.

The surface-treated pigments that are useful in the composition may also have been treated with a mixture of these compounds and/or may have undergone several surface treatments.

The surface-treated pigments that are useful in the context of the present invention may be prepared according to surface-treatment techniques that are well known to those skilled in the art, or may be commercially available as is.

Preferably, the surface-treated pigments are coated with an organic layer.

The organic agent with which the pigments are treated may be deposited on the pigments by evaporation of solvent, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is notably described in patent US 4 578 266.

An organic agent covalently bonded to the pigments will preferably be used.

The agent for the surface treatment may represent from 0.1% to 50% by weight relative to the total weight of the surface-treated pigment, preferably from 0.5% to 30% by weight and even more preferentially from 1% to 20% by weight relative to the total weight of the surface-treated pigment.

Preferably, the surface treatments of the pigments are chosen from the following treatments:
- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- a magnesium myristate treatment, for instance the MM surface treatment sold by LCW;
- an aluminium dimyristate treatment, for instance the MI surface treatment sold by Miyoshi;
- a perfluoropolymethyl isopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrogenosiloxane/perfluoroalkyl phosphate treatment, for instance the FS01 surface treatment sold by Daito;
- an acrylate copolymer/dimethicone treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

According to a particular embodiment of the invention, the dispersant is present with organic or mineral pigments in submicron-sized particulate form.

The term *"submicron-sized"* or *"submicronic"* refers to pigments having a particle size that has been micronized by a micronization method and having a mean particle size of less than a micrometre (µm), in particular between 0.1 and 0.9 µm, and preferably between 0.2 and 0.6 µm.

According to one embodiment, the dispersant and the pigment(s) are present in an amount (dispersant:pigment), according to a weight ratio, of between 1:4 and 4:1, particularly between 1.5:3.5 and 3.5:1 or better still between 1.75:3 and 3:1.

The dispersant(s) may thus have a silicone backbone, such as silicone polyether and dispersants of amino silicone type other than the amino silicones described previously. Among the suitable dispersants that may be mentioned are:
- amino silicones, i.e. silicones comprising one or more amine groups such as those sold under the names and references: BYK LPX 21879 by BYK, GP-4, GP-6, GP-344, GP-851, GP-965, GP-967 and GP-988-1, sold by Genesee Polymers,
- silicone acrylates such as Tego^{®} RC 902, Tego^{®} RC 922, Tego^{®} RC 1041, and Tego^{®} RC 1043, sold by Evonik,
- polydimethylsiloxane (PDMS) silicones bearing carboxyl groups such as X-22162 and X-22370 by Shin-Etsu, epoxy silicones such as GP-29, GP-32, GP-502, GP-504, GP-514, GP-607, GP-682, and GP-695 by Genesee Polymers, or Tego^{®} RC 1401, Tego^{®} RC 1403, Tego^{®} RC 1412 by Evonik.

According to a particular embodiment, the dispersant(s) are of amino silicone type other than the amino silicones described previously and are cationic.

Preferably, the pigment(s) are chosen from mineral, mixed mineral-organic or organic pigments.

In one variant of the invention, the pigment(s) are organic pigments, preferentially organic pigments surface-treated with an organic agent chosen from silicone compounds. In another variant of the invention, the pigment(s) are mineral pigments.

Preferably, the pigment(s) are chosen from iron oxides, notably red, yellow, brown or black iron oxides, nacres, notably mica coated with titanium or with bismuth oxychloride, mica coated with titanium and with iron oxides, or mica coated with iron oxide, and mixtures thereof.

As an example of an iron oxide, mention may be made of the iron oxide sold by the company Sun Chemical under the name SunPuro^{®} Red Iron Oxide).

As an example of a nacre, mention may be made of the iron oxide-coated mica sold by the company Sudarshan Chemical under the name Prestige Super Soft Bronze.

### Direct dye

Composition C used in the context of the process according to the invention may comprise one or more direct dyes.

The term *"direct dye"* means natural and/or synthetic dyes, other than oxidation dyes. These are dyes that will spread superficially on the fibre.

They may be ionic or nonionic, preferably cationic or nonionic.

Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.

The direct dyes are preferably cationic direct dyes. Mention may be made of the hydrazono cationic dyes of formulae (XIX) and (XX) and the azo cationic dyes (XXI) and (XXII) below:

Hét⁺-N(Ra)-N=C(Rb)-Ar, Q- (XIX),

Hét⁺-C(Ra)=N-N(Rb)-Ar, Q (XX),

Hét⁺-N=N-Ar, Q- (XXI),

Ar⁺-N=N-Ar", Q- (XXII),

in which formulae (XIX) to (XXII):
- Het⁺ represents a cationic heteroaryl radical, preferentially bearing an endocyclic cationic charge, such as imidazolium, indolium or pyridinium, which is optionally substituted, preferentially with at least one (C₁-C₈)alkyl group such as methyl;
- Ar⁺ represents an aryl radical, such as phenyl or naphthyl, bearing an exocyclic cationic charge, preferentially ammonium, particularly tri(C₁-C₈)alkylammonium, such as trimethylammonium;
- Ar represents an aryl group, notably phenyl, which is optionally substituted, preferentially with one or more electron-donating groups such as i) optionally substituted (C₁-C₈)alkyl, ii) optionally substituted (C₁-C₈)alkoxy, iii) (di)(C₁-C₈)(alkyl)amino optionally substituted on the alkyl group(s) with a hydroxyl group, iv) aryl(C₁-Cs)alkylamino, v) optionally substituted N-(C₁-C₈)alkyl-N-aryl(C₁-C₈)alkylamino or alternatively Ar represents a julolidine group;
- Ar" represents an optionally substituted (hetero)aryl group, such as phenyl or pyrazolyl, which are optionally substituted, preferentially with one or more (C₁-C₈)alkyl, hydroxyl, (di)(C₁-C₈)(alkyl)amino, (C₁-C₈)alkoxy or phenyl groups;
- Ra and Rb, which may be identical or different, represent a hydrogen atom or a (C₁-C₈)alkyl group, which is optionally substituted, preferentially with a hydroxyl group; or else the substituent Ra with a substituent of Het⁺ and/or Rb with a substituent of Ar form, together with the atoms that bear them, a (hetero)cycloalkyl; in particular, Ra and Rb represent a hydrogen atom or a (C₁-C₄)alkyl group optionally substituted with a hydroxyl group;
- Q- represents an organic or mineral anionic counterion, such as a halide or an alkyl sulfate.

In particular, mention may be made of the azo and hydrazono direct dyes bearing an endocyclic cationic charge of formulae (XIX) to (XXII) as defined previously, more particularly, the cationic direct dyes bearing an endocyclic cationic charge described in patent applications WO 95/15144, WO 95/01772 and EP 714 954, preferentially the following direct dyes: 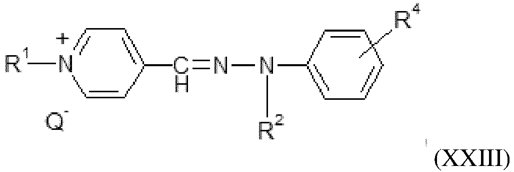 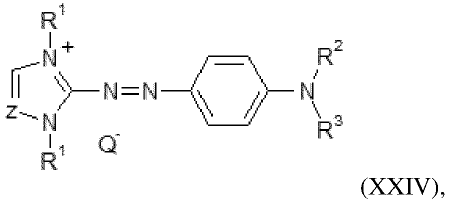 in which formulae (XXIII) and (XXIV):
- R¹ represents a (C₁-C₄)alkyl group such as methyl;
- R² and R³, which may be identical or different, represent a hydrogen atom or a (C₁-C₄)alkyl group, such as methyl; and
- R⁴ represents a hydrogen atom or an electron-donating group such as optionally substituted (C₁-C₈)alkyl, optionally substituted (C₁-C₈)alkoxy, or (di)(C₁-Cs)(alkyl)amino optionally substituted on the alkyl group(s) with a hydroxyl group; in particular, R⁴ is a hydrogen atom;
- Z represents a CH group or a nitrogen atom, preferentially CH,
- Q- is an anionic counterion as defined previously, in particular a halide, such as chloride, or an alkyl sulfate, such as methyl sulfate or mesyl.

In particular, the dyes of formulae (XXIII) and (XXIV) are chosen from Basic Red 51, Basic Yellow 87 and Basic Orange 31 or derivatives thereof with Q' being an anionic counterion as defined previously, particularly a halide such as chloride, or an alkyl sulfate such as methyl sulfate or mesyl.

The direct dyes may be chosen from anionic direct dyes. The anionic direct dyes of the invention are dyes commonly referred to as "acid" direct dyes owing to their affinity for alkaline substances. The term "anionic direct dye" means any direct dye including in its structure at least one CO₂R or SO₃R substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoid dyes and natural acid dyes.

As acid dyes that are useful for the invention, mention may be made of the dyes of formulae (XXV), (XXV'), (XXVI), (XXVI'), (XXVII), (XXVII'), (XXVIII), (XXVIII'), (XXIX), (XXX), (XXXI) and (XXXII) below:
a) the diaryl anionic azo dyes of formula (XXV) or (XXV'): 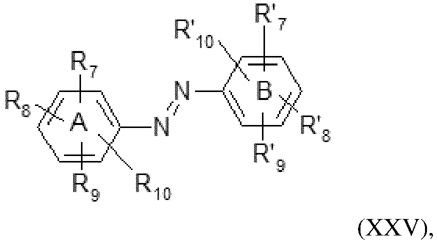 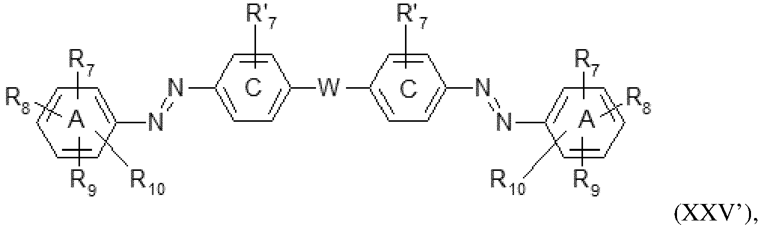 in which formulae (XXV) and (XXV'):
   - R₇, R₈, R₉, R₁₀, R'₇, R'₈, R'₉ and R'₁₀, which may be identical or different, represent a hydrogen atom or a group chosen from:
   - alkyl;
   - alkoxy, alkylthio;
   - hydroxyl, mercapto;
   - nitro, nitroso;
   - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
   - (O)₂S(O⁻)-, M⁺ with M⁺ representing a hydrogen atom or a cationic counterion;
   - (O)CO⁻-, M⁺ with M⁺ as defined previously;
   - R"-S(O)₂-, with R" representing a hydrogen atom or an alkyl, aryl, (di)(alkyl)amino or aryl(alkyl)amino group; preferentially a phenylamino or phenyl group;
   - R‴-S(O)₂-X'- with R‴ representing an optionally substituted alkyl or aryl group, X' as defined previously;
   - (di)(alkyl)amino;
   - aryl(alkyl)amino optionally substituted with one or more groups chosen from i) nitro; ii) nitroso; iii) (O)₂S(O⁻)-, M⁺ and iv) alkoxy with M⁺ as defined previously;
   - optionally substituted heteroaryl; preferentially a benzothiazolyl group;
   - cycloalkyl, notably cyclohexyl;
   - Ar-N=N- with Ar representing an optionally substituted aryl group; preferentially a phenyl optionally substituted with one or more alkyl, (O)₂S(O⁻)-, M⁺ or phenylamino groups;
   - or alternatively two contiguous groups R₇ with R₈ or R₈ with R₉ or R₉ with R₁₀ together form a fused benzo group A'; and R'₇ with R'₈ or R'₈ with R'₉ or R'₉ with R' ₁₀ together form a fused benzo group B'; with A' and B' optionally substituted with one or more groups chosen from i) nitro; ii) nitroso; iii) (O)₂S(O⁻)-, M⁺; iv) hydroxyl; v) mercapto; vi) (di)(alkyl)amino; vii) R°-C(X)-X'-; viii) R°-X'-C(X)-; ix) R°-X'-C(X)-X''-; x) Ar-N=N- and xi) optionally substituted aryl(alkyl)amino; with M⁺, R°, X, X', X" and Ar as defined previously;
   - W represents a sigma bond σ, an oxygen or sulfur atom, or a divalent radical i) -NR- with R as defined previously, or ii) methylene -C(Ra)(Rb)- with Ra and Rb, which may be identical or different, representing a hydrogen atom or an aryl group, or alternatively Ra and Rb form, together with the carbon atom that bears them, a spiro cycloalkyl;
   preferentially, W represents a sulfur atom or Ra and Rb together form a cyclohexyl;
   it being understood that formulae (XXV) and (XXV') comprise at least one sulfonate radical (O)₂S(O⁻)-, M⁺ or one carboxylate radical (O)CO⁻-, M⁺ on one of the rings A, A', B, B' or C; preferentially sodium sulfonate.

As examples of dyes of formula (XXV), mention may be made of: Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment Red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2, Food Yellow 3 or Sunset Yellow;
and, as examples of dyes of formula (XXV'), mention may be made of: Acid Red 111, Acid Red 134, Acid Yellow 38;
b) the pyrazolone anionic azo dyes of formulae (XXVI) and (XXVI'): 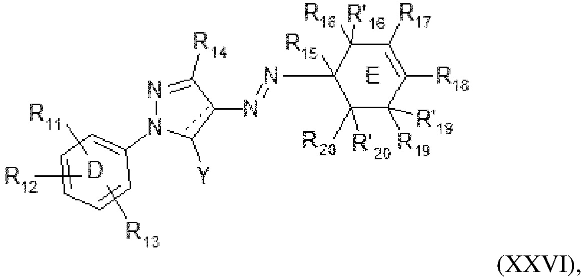 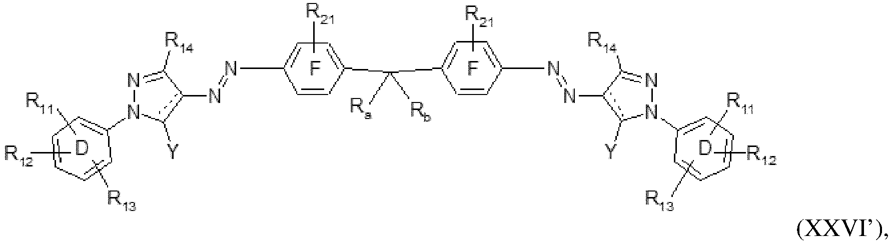
in which formulae (XXVI) and (XXVI'):
   - R₁₁, R₁₂ and R₁₃, which may be identical or different, represent a hydrogen or halogen atom, an alkyl group or -(O)₂S(O⁻), M⁺ with M⁺ as defined previously;
   - R₁₄ represents a hydrogen atom, an alkyl group or a group -C(O)O⁻, M⁺ with M⁺ as defined previously;
   - R₁₅ represents a hydrogen atom;
   - R₁₆ represents an oxo group, in which case R'₁₆ is absent, or alternatively R₁₅ with R₁₆ together form a double bond;
   - R₁₇ and R₁₈, which may be identical or different, represent a hydrogen atom, or a group chosen from:
      - (O)₂S(O⁻)-, M⁺ with M⁺ as defined previously;
      - Ar-O-S(O)₂- with Ar representing an optionally substituted aryl group; preferentially a phenyl optionally substituted with one or more alkyl groups;
      - R₁₉ and R₂₀ together form either a double bond, or a benzo group D', which is optionally substituted;
      - R'₁₆, R' ₁₉ and R'₂₀, which may be identical or different, represent a hydrogen atom or an alkyl or hydroxyl group;
      - R₂₁ represents a hydrogen atom or an alkyl or alkoxy group;
      - Ra and Rb, which may be identical or different, are as defined previously; preferentially, Ra represents a hydrogen atom and Rb represents an aryl group;
      - Y represents either a hydroxyl group or an oxo group;
      - - - - represents a single bond when Y is an oxo group; and represents a double bond when Y represents a hydroxyl group;
it being understood that formulae (XXVI) and (XXVI') comprise at least one sulfonate radical (O)₂S(O⁻)-, M⁺ or one carboxylate radical -C(O)O⁻, M⁺ on one of the rings D or E; preferentially sodium sulfonate.
As examples of dyes of formula (XXVI), mention may be made of: Acid Red 195, Acid Yellow 23, Acid Yellow 27, Acid Yellow 76, and as examples of dyes of formula (XXVI'), mention may be made of: Acid Yellow 17;
c) the anthraquinone dyes of formulae (XXVII) and (XXVII'): 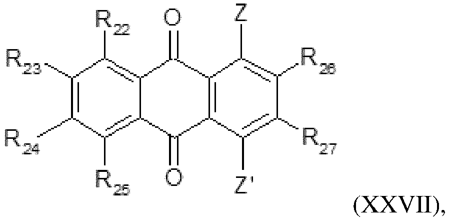 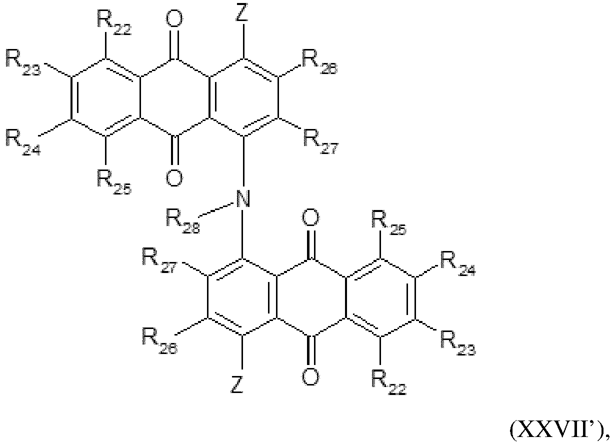
in which formulae (XXVII) and (XXVII'):
   - R₂₂, R₂₃, R₂₄, R₂₅, R₂₆ and R₂₇, which may be identical or different, represent a hydrogen or halogen atom, or a group chosen from:
   - alkyl;
   - hydroxyl, mercapto;
   - alkoxy, alkylthio;
   - optionally substituted aryloxy or arylthio, preferentially substituted with one or more groups chosen from alkyl and (O)₂S(O⁻)-, M⁺ with M⁺ as defined previously;
   - aryl(alkyl)amino optionally substituted with one or more groups chosen from alkyl and (O)₂S(O⁻)-, M⁺ with M⁺ as defined previously;
   - (di)(alkyl)amino;
   - (di)(hydroxyalkyl)amino;
   - (O)₂S(O⁻)-, M⁺ with M⁺ as defined previously;
   - Z' represents a hydrogen atom or a group NR₂₈R₂₉ with R₂₈ and R₂₉, which may be identical or different, representing a hydrogen atom or a group chosen from:
   - alkyl;
   - polyhydroxyalkyl such as hydroxyethyl;
   - aryl optionally substituted with one or more groups, particularly i) alkyl such as methyl, n-dodecyl, n-butyl; ii) (O)₂S(O⁻)-, M⁺ with M⁺ as defined previously; iii) R°-C(X)-X'-, R°-X'-C(X)-, R°-X'-C(X)-X"- with R°, X, X' and X" as defined previously, preferentially R° represents an alkyl group;
   - cycloalkyl, notably cyclohexyl;
   - Z represents a group chosen from hydroxyl and NR'₂₈R'₂₉ with R'₂₈ and R'₂₉, which may be identical or different, representing the same atoms or groups as R₂₈ and R₂₉ as defined previously;
it being understood that formulae (XXVII) and (XXVII') comprise at least one sulfonate radical (O)₂S(O⁻)-, M⁺ or one carboxylate radical -C(O)O⁻, M⁺; preferentially sodium sulfonate.
As examples of dyes of formula (XXII), mention may be made of: Acid Blue 25, Acid Blue 43, Acid Blue 62, Acid Blue 78, Acid Blue 129, Acid Blue 138, Acid Blue 140, Acid Blue 251, Acid Green 25, Acid Green 41, Acid Violet 42, Acid Violet 43, Mordant Red 3; EXT Violet No. 2; and, as an example of a dye of formula (XXVII'), mention may be made of: Acid Black 48;
d) the nitro dyes of formulae (XXVIII) and (XXVIII'): 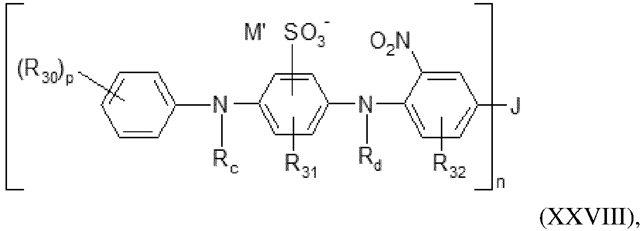 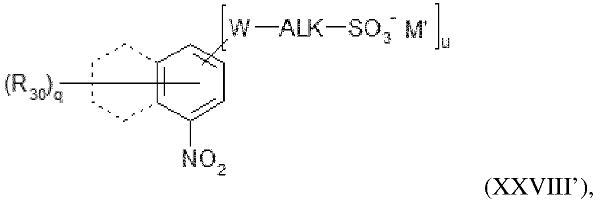
in which formulae (XXVIII) and (XXVIII'):
   - R₃₀, R₃₁ and R₃₂, which may be identical or different, represent a hydrogen or halogen atom, or a group chosen from:
   - alkyl;
   - alkoxy optionally substituted with one or more hydroxyl groups, alkylthio optionally substituted with one or more hydroxyl groups;
   - hydroxyl, mercapto;
   - nitro, nitroso;
   - polyhaloalkyl;
   - R°-C(X)-X'-, R°-X'-C(X)-, R°-X'-C(X)-X"- with R°, X, X' and X'' as defined previously;
   - (O)₂S(O⁻)-, M⁺ with M⁺ as defined previously;
   - (O)CO⁻-, M⁺ with M⁺ as defined previously;
   - (di)(alkyl)amino;
   - (di)(hydroxyalkyl)amino;
   - heterocycloalkyl such as piperidino, piperazino or morpholino; in particular, R₃₀, R₃₁ and R₃₂ represent a hydrogen atom;
   - Rc and Rd, which may be identical or different, represent a hydrogen atom or an alkyl group;
   - W is as defined previously; W particularly represents an -NH- group;
   - ALK represents a linear or branched divalent C₁-C₆ alkylene group; in particular, ALK represents a -CH₂-CH₂- group;
   - n is 1 or 2;
   - p represents an integer inclusively between 1 and 5;
   - q represents an integer inclusively between 1 and 4;
   - u is 0 or 1;
   - when n is 1, J represents a nitro or nitroso group; particularly nitro;
   - when n is 2, J represents an oxygen or sulfur atom, or a divalent radical -S(O)ₘ- with m representing an integer 1 or 2; preferentially, J represents an -SO₂- radical;
   - M' represents a hydrogen atom or a cationic counterion; 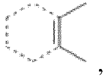 which may be present or absent, represents a benzo group optionally substituted with one or more groups R₃₀ as defined previously;
it being understood that formulae (XXVIII) and (XXVIII') comprise at least one sulfonate radical (O)₂S(O⁻)-, M⁺ or one carboxylate radical -C(O)O⁻, M⁺; preferentially sodium sulfonate.
As examples of dyes of formula (XXVIII), mention may be made of: Acid Brown 13 and Acid Orange 3; as examples of dyes of formula (XXVIII'), mention may be made of: Acid Yellow 1, the sodium salt of 2,4-dinitro-1-naphthol-7-sulfonic acid, 2-piperidino-5-nitrobenzenesulfonic acid, 2-(4'-N,N-(2"-hydroxyethyl)amino-2'-nitro)anilineethanesulfonic acid, 4-β-hydroxyethylamino-3-nitrobenzenesulfonic acid; EXT D&C Yellow 7;
e) the triarylmethane dyes of formula (XXIX): 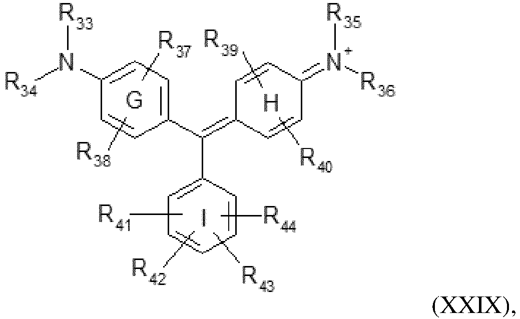
in which formula (XXIX):
   - R₃₃, R₃₄, R₃₅ and R₃₆, which may be identical or different, represent a hydrogen atom or a group chosen from alkyl, optionally substituted aryl and optionally substituted arylalkyl; particularly an alkyl and benzyl group optionally substituted with a group (O)ₘS(O⁻)-, M⁺ with M⁺ and m as defined previously;
   - R₃₇, R₃₈, R₃₉, R₄₀, R₄₁, R₄₂, R₄₃ and R₄₄, which may be identical or different, represent a hydrogen atom or a group chosen from:
   - alkyl;
   - alkoxy, alkylthio;
   - (di)(alkyl)amino;
   - hydroxyl, mercapto;
   - nitro, nitroso;
   - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
   - (O)₂S(O⁻)-, M⁺ with M⁺ representing a hydrogen atom or a cationic counterion;
   - (O)CO⁻-, M⁺ with M⁺ as defined previously;
   - or alternatively two contiguous groups R₄₁ with R₄₂ or R₄₂ with R₄₃ or R₄₃ with R₄₄ together form a fused benzo group: I'; with I' optionally substituted with one or more groups chosen from i) nitro; ii) nitroso; iii) (O)₂S(O⁻)-, M⁺; iv) hydroxyl; v) mercapto; vi) (di)(alkyl)amino; vii) R°-C(X)-X'-; viii) R°-X'-C(X)-; ix) R°-X'-C(X)-X"-; with M⁺, R°, X, X', X" as defined previously; in particular R₃₇ to R₄₀ represent a hydrogen atom, and R₄₁ to R₄₄, which may be identical or different, represent a hydroxyl or (O)₂S(O⁻)-, M⁺ group; and when R₄₃ with R₄₄ together form a benzo group, it is preferentially substituted with a (O)₂S(O-)- group;
it being understood that at least one of the rings G, H, I or I' comprises at least one sulfonate radical (O)₂S(O⁻)- or one carboxylate radical -C(O)O⁻; preferentially sulfonate.
As examples of dyes of formula (XXIX), mention may be made of: Acid Blue 1; Acid Blue 3; Acid Blue 7, Acid Blue 9; Acid Violet 49; Acid Green 3; Acid Green 5 and Acid Green 50.
f) the xanthene-based dyes of formula (XXX): 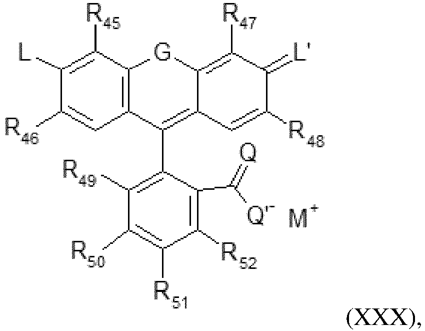
in which formula (XXX):
   - R₄₅, R₄₆, R₄₇ and R₄₈, which may be identical or different, represent a hydrogen or halogen atom;
   - R₄₉, R₅₀, R₅₁ and R₅₂, which may be identical or different, represent a hydrogen or halogen atom, or a group chosen from:
   - alkyl;
   - alkoxy, alkylthio;
   - hydroxyl, mercapto;
   - nitro, nitroso;
   - (O)₂S(O⁻)-, M⁺ with M⁺ representing a hydrogen atom or a cationic counterion;
   - (O)CO⁻-, M⁺ with M⁺ as defined previously;
particularly, R₄₉, R₅₀, R₅₁ and R₅₂ represent a hydrogen or halogen atom;
   - G represents an oxygen or sulfur atom or a group NRe with Re as defined previously; in particular, G represents an oxygen atom;
   - L represents an alkoxide O⁻, M⁺; a thioalkoxide S⁻, M⁺ or a group NRf, with Rf representing a hydrogen atom or an alkyl group, and M⁺ as defined previously; M⁺ is particularly sodium or potassium;
   - L' represents an oxygen or sulfur atom or an ammonium group: N⁺RfRg, with Rf and Rg, which may be identical or different, representing a hydrogen atom or an optionally substituted alkyl or aryl group; L' particularly represents an oxygen atom or a phenylamino group optionally substituted with one or more alkyl or (O)ₘS(O⁻)-, M⁺ groups with m and M⁺ as defined previously;
   - Q and Q', which may be identical or different, represent an oxygen or sulfur atom;
particularly, Q and Q' represent an oxygen atom;
   - M⁺ is as defined previously.
As examples of dyes of formula (XXX), mention may be made of: Acid Yellow 73; Acid Red 51; Acid Red 52; Acid Red 87; Acid Red 92; Acid Red 95; Acid Violet 9;
g) the indole-based dyes of formula (XXXI): 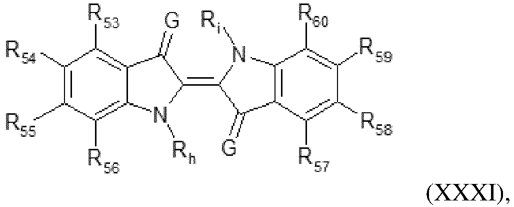
in which formula (XXXI):
   - R₅₃, R₅₄, R₅₅, R₅₆, R₅₇, R₅₈, R₅₉ and R₆₀, which may be identical or different, represent a hydrogen atom or a group chosen from:
   - alkyl;
   - alkoxy, alkylthio;
   - hydroxyl, mercapto;
   - nitro, nitroso;
   - R°-C(X)-X'-, R°-X'-C(X)-, R°-X'-C(X)-X''- with R° representing a hydrogen atom or an alkyl or aryl group; X, X' and X", which may be identical or different, representing an oxygen or sulfur atom, or NR with R representing a hydrogen atom or an alkyl group;
   - (O)₂S(O⁻)-, M⁺ with M⁺ representing a hydrogen atom or a cationic counterion;
   - (O)CO⁻-, M⁺ with M⁺ as defined previously;
   - G represents an oxygen or sulfur atom or a group NRe with Re as defined previously; in particular, G represents an oxygen atom;
   - Ri and Rh, which may be identical or different, represent a hydrogen atom or an alkyl group;
it being understood that formula (XXXI) comprises at least one sulfonate radical (O)₂S(O⁻)-, M⁺ or one carboxylate radical -C(O)O⁻, M⁺; preferentially sodium sulfonate. As examples of dyes of formula (XXXI), mention may be made of: Acid Blue 74;
h) the quinoline-based dyes of formula (XXXII): 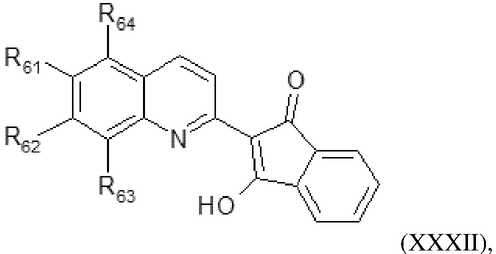
in which formula (XXXII):
   - R₆₁ represents a hydrogen or halogen atom or an alkyl group;
   - R₆₂, R₆₃ and R₆₄, which may be identical or different, represent a hydrogen atom or a group (O)₂S(O⁻)-, M⁺ with M⁺ representing a hydrogen atom or a cationic counterion;
or alternatively R₆₁ with R₆₂, or R₆₁ with R₆₄, together form a benzo group optionally substituted with one or more groups (O)₂S(O⁻)-, M⁺ with M⁺ representing a hydrogen atom or a cationic counterion;
it being understood that formula (XXXII) comprises at least one sulfonate radical (O)₂S(O⁻)-, M⁺, preferentially sodium sulfonate.

As examples of dyes of formula (XXXII), mention may be made of: Acid Yellow 2, Acid Yellow 3 and Acid Yellow 5.

Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin, apigenidin and orceins. Use may also be made of extracts or decoctions containing these natural dyes and particularly henna-based poultices or extracts.

Preferably, the direct dyes are chosen from anionic direct dyes.

The colouring agent(s) may be present in a total amount ranging from 0.001% to 20% by weight and preferably from 0.005% to 15% by weight relative to the total weight of composition C; preferably, the colouring agents are chosen from pigments.

The pigment(s) may be present in a total amount ranging from 0.05% to 20% by weight, preferably from 0.1% to 15% by weight and better still from 0.5% to 10% by weight, relative to the total weight of composition C.

The direct dye(s) may be present in a total amount ranging from 0.001% to 10% by weight relative to the total weight of the composition, preferably from 0.005% to 5% by weight relative to the total weight of composition C.

### Organic solvent

Composition C used in the context of the process according to the invention may comprise one or more organic solvents.

Preferably, the composition comprises ethanol.

The organic solvents may be present in a total amount ranging from 1% to 30% by weight, preferably ranging from 3% to 20% by weight, preferably ranging from 5% to 15% by weight, relative to the total weight of composition C.

Composition C used in the context of the process according to the invention may be aqueous. The water content may range from 1% to 90% by weight, preferably from 10% to 80% by weight and more preferentially from 20% to 75% by weight relative to the total weight of composition C.

### Additives

Composition C used in the context of the process according to the invention may contain any adjuvant or additive usually used.

Among the additives that may be contained in the composition, mention may be made of reducing agents, softeners, antifoams, moisturizers, UV-screening agents, peptizers, solubilizers, thickeners, fragrances, proteins, vitamins, preserving agents, oils, waxes and mixtures thereof.

Composition C used in the context of the process according to the invention may notably be in the form of a suspension, a dispersion, a gel, an emulsion, notably an oil-in-water (O/W) or water-in-oil (W/O) emulsion, or a multiple emulsion (W/O/W or polyol/O/W or O/W/O), in the form of a cream, a mousse, a stick, a dispersion of vesicles, notably of ionic or nonionic lipids, or a two-phase or multi-phase lotion.

A person skilled in the art may select the appropriate presentation form, and also the method for preparing it, on the basis of his general knowledge, taking into account firstly the nature of the constituents used, notably their solubility in the support, and secondly the intended application of the composition.

### Application of composition D

The process for dyeing keratin hair fibers according to the invention may also comprise the application to the keratin hair fibers of a composition D comprising at least one silicone compound comprising at least one carboxylic group.

Preferably, the silicone compound comprising at least one carboxylic group is a compound other than b) the compound containing at least one carboxylic acid group, c) the amino silicone of formula (XIII) and the block silicone copolymers.

The term *"carboxylic group"* means a COOH or COO⁻ functional group, the counterion of the COO⁻ group possibly being chosen from alkali metals, alkaline-earth metals and quaternary ammoniums.

The silicones that may be used may be soluble or insoluble in composition D; they may be in the form of oil, wax, resin or gum; silicone oils and gums are preferred.

Silicones are notably described in detail in Walter Noll's Chemistry and Technology of Silicones (1968), Academic Press.

Preferably, the silicone compound(s) comprising at least one carboxylic group are chosen from the organosiloxanes of formula (XXXIII) below: 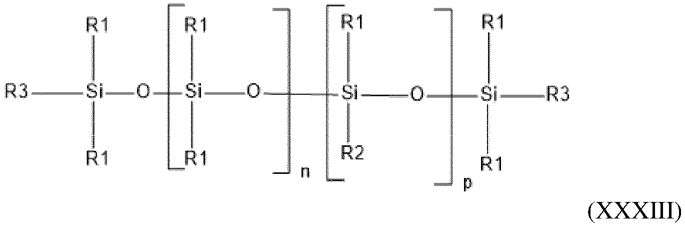 in which:
- **R1** independently represent an alkyl group containing from 1 to 20 carbon atoms, preferably from 1 to 10 carbon atoms; a hydroxyl group; an alkoxy group containing from 1 to 20 carbon atoms or an aryl group containing from 6 to 12 carbon atoms;
- R2 independently represents a group R4-COOM with R4 representing a linear or branched alkylene group containing from 1 to 20 carbon atoms, preferably from 4 to 16 carbon atoms, optionally interrupted with at least one heteroatom chosen from a sulfur atom, a nitrogen atom, an oxygen atom and mixtures thereof, and M representing a hydrogen atom; an alkali metal or alkaline-earth metal or a quaternary ammonium NR'3, with R', which may be identical or different, representing H or alkyl containing from 1 to 4 carbon atoms; a pyrrolidone radical comprising a carboxylic group COOH or a group Ra-(ORb)x-COOM with Ra representing a linear or branched alkylene group containing from 1 to 4 carbon atoms, Rb representing an alkyl group containing from 1 to 4 carbon atoms, x being an integer ranging from 1 to 200; and M representing a hydrogen atom, an alkali metal or alkaline-earth metal or a quaternary ammonium NR' ₃, with R', which may be identical or different, representing H or an alkyl containing from 1 to 4 carbon atoms;
- **R3** independently represent an alkyl group containing from 1 to 20 carbon atoms; a hydroxyl group; a group R4-COOM with **R4** representing a linear or branched alkylene group containing from 1 to 20 carbon atoms, preferably from 4 to 16 carbon atoms, optionally interrupted with at least one heteroatom chosen from a sulfur atom, a nitrogen atom, an oxygen atom and mixtures thereof, and **M** representing a hydrogen atom; an alkali metal or alkaline-earth metal or a quaternary ammonium NR'₃, with **R',** which may be identical or different, representing H or alkyl containing from 1 to 4 carbon atoms; an alkoxy group containing from 1 to 20 carbon atoms; an aryl group containing from 6 to 12 carbon atoms or a group Rₐ-(OR_{b})ₓ-COOM with **Rₐ** representing a linear or branched alkylene group containing from 1 to 4 carbon atoms, **R_{b}** representing an alkyl group containing from 1 to 4 carbon atoms, x being an integer ranging from 1 to 200; and **M** representing a hydrogen atom, an alkali metal or alkaline-earth metal or a quaternary ammonium NR'₃, with **R',** which may be identical or different, representing H or an alkyl containing from 1 to 4 carbon atoms;
- **n** denotes an integer ranging from 1 to 1000;
- **p** denotes an integer ranging from 0 to 1000;
it being understood that at least one of the radicals R2 and/or R3 comprises a carboxylic group COOH or COOM with M representing an alkali metal or alkaline-earth metal or a quaternary ammonium NR'₃, with **R',** which may be identical or different, representing H or an alkyl containing from 1 to 4 carbon atoms.

Notably, the silicone compound(s) comprising at least one carboxylic group may be chosen from the organosiloxanes of formula (XXXIV) below: 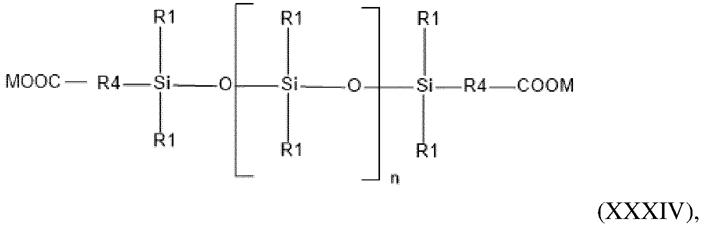 in which:
- **R1** independently represents a linear or branched alkyl group containing from 1 to 20 carbon atoms, preferably from 1 to 10 carbon atoms and better still from 1 to 6 carbon atoms, preferentially methyl;
- **R4** independently represents a linear or branched alkylene group containing from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group Rₐ-(OR_{b})ₓ- with **Rₐ** representing a linear or branched alkylene group containing from 1 to 4 carbon atoms, **R_{b}** representing an alkylene group containing from 1 to 4 carbon atoms, and x being an integer ranging from 1 to 200;
- **M** independently represents a hydrogen atom, an alkali metal or alkaline-earth metal or a quaternary ammonium NR'₃, with **R',** which may be identical or different, representing H or an alkyl containing from 1 to 4 carbon atoms;
- **n** denotes an integer ranging from 1 to 1000;
- the organosiloxanes of formula (XXXV) below: 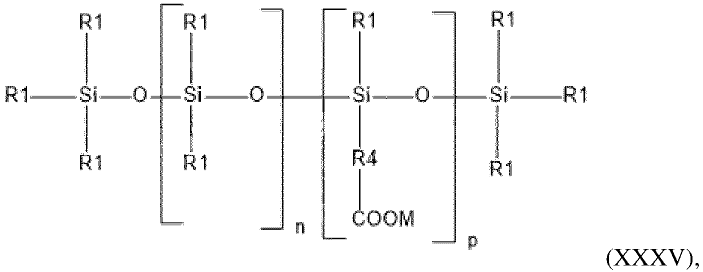 in which:
   - **R1** independently represents an alkyl group containing from 1 to 10 carbon atoms, preferably from 1 to 6 carbon atoms, more preferentially a methyl;
   - **R4** represents a linear or branched, saturated or unsaturated alkylene group containing from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group Rₐ-(OR_{b})ₓ- with **Rₐ** representing a linear or branched alkylene group containing from 1 to 4 carbon atoms, **R_{b}** representing an alkylene group containing from 1 to 4 carbon atoms, and **x** being an integer ranging from 1 to 200;
   - **M** represents a hydrogen atom, an alkali metal or alkaline-earth metal or a quaternary ammonium NR'₃, with **R',** which may be identical or different, representing H or an alkyl containing from 1 to 4 carbon atoms;
   - **p** denotes an integer ranging from 1 to 1000;
   - **n** denotes an integer ranging from 1 to 1000;
   - the organosiloxanes of formula (XXXVI) below: 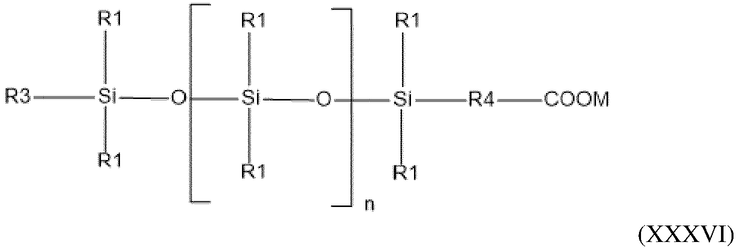 in which:
      - **R1** independently represents an alkyl group containing from 1 to 20 carbon atoms, preferably from 1 to 10 carbon atoms and better still from 1 to 6 carbon atoms, preferentially methyl;
      - **R4** represents a linear or branched alkylene group containing from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group Rₐ-(OR_{b})ₓ- with **Rₐ** representing a linear or branched alkylene group containing from 1 to 4 carbon atoms, **R_{b}** representing an alkylene group containing from 1 to 4 carbon atoms, and x being an integer ranging from 1 to 200;
      - **R3** represents an alkyl group containing from 1 to 20 carbon atoms, an alkoxy group containing from 1 to 20 carbon atoms or an aryl group containing from 6 to 12 carbon atoms;
      - **M** independently represents a hydrogen atom, an alkali metal or alkaline-earth metal or a quaternary ammonium NR'₃, with **R',** which may be identical or different, representing H or an alkyl containing from 1 to 4 carbon atoms;
      - n denotes an integer ranging from 1 to 1000;
      - the organosiloxanes of formula (XXXVII) below: 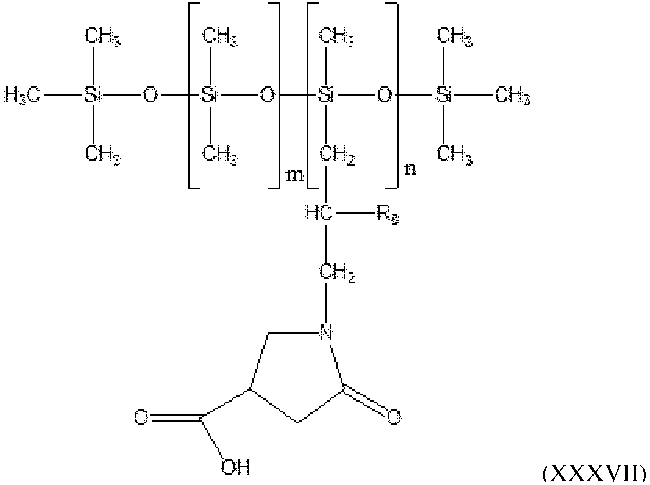 in which:
         - **R8** represents an alkyl group containing from 1 to 6 carbon atoms, preferably a methyl;
         - **m** denotes an integer ranging from 1 to 1000;
         - **n** denotes an integer ranging from 1 to 1000;
         - and mixtures thereof.

Among the organosiloxanes of formula (XXXIV), mention may be made of polydimethylsiloxanes (PDMS) bearing a carboxyl end function, such as the compounds sold by the company Momentive under the trade name Silform INX (INCI name: Bis-Carboxydecyl Dimethicone).

Among the organosiloxanes of formula (XXXV), mention may be made of polydimethylsiloxanes (PDMS) bearing a carboxyl side function, such as the compounds sold by the company Shin-Etsu under the trade name X-22-3701E.

Among the organosiloxanes of formula (XXXVI), mention may be made of polydimethylsiloxanes (PDMS) bearing a carboxyl end function, such as the compounds sold by the company Shin-Etsu under the trade name X-22-3710.

Among the organosiloxanes of formula (XXXVII), mention may be made of the compounds sold by the company Grant Industries under the trade name Grandsil SiW-PCA-10 (INCI name: Dimethicone (and) PCA Dimethicone (and) Butylene Glycol (and) Decyl Glucoside).

The silicone compounds comprising a carboxylic group may correspond, for example, to the compounds described in the patent application EP 186 507 in the name of Chisso Corporation.

Preferably, the silicone compound(s) comprising at least one carboxylic group are chosen from the organopolysiloxanes of formula (XXXIV), the organopolysiloxanes of formula (XXXV) and mixtures thereof.

More preferentially, the silicone compound(s) comprising at least one carboxylic group are chosen from the organopolysiloxanes of formula (XXXIV) below: 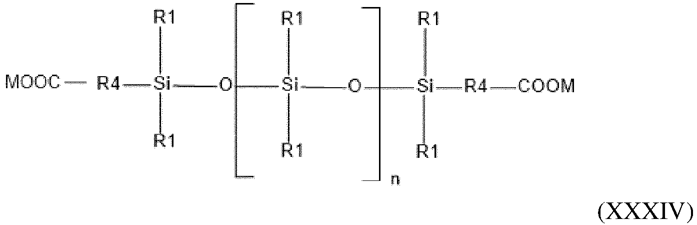 in which:
- **R1** independently represents a linear or branched alkyl group containing from 1 to 20 carbon atoms, preferably from 1 to 10 carbon atoms and better still from 1 to 6 carbon atoms, preferentially methyl;
- R4 independently represents a linear or branched alkylene group containing from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group Rₐ-(OR_{b})ₓ- with **Rₐ** representing a linear or branched alkylene group containing from 1 to 4 carbon atoms, **R_{b}** representing an alkylene group containing from 1 to 4 carbon atoms, and **x** being an integer ranging from 1 to 200;
- **M** independently represents a hydrogen atom, an alkali metal or alkaline-earth metal or a quaternary ammonium NR'₃, with **R',** which may be identical or different, representing H or an alkyl containing from 1 to 4 carbon atoms;
- **n** denotes an integer ranging from 1 to 1000.

Advantageously, the total amount of silicone compound(s) comprising at least one carboxylic group ranges from 0.01% to 20% by weight, preferably from 0.1% to 15% by weight, more preferentially from 0.5% to 10% by weight and better still from 1% to 5% by weight relative to the total weight of composition D.

### Oils

Composition D may comprise one or more oils.

Preferably, composition D comprises one or more oils. More preferentially, composition D comprises one or more oils chosen from alkanes.

The term *"oil"* means a fatty substance that is liquid at room temperature (25°C) and at atmospheric pressure (760 mmHg or 1.013×10⁵ Pa).

The oil may be volatile or non-volatile.

The term *"volatile oil"* refers to an oil that can evaporate on contact with the skin in less than one hour, at room temperature and atmospheric pressure. The volatile oil is a cosmetic volatile oil, which is liquid at room temperature. More specifically, a volatile oil has an evaporation rate of between 0.01 and 200 mg/cm²/min, limits included (see protocol for measuring the evaporation rate indicated in the text below).

The term *"non-volatile oil"* refers to an oil that remains on the skin or the keratin fibre at room temperature and atmospheric pressure. More specifically, a non-volatile oil has an evaporation rate of strictly less than 0.01 mg/cm²/min (see protocol for measuring the evaporation rate indicated in the text below).

Preferably, the composition comprises one or more oils chosen from C₆-C₁₆ alkanes and/or mixtures thereof.

As regards the C₆-C₁₆ alkanes, they may be linear or branched, and possibly cyclic. Mention may notably be made of branched C₈-C₁₆ alkanes, such as C₈-C₁₆ isoalkanes (also known as isoparaffins), isododecane, isodecane or isohexadecane, and for example the oils sold under the Isopar or Permethyl trade names, and mixtures thereof.

Mention may also be made of linear alkanes, preferably of plant origin, comprising from 7 to 15 carbon atoms, in particular from 9 to 14 carbon atoms and more particularly from 11 to 13 carbon atoms.

As examples of linear alkanes that are suitable for use in the invention, mention may be made of n-heptane (C7), n-octane (C8), n-nonane (C9), n-decane (C10), n-undecane (C11), n-dodecane (C12), n-tridecane (C13), n-tetradecane (C14) and n-pentadecane (C15), and mixtures thereof, and in particular the mixture of n-undecane (C11) and n-tridecane (C13) described in Example 1 of patent application WO 2008/155 059 by the company Cognis.

Mention may also be made of n-dodecane (C12) and n-tetradecane (C14) sold by Sasol under the references, respectively, Parafol 12-97 and Parafol 14-97, and also mixtures thereof.

As examples of alkanes that are suitable for use in the invention, mention may be made of the alkanes described in patent applications WO 2007/068 371 and WO 2008/155 059. These alkanes are obtained from fatty alcohols, which are themselves obtained from coconut kernel oil or palm oil.

According to a particular embodiment, the composition comprises isododecane. Such a compound is, for example, the isododecane sold under the reference Isododecane by Ineos.

Preferably, composition D comprises one or more oils chosen from C₈-C₁₆ alkanes, more preferentially from isododecane, isohexadecane, tetradecane and/or mixtures thereof. More preferentially, composition D comprises isododecane.

Composition D may comprise one or more oils present in a total amount of between 30% and 99% by weight, preferably between 50% and 99% by weight and better still between 70% and 99% by weight, relative to the total weight of composition D.

Composition D may comprise at least one colouring agent chosen from pigments, direct dyes and mixtures thereof as described previously.

### Protocol

Composition C and the optional composition D described above may be used on wet or dry keratin hair fibers, and also on any type of fair or dark, natural or dyed, permanent-waved, bleached or relaxed keratin hair fibers.

According to a preferred embodiment, composition C and composition D are applied simultaneously to the keratin fibres.

According to another preferred embodiment, composition D is applied to the keratin hair fibers after applying composition C to the keratin hair fibers.

According to another preferred embodiment, composition D is applied to the keratin hair fibers before applying composition C to the keratin hair fibers.

More preferentially, composition D is applied to the keratin hair fibers after applying composition C to the keratin hair fibers.

According to a particular embodiment of the invention, the keratin hair fibers is washed before applying composition C and composition D.

Preferably, a washing, rinsing, draining or drying step is performed after applying composition C to the keratin hair fibers and optionally before applying composition D to the keratin hair fibers.

More preferentially, a drying step is performed after applying composition C to the keratin hair fibers and optionally before applying composition D to the keratin hair fibers.

The application to the keratin hair fibers may be performed via any conventional means, in particular using a comb, a fine brush, a coarse brush, a sponge or with the fingers.

The application of composition C and/or of composition C followed by composition D to the keratin hair fibers is generally performed at room temperature (between 15 and 25°C). After applying composition C to the keratin hair fibers, it is possible to wait for between 1 minute and 6 hours, in particular between 1 minute and 2 hours, more particularly between 1 minute and 1 hour, more preferentially between 1 minute and 30 minutes, before, for example, applying composition D to the keratin hair fibers or, for example, a washing, rinsing, draining or drying step.

Preferably, there is no leave-on time after applying composition C to the keratin hair fibers and before applying composition D to the keratin hair fibers.

After applying composition C and/or of composition C followed by composition D, the keratin hair fibers may be left to dry or may be dried, for example at a temperature of greater than or equal to 30°C.

The process according to the invention may thus comprise a step of applying heat to the keratin fibres using a heating tool.

The heat application step of the process of the invention may be performed using a hood, a hair dryer, a straightening iron, a curling iron, a Climazon hood, etc.

Preferably, the heat application step of the process of the invention is performed using a hair dryer.

When the process of the invention involves a step of applying heat to the keratin hair fibers, the step of applying heat to the keratin hair fibers takes place after applying composition C and the optional composition D to the keratin hair fibers.

During the step of applying heat to the keratin hair fibers, a mechanical action may be exerted on the locks, such as combing, brushing or running the fingers through.

When the step of applying heat to the keratin hair fibers is performed using a hood or a hair dryer, the temperature is preferably between 30°C and 110°C, preferentially between 50°C and 90°C.

When the step of applying heat to the keratin hair fibers is performed using a straightening iron, the temperature is preferably between 110°C and 220°C, preferably between 140°C and 200°C.

In a particular variant, the process of the invention involves a step (cl) of applying heat using a hood, a hair dryer or a Climazon hood, preferably a hair dryer, and a step (c2) of applying heat using a straightening or curling iron, preferably a straightening iron.

Step (c1) may be performed before step (c2).

During step (c1), also referred to as the drying step, the keratin hair fibers may be dried, for example at a temperature above or equal to 30°C. According to a particular embodiment, this temperature is above 40°C. According to a particular embodiment, this temperature is above 45°C and below 110°C.

Preferably, if the keratin hair fibers is dried, it is dried, in addition to a supply of heat, with a flow of air. This flow of air during drying makes it possible to improve the strand separation of the coating.

During drying, a mechanical action may be exerted on the locks, such as combing, brushing or running the fingers through.

During step (c2), the passage of the straightening or curling iron, preferably the straightening iron, may be performed at a temperature ranging from 110°C to 220°C, preferably between 140°C and 200°C.

After the heating step, a shaping step may be performed, for example with a straightening iron; the temperature for the shaping step is between 110 and 220°C, preferably between 140 and 200°C.

Preferably, the invention is a process for dyeing keratin hair fibers comprising the following steps:
i) the application to the keratin hair fibers of at least one composition C comprising:
   a) at least one (poly)carbodiimide compound as described previously;
   b) at least one compound containing at least one carboxylic acid group, as described previously;
   c) at least one amino silicone of formula (XIII) as described previously; and
   d) at least one colouring agent chosen from pigments, direct dyes and mixtures thereof; and then
ii) optionally a leave-on time of said composition C on the keratin hair fibers of from 1 minute to 30 minutes, preferably from 1 to 20 minutes; and then
iii) optionally a step of washing, rinsing, draining or drying said keratin hair fibers; and then
iv) optionally the application to the keratin hair fibers of at least one composition D comprising at least one silicone compound comprising at least one carboxylic group as described previously; and then
v) optionally a leave-on time of said composition D on the keratin hair fibers of from 1 minute to 30 minutes, preferably from 1 to 20 minutes; and then
vi) optionally a step of washing, rinsing, draining or drying said keratin hair fibers.

Preferably, the step of applying composition C to the keratin hair fibers is repeated several times.

Advantageously, the dyeing process comprises a step iv) of applying to the keratin hair fibers a composition D comprising at least one silicone compound comprising at least one carboxylic group as described previously.

According to a preferred embodiment, the dyeing process according to the invention is a process for dyeing keratin hair fibers which consists in extemporaneously mixing, at the time of use, at least two compositions A and B to obtain a composition C and in applying composition C to the keratin hair fibers, with:
- composition A comprising at least one (poly)carbodiimide compound as described previously;
- composition B comprising at least one compound containing at least one carboxylic acid group, as described previously;

composition A and/or composition B comprising at least one colouring agent chosen from pigments, direct dyes and mixtures thereof; and
composition A and/or composition B comprising at least one amino silicone of formula (XIII) as described previously.

Advantageously, the dyeing process also comprises the application to the keratin hair fibers of at least one composition D comprising at least one silicone compound comprising at least one carboxylic group as described previously, said composition D being applied to the keratin hair fibers before and/or after the application of composition C to the keratin hair fibers.

Preferably, composition B comprises at least one colouring agent chosen from pigments, direct dyes and mixtures thereof.

Preferably, composition A does not comprise at least one colouring agent chosen from pigments, direct dyes and mixtures thereof.

According to this embodiment, compositions A and B are mixed preferably less than 15 minutes before application to the keratin hair fibers, more preferentially less than 10 minutes before application to the keratin hair fibers, better still less than 5 minutes before application to the keratin hair fibers.

The weight ratio between composition A and composition B preferably ranges from 0.1 to 10, preferentially from 0.2 to 5 and better still from 0.5 to 2, or even from 0.6 to 1.5.

In a particular embodiment, the weight ratio between composition A and composition B is equal to 1.

According to a preferred embodiment, the dyeing process according to the invention is a process for dyeing keratin hair fibers which consists in extemporaneously mixing, at the time of use, at least two compositions A and B to obtain a composition C and in applying composition C to the keratin hair fibers, with:
- composition A comprising at least one (poly)carbodiimide compound as described previously; and
- composition B comprising at least one compound containing at least one carboxylic acid group as described previously, and at least one colouring agent chosen from pigments, direct dyes, and mixtures thereof;
   composition A and/or composition B comprising at least one amino silicone of formula (XIII) as described previously, and
- optionally, the application to the keratin hair fibers of at least one composition D comprising at least one silicone compound comprising at least one carboxylic group as described previously, said composition D being applied to the keratin hair fibers before and/or after the application of composition C to the keratin hair fibers.

According to a preferred embodiment, the dyeing process according to the invention is a process for dyeing keratin hair fibers which consists in extemporaneously mixing, at the time of use, at least two compositions A and B to obtain a composition C and in applying composition C to the keratin hair fibers, with:
- composition A comprising at least one (poly)carbodiimide compound as described previously; and
- composition B comprising at least one compound containing at least one carboxylic acid group as described previously, and at least one colouring agent chosen from pigments, direct dyes, and mixtures thereof; and at least one amino silicone of formula (XIII) as described previously; and
- optionally, the application to the keratin hair fibers of at least one composition D comprising at least one silicone compound comprising at least one carboxylic group as described previously, said composition D being applied to the keratin hair fibers before and/or after the application of composition C to the keratin hair fibers.

The total amount of the (poly)carbodiimide compound(s) preferably ranges from 0.01% to 40% by weight, more preferentially from 0.1% to 30% by weight, better still from 0.5% to 20% by weight and even more preferentially from 1% to 12% by weight relative to the total weight of composition A.

The total amount of the compound(s) containing at least one carboxylic acid group preferably ranges from 2% to 40% by weight, more preferentially from 5% to 30% by weight and better still from 5% to 20% by weight relative to the total weight of composition B.

The amino silicone(s) of formula (XIII) as described previously may be present in a total amount ranging from 0.01% to 20%, preferably from 0.05% to 15%, more preferentially from 0.1% to 10% and even more preferentially from 0.5% to 5% by weight relative to the total weight of composition B.

### Multi-compartment device (kit)

The present invention also relates to a device for dyeing the keratin hair fibers, comprising one or more compartments containing:
- in a first compartment, a composition C comprising:
   a) at least one (poly)carbodiimide compound as described previously;
   b) at least one compound containing at least one carboxylic acid group, as described previously;
   c) at least one amino silicone of formula (XIII) as described previously; and
   d) at least one colouring agent chosen from pigments, direct dyes and mixtures thereof, and
- optionally, in a second compartment, a composition D comprising at least one silicone compound comprising at least one carboxylic group as described previously.

The present invention also relates to a device for dyeing the keratin hair fibers, comprising several compartments containing:
- in a first compartment, a composition A comprising:
   a) at least one (poly)carbodiimide compound as described previously;
- in a second compartment, a composition B comprising:
   b) at least one compound containing at least one carboxylic acid group as described previously; and
   composition A and/or composition B comprising c) at least one amino silicone of formula (XIII) as described previously;
   composition A and/or composition B comprising d) at least one colouring agent chosen from pigments, direct dyes and mixtures thereof; and
- optionally, in a third compartment, a composition D comprising at least one silicone compound comprising at least one carboxylic group as described previously.

According to a preferred embodiment, the device according to the invention is a device for dyeing the keratin hair fibers, comprising several compartments containing:
- in a first compartment, a composition A comprising:
   a) at least one (poly)carbodiimide compound as described previously;
- in a second compartment, a composition B comprising:
   b) at least one compound containing at least one carboxylic acid group as described previously, and d) at least one colouring agent chosen from pigments, direct dyes, and mixtures thereof; and
   composition A and/or composition B comprising c) at least one amino silicone of formula (XIII) as described previously; and
- optionally, in a third compartment, a composition D comprising at least one silicone compound comprising at least one carboxylic group as described previously.

According to a preferred embodiment, the device according to the invention is a device for dyeing the keratin hair fibers, comprising several compartments containing:
- in a first compartment, a composition A comprising:
   a) at least one (poly)carbodiimide compound as described previously;
- in a second compartment, a composition B comprising:
   b) at least one compound containing at least one carboxylic acid group as described previously, c) at least one amino silicone of formula (XIII) as described previously, and d) at least one colouring agent chosen from pigments, direct dyes, and mixtures thereof; and
- optionally, in a third compartment, a composition D comprising at least one silicone compound comprising at least one carboxylic group as described previously.

The present invention will now be described more specifically by means of examples, which do not in any way limit the scope of the invention. However, the examples make it possible to support specific characteristics, variants and preferred embodiments of the invention.

### EXAMPLES

The (poly)carbodiimide(s) of the invention are accessible via synthetic methods known to a person skilled in the art starting from commercial products or reagents that can be synthesized according to chemical reactions that are also known to a person skilled in the art. Mention may be made, for example, of the book Sciences of Synthesis - Houben - Weyl Methods of Molecular Transformations, 2005, Georg Thiem Verlag Kg, Rudigerstrasse 14, D-70469 Stuttgart, or the American patent US 4 284 730 or the Canadian patent application CA 2 509 861.

More particularly, the process for preparing the (poly)carbodiimides of the invention involves, in a first step, a diisocyanate reagent (1):

O=C=N-L₁-N=C=O (1),

in which formula (1) L₁ is as defined previously, which reacts in the presence of a carboimidation catalyst (2) such as those described in US 4 284 730, notably phosphorus-based catalysts particularly chosen from phospholene oxides and phospholene sulfoxides, diaza- and oxaza-phospholanes, preferably under an inert atmosphere (nitrogen or argon), and in particular in a polar solvent which is preferably aprotic such as THF, glyme, diglyme, 1,4-dioxane or DMF, at a temperature between room temperature and the reflux temperature of the solvent, preferably at about 140°C; to give the carbodiimide diisocyanate compound (3):

O=C=N-L₁-(N=C=N-L₁)ₙ-N=C=O (3),

in which formula (3) L₁ and n are as defined previously. Benzoyl halogen such as benzoyl chloride may be added to deactivate the catalyst.

To obtain "symmetrical" (poly)carbodiimides, during the second step of the preparation process, compound (3) reacts with 1 molar equivalent (1 eq.) of nucleophilic reagent R₁-X₁-H and then 0.5 eq. of reagent H-E-H with R₁, X₁ and E as defined previously, to give the "symmetrical" compound (4) according to the invention:

[R₁-X₁-C(O)-NH-L₁-(N=C=N-L₁)ₙ-NH-C(O)]₂-E (4),

in which formula (4) R₁, X₁, L₁, n and E are as defined previously. According to one variant to obtain compound (4) from (3), it is possible first to add 0.5 eq. of reagent H-EH and then 1 eq. of reagent R₁-X₁-H.

To obtain "dissymmetrical" (poly)carbodiimides, during the second step of the preparation process, compound (3) reacts with 1 molar equivalent (1 eq.) of nucleophilic reagent R₁-X₁-H and then 1 eq. of reagent H-E-H with R₁, X₁ and E as defined previously, to give compound (5):

R₁-X₁-C(O)-NH-L₁-(N=C=N-L₁)ₙ-NH-C(O)-E-H (5),

in which formula (5) R₁, X₁, L₁, n and E are as defined previously.

According to one variant to obtain compound (5) from (3), it is possible first to add 1 eq. of reagent R₁-X₁-H and then 0.5 eq. of reagent H-E-H.

During a third step, compound (5) reacts with 1 eq. of compound (6)

R₂-X₂-C(O)-NH-L₁-(N=C=N-L₁)_{z}-N=C=O (6),

said compound (6) is prepared beforehand from compound (3')

O=C=N-L₁-(N=C=N-L₁)_{z}-N=C=O (3'),

in which formula (3') L₁ and z are as defined previously, which reacts with 1 eq. of nucleophilic reagent R₂-X₂-H with L₁, R₂, X₂ and z as defined previously, to give the dissymmetrical compound (7):

R₁-X₁-C(O)-NH-L₁-(N=C=N-L₁)ₙ-NH-C(O)-E-C(O)-NH-L₁-(N=C=N-L₁)_{z}-NH-C(O)- X₂-R₂ (7),

in which formula (7) R₁, X₁, L₁, R₂, X₂, n, z and E are as defined previously.

It is also possible to react 1 molar equivalent of compound O=C=N-L₁-(N=C=N-L₁)_{z}-N=C=O (3') with 1/w molar equivalent of H-E-H, followed by 1 eq. of nucleophilic reagent R₂-X₂-H to give compound (8):

H-[E-C(O)-NH-L₁-(N=C=N-L₁)_{z}]_{w}-NH-C(O)-X₂-R₂ (8),

in which formula (8) L₁, R₂, X₂, z and E are as defined previously, and w is an integer between 1 and 3; more preferentially, w = 1.

This last compound (8) can then react with 1 eq. of compound (4'):

R₁-X₁-C(O)-NH-L₁-(N=C=N-L₁)ₙ-N=C=O (4'),

(said compound (4') being able to be synthesized by reaction of 0.5 eq. of nucleophilic reagent R₁-X₁-H with 1 equivalent of compound (3)), to give the (poly)carbodiimide (9) of the invention:

R₁-X₁-C(O)-NH-L₁-(N=C=N-L₁)ₙ-NH-C(O)-[E-C(O)-NH-L₁-(N=C=N-L₁)_{z}]_{w}-NH C(O)-X₂-R₂ (9),

in which formula (9) L₁, R₁, X₁, R₂, X₂, n, z, w and E are as defined previously.

The (poly)carbodiimide compounds, and similarly all the reaction intermediates and reagents, may be purified via conventional methods known to those skilled in the art, such as extraction with water and water-immiscible organic solvent, precipitation, centrifugation, filtration and/or chromatography.

### Example 1: Process for synthesizing the (poly)carbodiimide compound

50 g of 4,4'-dicyclohexylmethane diisocyanate and 0.5 g of 4,5-dihydro-3-methyl-1-phenyl-1H-phosphole 1-oxide were placed with stirring in a 500 mL three-necked roundbottomed flask equipped with a thermometer, a stirrer and a reflux tube.

The reaction medium was heated at 140°C under nitrogen for 4 hours, the reaction being monitored by infrared spectroscopy by means of the absorption of the isocyanate functions between 2200 and 2300 cm⁻¹, and then cooled to 120°C.

A mixture of 5.3 g of polyethylene glycol monomethyl ether and 1.2 g of 1,4-butanediol are introduced with stirring into the reaction medium. The temperature of 120°C is maintained until the isocyanate functions have totally disappeared, monitored by infrared spectroscopy at 2200-2300 cm⁻¹, and is then cooled to room temperature.

After cooling to room temperature, the reaction medium is poured dropwise with vigorous stirring into a 500 mL glass beaker containing 85 g of distilled water, to give the desired product in the form of a translucent yellow liquid.

### Example 2: Process for synthesizing the amino silicone of formula (XIII)

1.1 stoichiometric equivalents of diethyl L-tartrate are added to an aqueous emulsion containing 35% of amodimethicone bearing 3-(2-aminoethylamino)propyl functional groups.

The mixture obtained is homogenized at room temperature, giving a white, smooth, low-viscosity silicone emulsion.

### Example 3:

The compositions as described below were prepared: the amounts are expressed as g of starting material as obtained/100 g, unless otherwise mentioned. In Table 2 below, "a.m." means "active material".

**[Table 1]**

| **Composition** | **A** |
|---|---|
| Polycarbodiimide⁽¹⁾ | 17.5 |
| Hydroxyethyl acrylate/sodium acrvlovldimethvl taurate copolymer ⁽²⁾ | 3 |
| Ethanol | 20 |
| Water | qs 100 |

| | |
|---|---|
| (1) synthesized according to the synthetic process described in Example 1 (containing 40% active material in water), (2) sold by the company SEPPIC under the name Sepinov EMT10 (containing 90% active material), | |

**[Table 2]**

| **Compositions** | **B1** | **B2** | **B3** |
|---|---|---|---|
| Acrylates copolymer ⁽³⁾ | 29.2 | 29.2 | 29.2 |
| Acrylates/Steareth-20 methacrylate copolymer ⁽⁴⁾ | 2.2 | 2.2 | 2.2 |
| Triethanolamine | 0.4 | 0.4 | 0.4 |
| Yellow iron oxide (CI 77492) | 2.7 | 2.7 | 2.7 |
| Mica and red iron oxide (CI 77491) | 9.3 | 9.3 | 9.3 |
| Amodimethicone (and) Trideceth-5 (and) Trideceth-10 ⁽⁵⁾ | 4.6 a.m. | | - |
| Amino silicone of formula (XIII)⁽⁶⁾ | - | 4.6 a.m. | 1.5 a.m. |
| Divinyl dimethicone/dimethicone (and) C₁₂-₁₃ Pareth-3 (and) C₁₂-₁₃ Pareth-23 copolymer⁽⁷⁾ | - | | 3.1 a.m. |
| Ethanol | 3.8 | 3.8 | 3.8 |
| Chlorphenesin | 0.3 | 0.3 | 0.3 |
| p-Hydroxyacetophenone | 0.4 | 0.4 | 0.4 |
| Phenoxyethanol | 0.5 | 0.5 | 0.5 |
| Pentylene glycol | 1.9 | 1.9 | 1.9 |
| 1,2-Hexanediol | 0.8 | 0.8 | 0.8 |
| Water | qs 100 | qs 100 | qs 100 |

| | | | |
|---|---|---|---|
| (3) sold by the company Daito Kasei Kogyo under the trade name Daitosol 3000SLPN-SD (aqueous dispersion containing 30% active material) (4) sold by the company Röhm & Haas under the trade name Aculyn 22^{®} (oxyalkylenated methacrylic acid/ethyl acrylate/stearyl methacrylate terpolymer containing 30% active material) (5) sold by the company Wacker under the name Belsil ADM LOG 1 (containing 15% active material). (6) synthesized according to the synthetic process described in Example 2 (7) sold by the company Dow Corning under the name Dow Corning HMW 2220 Nonionic Emulsion (divinyl dimethicone/dimethicone block copolymer as an aqueous emulsion) (60% active material) | | | |

Three mixtures are then made. Composition A is mixed with each of the compositions B1, B2 and B3 in a 50/50 mass ratio to obtain three compositions C1, C2 and C3.

Thus, the process using composition C1 is a comparative process. The processes using compositions C2 and C3 are processes according to the invention.

Next, composition D as described below was prepared: the amounts are expressed as g of starting material as obtained/100 g.

**[Table 3]**

| **Composition** | **D** |
|---|---|
| Bis-carboxydecyl dimethicone ⁽⁸⁾ | 2 |
| Isododecane | qs 100 |

| | |
|---|---|
| (8) sold by the company Momentive Performance Materials under the trade name Silform INX | |

### Example 4:

### Protocol for evaluating the resistance to shampoo washing:

Each of the compositions C1 and C3 is applied using a small brush to locks of dry natural keratin hair fibers containing 90% white keratin hair fibers strands, in a proportion of 0.8 g of composition per gram of lock. The locks of keratin hair fibers are then disentangled and dried with a hair dryer at medium heat.

Next, composition D is applied to said locks of keratin hair fibers pretreated with compositions C1 and C3, in a proportion of 0.5 g of composition per gram of lock.

The locks of keratin hair fibers are then disentangled and dried with a hair dryer at medium heat, and are then stored at a temperature of 22°C and a humidity of 35% for 24 hours.

The locks of keratin hair fibers thus dyed are then subjected to a test of several repeated shampoo washes so as to evaluate the fastness (persistence) of the colouring obtained with respect to shampoo washing, according to the shampoo washing protocol described below.

### Shampoo washing protocol:

The locks of dyed keratin hair fibers are combed, moistened with water at 35°C and then passed between the fingers five times for 5 seconds. The locks of keratin hair fibers are then squeezed dry between two fingers.

A standard shampoo (Garnier Ultra Doux) is applied uniformly to the dyed locks, in a proportion of 0.4g of standard shampoo per gram of locks, the locks of keratin hair fibers being massaged gently along the length (6 passes) for 15 seconds, from the root to the end.

The locks of keratin hair fibers are then placed on a watch glass and left to stand for 1 minute.

Next, the locks of keratin hair fibers are rinsed with water while passing the lock between the fingers (15 passes). The locks of keratin hair fibers are then squeezed dry between two fingers before the next shampoo wash.

Once the tests of several shampoo washes have been performed, the locks of keratin hair fibers are combed and dried with a hair dryer.

### Results

The persistence of the colour of the locks was evaluated in the CIE L*a*b* system, using a Minolta Spectrophotometer CM3600A colorimeter (illuminant D65, angle 10°, specular component included).

In this L*a*b* system, L* represents the intensity of the colour, a* indicates the green/red colour axis and b* the blue/yellow colour axis.

The persistence of the colouring is evaluated by the colour difference ΔE between the coloured locks before shampoo washing, then after having undergone five shampoo washes according to the protocol described above. The lower the ΔE value, the more persistent the colour with respect to shampoo washing.

The ΔE value is calculated according to the following equation: $ΔE = \sqrt{{\left(L*-{L}_{o}*\right)}^{2} + {\left(a*-{a}_{o}*\right)}^{2} + {\left(b*-{b}_{o}*\right)}^{2}}$

In this equation, L*a*b* represent the values measured after dyeing the keratin hair fibers and after performing the shampoo washes, and L₀*a₀*b₀* represent the values measured after dyeing the keratin hair fibers but before shampoo washing.

**[Table 4]**

| Processes | Number of shampoo washes | L* | a* | b* | ΔE |
|---|---|---|---|---|---|
| C1 + D (Comparative) | 0 | 48.9 | 19.4 | 27.8 | - |
| | 5 | 53.5 | 15.6 | 24.6 | 6.7 |
| C3 + D (Invention) | 0 | 49.6 | 19.8 | 28.7 | - |
| | 5 | 50.9 | 17.6 | 26.6 | 3.3 |

The locks of keratin hair fibers treated by means of the process according to the invention and washed with five shampoo washes have lower ΔE values than those of the locks of keratin hair fibers treated by means of the comparative process.

Thus, the coloured coating that is obtained by means of the process according to the invention shows improved resistance to shampoo washing.

### Example 5:

### Protocol for evaluating the smooth feel and the suppleness:

Each of the compositions C1, C2 and C3 as described in Example 3 is applied to locks of natural chestnut-brown keratin hair fibers (tone height 4), at a rate of 0.8 g of composition per gram of lock. The locks of keratin hair fibers are then dried with a hair dryer at medium heat, while at the same time combing them with a comb.

The keratin hair fibers strands of the locks are then individualized using a blow-drying brush.

The locks of keratin hair fibers are then stored at a temperature of 22°C and a humidity of 35% for 24 hours.

The properties in terms of the smooth feel and the suppleness were then evaluated.

### Evaluation of the smooth feel

The performance in terms of the smooth feel of the locks of dyed keratin hair fibers as prepared previously was evaluated by five experts. The performance was evaluated in a blind test in which each of the five experts assigned a score ranging from 0 (very poor smoothness) to 5 (very good smoothness) to each lock of keratin hair fibers.

To evaluate the smooth feel, the expert takes the lock of keratin hair fibers between thumb and forefinger and slides his or her fingers along the lock from the upper part to the ends. He or she evaluates whether the keratin hair fibers hves any bumps or whether the fingers catch on the keratin hair fibers.

The results obtained are collated in Table 5 below.

**[Table 5]**

| **Process using the composition** | **C1 (comparative)** | **C2 (invention)** | **C3 (invention)** |
|---|---|---|---|
| **Mean** | 3.4 | 4 | 3.9 |

It is clearly seen that locks of keratin hair fibers treated by means of the processes according to the invention have a significantly smoother feel than the locks of keratin hair fibers treated by means of a comparative process.

Thus, the coloured coating that is obtained by means of the processes according to the invention has an improved smooth feel.

### Evaluation of the suppleness

The performance in terms of the suppleness of the locks of dyed keratin hair fibers as prepared previously was evaluated by five experts. The performance was evaluated in a blind test in which each of the five experts assigned a score ranging from 0 (very poor suppleness) to 5 (very good suppleness) to each of the locks of keratin hair fibers.

To evaluate the suppleness property, the expert takes the lock of keratin hair fibers and bends it twice in the palm of his or her hand and squeezes it three times, at a rate of one second per squeeze.

The more easily the keratin hair fibers can be folded or bent, the more supple it is.

The results obtained are collated in Table 6 below.

**[Table 6]**

| **Process using the composition** | **C1 (comparative)** | **C2 (invention)** | **C3 (invention)** |
|---|---|---|---|
| **Mean** | **3.1** | **3.8** | **3.7** |

It is clearly seen that locks of keratin hair fibers treated by means of the processes according to the invention have significantly greater suppleness than the locks of keratin hair fibers treated by means of a comparative process.

Thus, the coloured coating that is obtained by means of the processes according to the invention shows improved suppleness.

## Claims

1. Process for dyeing keratin hair fibers comprising the application to the keratin hair fibers of at least one composition C comprising:
a) at least one (poly)carbodiimide compound;
b) at least one compound containing at least one carboxylic acid group;
c) at least one amino silicone of formula (XIII) below: 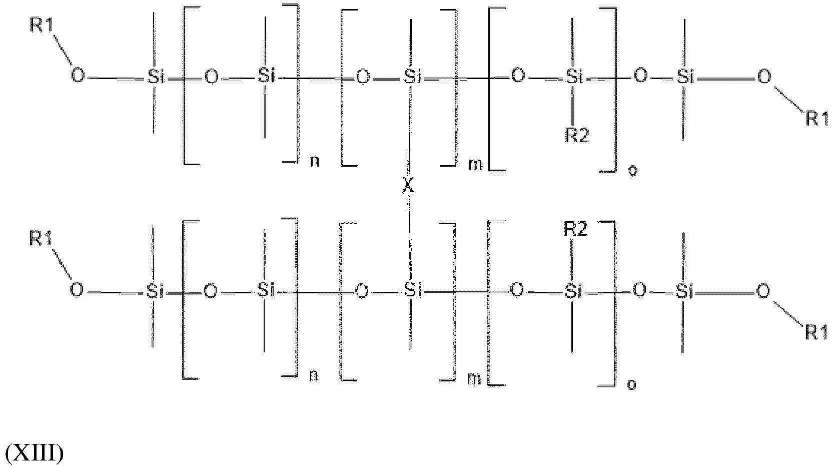 in which:
- **R1** independently represents a hydrogen atom or an alkyl group containing from 1 to 10 carbon atoms, preferably from 1 to 4 carbon atoms;
- **R2** independently represents a monovalent radical of formula -C_{q}H_{2q}L in which q is a number ranging from 2 to 8 and L is an optionally quaternized amine group chosen from the following groups:
-N(R3)₂; -N⁺(R3)₃ A⁻; -NR3-Y-N(R3)₂ and -NR-Y-N⁺(R3)₃ A⁻,
in which **R3,** which may be identical or different, represents a hydrogen atom, a phenyl group, a benzyl group or a saturated monovalent hydrocarbon-based radical, for example a C₁-C₂₀ alkyl group; **Y** denotes a linear or branched group of formula CᵣH₂ᵣ, r being an integer ranging from 2 to 6, preferably from 2 to 4; and **A⁻** represents a cosmetically acceptable anion, notably a halide such as fluoride, chloride, bromide or iodide;
- **X** represents a radical corresponding to formula (XIV) below: 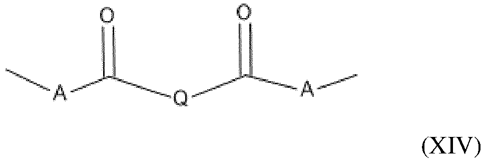
in which:
- **A** independently represents a linear or branched alkylene group containing from 1 to 10 carbon atoms, which may be interrupted with at least one heteroatom chosen from O, S, NH or a carbonyl group (CO), preferably NH; and
- **Q** represents an alkylene group containing from 1 to 20 carbon atoms, said alkylene group being optionally substituted with at least one alkoxy group containing from 1 to 10 carbon atoms, a hydroxyl group or an aminoalkyl group containing from 1 to 20 carbon atoms, and
- **n** denotes an integer ranging from 0 to 500, **m** denotes an integer ranging from 1 to 500 and **o** denotes an integer ranging from 0 to 500 with n+m+o ranging from 250 to 500, and
d) at least one colouring agent chosen from pigments, direct dyes and mixtures thereof.

2. Process according to Claim 1, **characterized in that** the (poly)carbodiimide compound(s) are chosen from the compounds of formula (I) below: 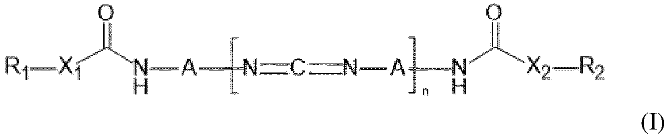 in which:
- X₁ and X₂ independently represent an oxygen atom O, a sulfur atom S or an NH group;
- R₁ and R₂ independently represent a group chosen from a hydrocarbon-based radical, preferably alkyl, optionally interrupted with one or more heteroatoms, a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups, and a hydrocarbon-based radical, preferably alkyl, optionally interrupted with one or more heteroatoms and with one or more groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- n denotes an integer ranging from 1 to 1000; and
- A is a monomer chosen from the compounds below:

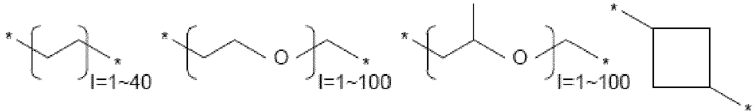

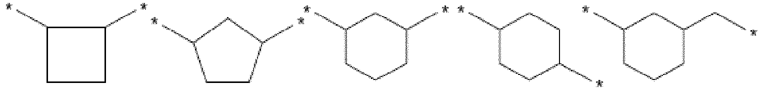

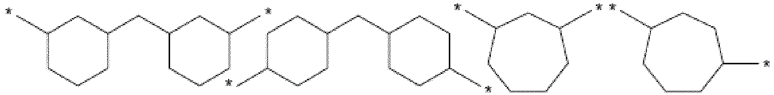

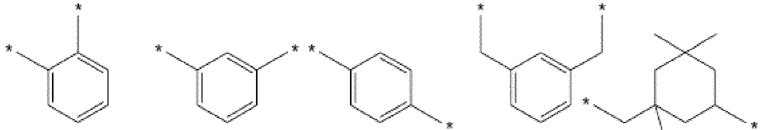

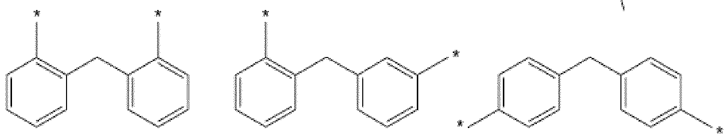

3. Process according to Claim 1, **characterized in that** the (poly)carbodiimide compound(s) are chosen from the compounds of formula (II) below: 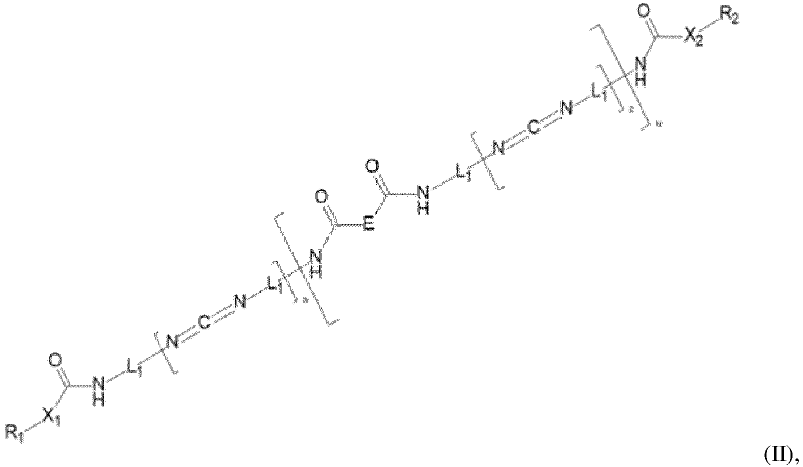 in which:
- X₁ and X₂ independently represent an oxygen atom O, a sulfur atom S or an NH group;
- R₁ and R₂ independently represent a hydrocarbon-based radical optionally interrupted with one or more heteroatoms;
- n and z denote an integer ranging from 1 to 20, with n+z ≥ 2 and w denotes an integer ranging from 1 to 3;
- L₁ independently represents a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:
-O-R₃-O-; -S-R₄-S-; -R₅-N(R₆)-R₄-N(R₆)-R₅-;
in which R₃ and R₄ independently represent a divalent hydrocarbon-based radical optionally interrupted with one or more heteroatoms;
- R₅ independently represents a covalent bond or a saturated divalent hydrocarbon-based radical, optionally interrupted with one or more heteroatoms;
- R₆ independently represents a hydrogen atom or a hydrocarbon-based radical, optionally interrupted with one or more heteroatoms.

4. Process according to Claim 3, **characterized in that** the (poly)carbodiimide compound(s) are chosen from the compounds of formula (II) in which:
- X₁ and X₂ independently represent an oxygen atom;
- R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof;
- n and z denote an integer ranging from 1 to 20, with n+z ≥ 2 and w is equal to 1;
- L1 is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:
-O-R₃-O-; -S-R₄-S-; -R₅-N(R₆)-R₄-N(R₆)-R₅-;
in which R3 and R4 are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

5. Process according to Claim 3 or 4, **characterized in that** the (poly)carbodiimide compound(s) are chosen from the compounds of formula (II) in which:
- X₁ and X₂ independently represent an oxygen atom;
- R₁ and R₂ are, independently, monoalkyl ethers of (poly)alkylene glycol, in which a hydroxyl group has been removed;
- n and z denote an integer ranging from 1 to 20, with n+z ≥ 2 and w is equal to 1;
- L₁ is a C₃-C₁₅ cycloalkylene radical;
- E independently represents a group chosen from:
-O-R₃-O-; -S-R₄-S-; -R₅-N(R₆)-R₄-N(R₆)-R₅-;
in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

6. Process according to any one of Claims 3 to 5, **characterized in that** the (poly)carbodiimide compound(s) are chosen from the compounds of formula (II) in which:
- X₁ and X₂ independently represent an oxygen atom;
- R₁ and R₂ independently represent the compound of formula (VI) below:
R₁₃-[O-CH₂-C(H)(R₁₄)]_{q}- (VI),
in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane; and
- E represents a group -O-R₃-O- in which R₃ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

7. Process according to any one of Claims 3 to 6, **characterized in that** the (poly)carbodiimide compound(s) are chosen from the compounds of formula (II) in which:
- X₁ and X₂ independently represent an oxygen atom;
- R₁ and R₂ independently represent the compound of formula (VI) below:
R₁₃-[O-CH₂-C(H)(R₁₄)]_{q}- (VI),
in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane, preferably 4,4-dicyclohexylenemethane; and
- E represents a group -O-R₃-O- in which R₃ represents a linear or branched C₁-C₁₈ alkylene radical such as methylene, propylene, butylene or ethylene, optionally interrupted with one or more heteroatoms.

8. Process according to any one of Claims 3 to 7, **characterized in that** the (poly)carbodiimide compound(s) are chosen from the compounds of formula (XII) below: 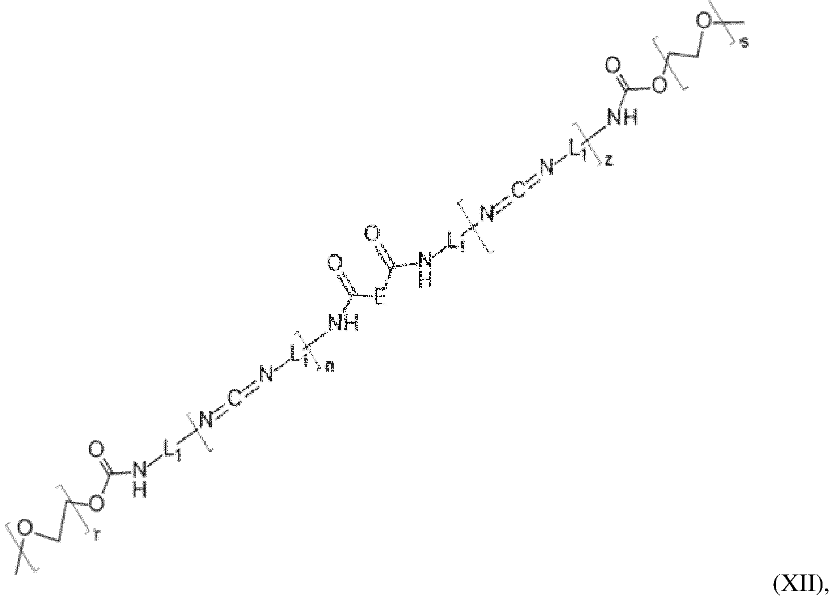 in which L₁ is 4,4-dicyclohexylenemethane, n and z denote an integer ranging from 1 to 20, with n+z ranging from 4 to 10, E represents a group -O-R₃-O- in which R₃ represents a linear or branched C₁-C₁₈ alkylene radical such as methylene, propylene, butylene or ethylene, optionally interrupted with one or more heteroatoms, and r and s denote an integer ranging from 4 to 30.

9. Process according to any one of the preceding claims, **characterized in that** the total amount of the (poly)carbodiimide compound(s) ranges from 0.01% to 20% by weight, preferably from 0.1% to 15% by weight, more preferentially from 0.2% to 10% by weight, even more preferentially from 0.5% to 8% by weight, and better still from 1% to 6% by weight, relative to the total weight of composition C.

10. Process according to any one of the preceding claims, **characterized in that** the compound(s) containing at least one carboxylic acid group are chosen from silicone compounds comprising at least one carboxylic group, polyurethanes, acrylic polymers and mixtures thereof, preferably from polyurethanes, acrylic polymers and mixtures thereof.

11. Process according any one of the preceding claims, **characterized in that** the compound(s) containing at least one carboxylic acid group are in the form of aqueous dispersions of particles of polymer(s) chosen from polyurethanes, acrylic polymers and mixtures thereof, preferably in the form of aqueous dispersions of acrylic polymer particles, more preferentially in the form of aqueous dispersions of film-forming acrylic polymer particles.

12. Process according to any one of the preceding claims, **characterized in that** the total amount of the compound(s) containing at least one carboxylic acid group ranges from 0.1% to 30% by weight, more preferentially from 0.5% to 20% by weight, better still from 0.5% to 15% by weight and even more preferentially from 1% to 10% by weight, relative to the total weight of composition C.

13. Process according to any one of the preceding claims, **characterized in that** the amino silicone(s) of formula (XIII) are such that:
- **R1** independently represents a hydrogen atom or an alkyl group containing from 1 to 10 carbon atoms, preferably from 1 to 4 carbon atoms;
- **R2** independently represents a monovalent radical of formula -C_{q}H_{2q}L in which q is a number ranging from 2 to 8 and L is an optionally quaternized amine group chosen from the following groups:
-N(R3)₂;
-N(R3)-CH₂-CH₂-N(R3)₂;
in which R3, which may be identical or different, represents a hydrogen atom, a phenyl group, a benzyl group or a saturated monovalent hydrocarbon-based radical, for example a C₁-C₂₀ alkyl group;
- X represents a radical corresponding to formula (XIV) below: 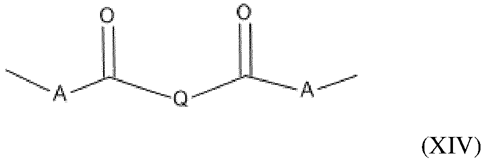 in which:
- A independently represents a linear or branched alkylene group containing from 1 to 6 carbon atoms, which is interrupted with at least one heteroatom chosen from O, S, NH or a carbonyl group (CO), preferably NH; and
- **Q** represents an alkylene group containing from 1 to 10 carbon atoms, said alkylene group being optionally substituted with at least one alkoxy group containing from 1 to 10 carbon atoms, a hydroxyl group or an aminoalkyl group containing from 1 to 20 carbon atoms, and
- **n** denotes an integer ranging from 0 to 500, **m** denotes an integer ranging from 1 to 500 and **o** denotes an integer ranging from 0 to 500 with n+m+o ranging from 250 to 500.

14. Process according to any one of the preceding claims, **characterized in that** the amino silicone(s) of formula (XIII) are such that:
- **R1** independently represents a hydrogen atom or an alkyl group containing from 1 to 10 carbon atoms, preferably from 1 to 4 carbon atoms;
- **R2** independently represents a monovalent radical of formula -C_{q}H_{2q}L in which q is a number ranging from 2 to 8 and L is an amine group having the following formula: -N(R3)-CH₂-CH₂-N(R3)₂;
in which **R3** represents a hydrogen atom,
- **X** represents a radical corresponding to formula (XV) below: 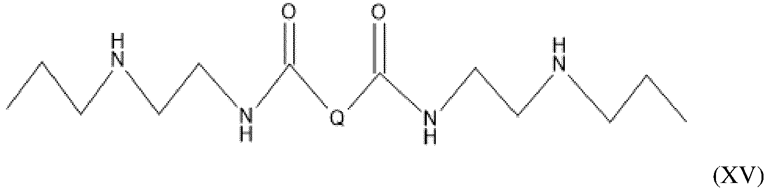 in which:
- **Q** represents an alkylene group containing from 1 to 10 carbon atoms, said alkylene group being optionally substituted with at least one alkoxy group containing from 1 to 10 carbon atoms, a hydroxyl group or an aminoalkyl group containing from 1 to 20 carbon atoms, and
- **n** denotes an integer ranging from 0 to 500, **m** denotes an integer ranging from 1 to 500 and **o** denotes an integer ranging from 0 to 500 with n+m+o ranging from 250 to 500.

15. Process according to any one of the preceding claims, **characterized in that** the amino silicone of formula (XIII) is of formula (XVI) below: 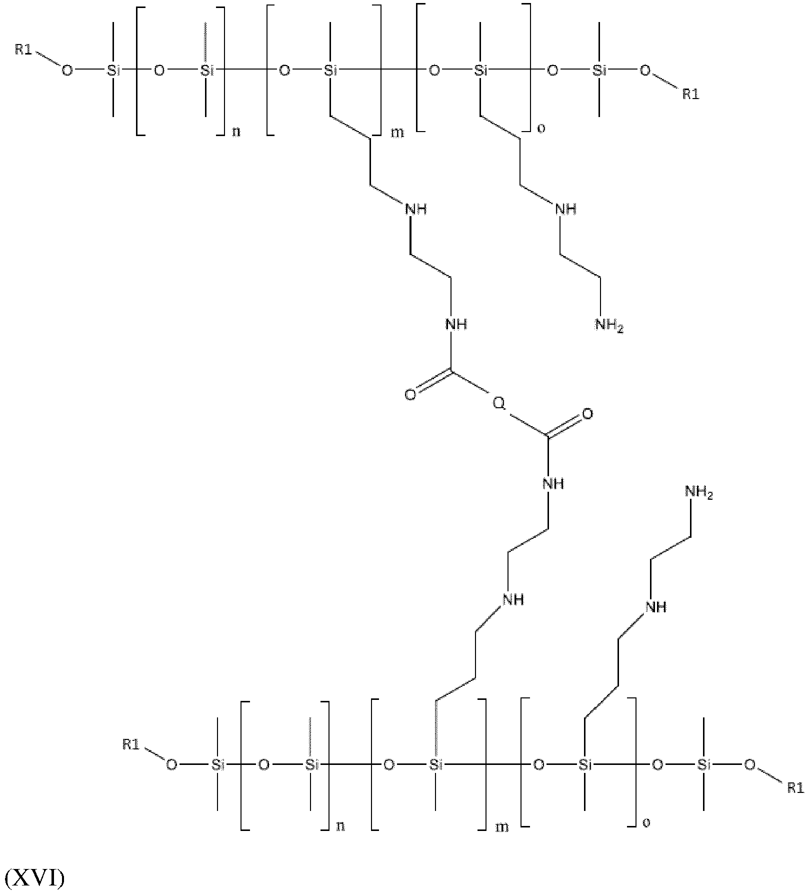 in which:
- **R1** independently represents a hydrogen atom or an alkyl group containing from 1 to 10 carbon atoms, preferably from 1 to 4 carbon atoms;
- **Q** represents an alkylene group containing from 1 to 10 carbon atoms, said alkylene group being optionally substituted with at least one alkoxy group containing from 1 to 10 carbon atoms, a hydroxyl group or an aminoalkyl group containing from 1 to 20 carbon atoms, and
- **n** denotes an integer ranging from 0 to 500, **m** denotes an integer ranging from 1 to 500 and **o** denotes an integer ranging from 0 to 500 with n+m+o ranging from 250 to 500.

16. Process according to any one of the preceding claims, **characterized in that** the amino silicone(s) of formula (XIII) are present in a total amount ranging from 0.01% to 20%, preferably from 0.05% to 15%, more preferentially from 0.1% to 10% and even more preferentially from 0.25% to 8% by weight relative to the total weight of composition C.

17. Process according to any one of the preceding claims, **characterized in that** the composition comprises one or more block silicone copolymers, preferably obtained from dimethylvinylsiloxy-polydimethylsiloxane (or divinyl dimethicone), and the compound of formula (XVIII): 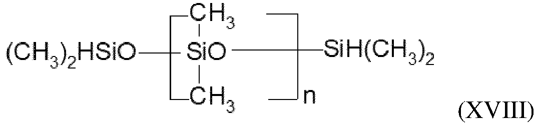 where n is an integer greater than 1 and preferably greater than 10, for example ranging from 10 to 30, preferably with n = 20, preferably in the form of a particle dispersion obtained in the presence of C₁₂-C₁₃ Pareth-3 and C₁₂-C₁₃ Pareth-23 as emulsifiers.

18. Process according to Claim 17, **characterized in that** the block silicone copolymer(s) are present in a total amount ranging from 0.01% to 15%, preferably from 0.05% to 10%, more preferentially from 0.1% to 8% and even more preferentially from 0.25% to 5% by weight relative to the total weight of composition C.

19. Process according to any one of the preceding claims, **characterized in that** it also comprises a step of applying to the keratin hair fibers a composition D comprising at least one silicone compound comprising at least one carboxylic group.

20. Process according to Claim 19, **characterized in that** the total amount of silicone compound(s) comprising at least one carboxylic group ranges from 0.01% to 20% by weight, preferably from 0.1% to 15% by weight, more preferentially from 0.5% to 10% by weight and better still from 1% to 5% by weight relative to the total weight of composition D.

21. Process according to Claim 19 or 20, **characterized in that** composition D also comprises one or more oil(s), preferably chosen from C₈-C₁₆ alkanes, more preferentially from isododecane, isohexadecane, tetradecane and/or mixtures thereof.

22. Process according to any one of Claims 1 to 18, **characterized in that** it consists in extemporaneously mixing, at the time of use, at least two compositions A and B to obtain a composition C and in applying composition C to the keratin hair fibers, with:
- composition A comprising at least one (poly)carbodiimide compound as defined according to any one of Claims 1 to 8;
- composition B comprising at least one compound containing at least one carboxylic acid group as defined according to any one of Claims 1, 10 and 11,
composition A and/or composition B comprising at least one amino silicone of formula (XIII) as defined according to any one of Claims 1 and 13 to 15;
composition A and/or composition B comprising at least one colouring agent chosen from pigments, direct dyes and mixtures thereof.

23. Device for dyeing the keratin hair fibers, comprising several compartments containing:
- in a first compartment, a composition A comprising:
a) at least one (poly)carbodiimide compound as defined according to any one of Claims 1 to 8,
- in a second compartment, a composition B comprising:
b) at least one compound containing at least one carboxylic acid group as defined according to any one of Claims 1, 10 and 11,
composition A and/or composition B comprising:
c) at least one amino silicone of formula (XIII) as defined according to any one of Claims 1 and 13 to 15; and
composition A and/or composition B comprising:
d) at least one colouring agent chosen from pigments, direct dyes and mixtures thereof, and
- optionally, in a third compartment, a composition D comprising at least one silicone compound comprising at least one carboxylic group as defined according to any one of Claims 19 to 21.

## Patentansprüche

1. Verfahren zum Färben von Keratin-Haarfasern, umfassend Aufbringen auf die Keratin-Haarfasern wenigstens einer Zusammensetzung C umfassend:
a) wenigstens eine (Poly)carbodiimidverbindung;
b) wenigstens eine Verbindung, die wenigstens eine Carbonsäuregruppe enthält;
c) wenigstens ein Aminosilicon der folgenden Formel (XIII): 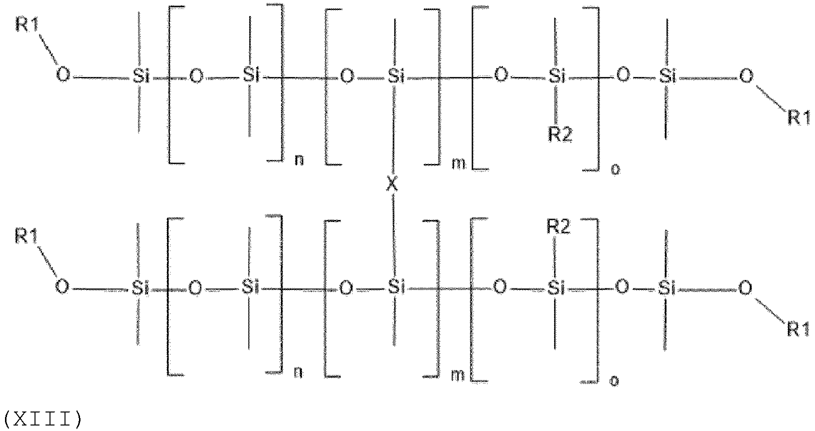 wobei:
- R1 unabhängig ein Wasserstoffatom oder eine Alkylgruppe mit von 1 bis 10 Kohlenstoffatomen, vorzugsweise von 1 bis 4 Kohlenstoffatomen, darstellt;
- R2 unabhängig einen einwertigen Rest der Formel -C_{q}H_{2q}L darstellt, wobei q eine Zahl in dem Bereich von 2 bis 8 ist und L eine gegebenenfalls quaternisierte Amingruppe ausgewählt aus den folgenden Gruppen ist:
- N(R3)₂; -N⁺(R3)₃A⁻; -NR3-Y-N(R3)₂ und -NR-Y-N⁺(R3)₃ A⁻, wobei R3, das gleich oder verschieden sein kann, ein Wasserstoffatom, eine Phenylgruppe, eine Benzylgruppe oder einen gesättigten einwertigen Rest auf Kohlenwasserstoffbasis, beispielsweise eine C₁-C₂₀-Alkylgruppe, darstellt; Y eine lineare oder verzweigte Gruppe der Formel CᵣH₂ᵣ bezeichnet, r eine ganze Zahl in dem Bereich von 2 bis 6, vorzugsweise von 2 bis 4, ist und A⁻ ein kosmetisch annehmbares Anion, insbesondere ein Halogenid, wie z.B. Fluorid, Chlorid, Bromid oder Iodid, darstellt;
- X einen Rest der folgenden Formel (XIV) darstellt: 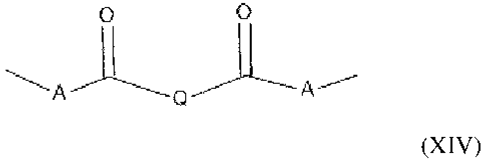 wobei:
- A unabhängig eine lineare oder verzweigte Alkylengruppe mit von 1 bis 10 Kohlenstoffatomen darstellt, die von wenigstens einem Heteroatom ausgewählt aus O, S, NH oder einer Carbonylgruppe (CO), vorzugsweise NH, unterbrochen sein kann; und
- Q eine Alkylengruppe mit von 1 bis 20 Kohlenstoffatomen darstellt, die gegebenenfalls mit wenigstens einer Alkoxygruppe mit von 1 bis 10 Kohlenstoffatomen, einer Hydroxygruppe oder einer Aminoalkylgruppe mit von 1 bis 20 Kohlenstoffatomen substituiert ist, und
- n eine ganze Zahl in dem Bereich von 0 bis 500 bezeichnet, m eine ganze Zahl in dem Bereich von 1 bis 500 bezeichnet und o eine ganze Zahl in dem Bereich von 0 bis 500 bezeichnet, wobei n+m+o in dem Bereich von 250 bis 500 liegt, und
d) wenigstens ein Farbmittel ausgewählt aus Pigmenten, Direktfarbstoffen und Gemischen davon.

2. Verfahren nach Anspruch 1, **dadurch gekennzeichnet, dass** die (Poly)carbodiimidverbindung(en) ausgewählt sind aus den Verbindungen der folgenden Formel (I): 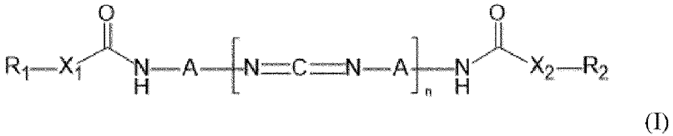 wobei:
- X₁ und X₂ unabhängig ein Sauerstoffatom O, ein Schwefelatom S oder eine NH-Gruppe darstellen;
- R₁ und R₂ unabhängig eine Gruppe ausgewählt aus einem Rest auf Kohlenwasserstoffbasis, vorzugsweise Alkyl, gegebenenfalls unterbrochen durch ein oder mehrere Heteroatome, einer Gruppe ausgewählt aus Alkoxysilyl-, Hydroxysilyl-, Acetoxysilyl-, Vinylsilyl-, Acrylalkylsilyl-, Methacrylalkylsilyl-, Crotonylalkylsilyl-, Carboxyanhydridoalkylsilyl-, Carboxyalkylsilyl-, Hydroxyalkylsilyl-, Aldehydoalkylsilyl-, Mercaptoalkylsilyl-, Norbornenylsilyl-, Acylpentadienylalkylsilyl-, Maleimidoalkylsilyl-, Sulfonylalkylsilyl-, (Meth)acrylalkyl-, Crotonylalkyl-, Alkylepoxid- wie Propylepoxid- oder Butylepoxid- und Azacyclopropangruppen, und einen Rest auf Kohlenwasserstoffbasis, vorzugsweise Alkyl, gegebenenfalls unterbrochen durch ein oder mehrere Heteroatome und durch eine oder mehrere Gruppen ausgewählt aus Alkoxysilyl-, Hydroxysilyl-, Acetoxysilyl-, Vinylsilyl-, Acrylalkylsilyl-, Methacrylalkylsilyl-, Crotonylalkylsilyl-, Carboxyanhydridoalkylsilyl-, Carboxyalkylsilyl-, Hydroxyalkylsilyl-, Aldehydoalkylsilyl-, Mercaptoalkylsilyl-, Norbornenylsilyl-, Acylpentadienylalkylsilyl-, Maleimidoalkylsilyl-, Sulfonylalkylsilyl-, (Meth)acrylalkyl-, Crotonylalkyl-, Alkylepoxid- wie Propylepoxid- oder Butylepoxid- und Azacyclopropangruppen, darstellen;
- n eine ganze Zahl in dem Bereich von 1 bis 1000 bezeichnet; und
- A ein Monomer ausgewählt aus den folgenden Verbindungen ist:

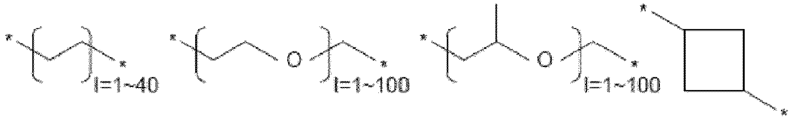

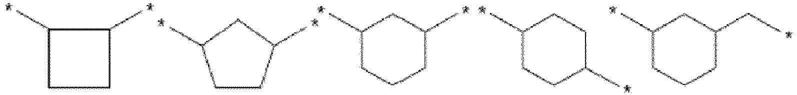

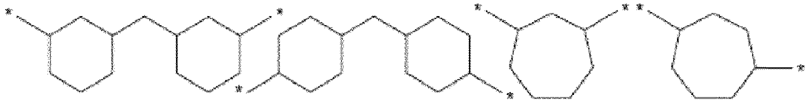

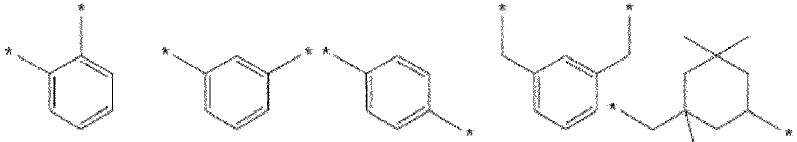

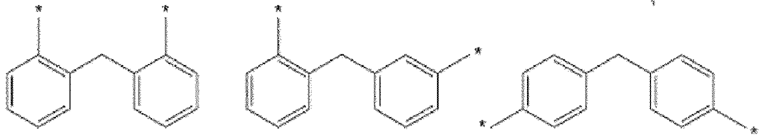

3. Verfahren nach Anspruch 1, **dadurch gekennzeichnet, dass** die (Poly)carbodiimidverbindung(en) ausgewählt sind aus den Verbindungen der folgenden Formel (II): 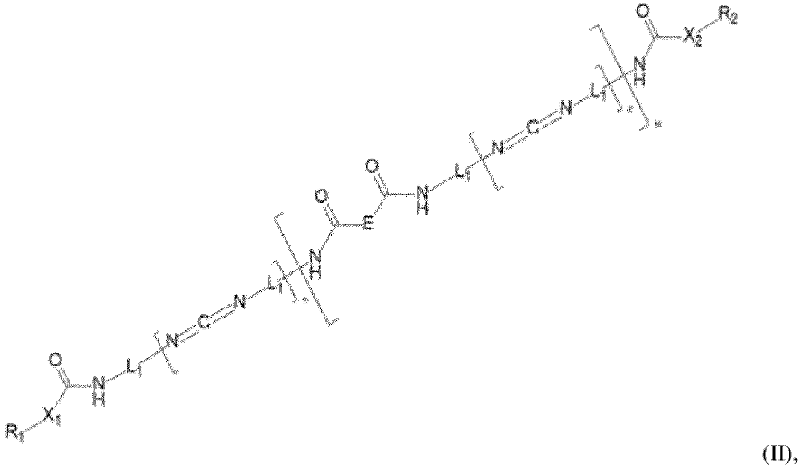 wobei:
- X₁ und X₂ unabhängig ein Sauerstoffatom 0, ein Schwefelatom S oder eine NH-Gruppe darstellen;
- R₁ und R₂ unabhängig einen Rest auf Kohlenwasserstoffbasis, gegebenenfalls unterbrochen durch ein oder mehrere Heteroatome, darstellen;
- n und z eine ganze Zahl in dem Bereich von 1 bis 20 bezeichnen, wobei n+z ≥ 2 und wobei w eine ganze Zahl in dem Bereich von 1 bis 3 bezeichnet;
- L₁ unabhängig einen zweiwertigen aliphatischen C₁-C₁₈-Rest auf Kohlenwasserstoffbasis, einen C₃-C₁₅-Cycloalkylenrest, eine C₃-C₁₂-Heterocycloalkylengruppe oder eine C₆-C₁₄-Arylengruppe und Gemische davon darstellt;
- E unabhängig eine Gruppe darstellt, die ausgewählt ist aus:
-O-R₃-O-; -S-R₄-S-; -R₅-N(R₆)-R₄-N(R₆)-R₅-;
wobei R₃ und R₄ unabhängig einen zweiwertigen Rest auf Kohlenwasserstoffbasis, gegebenenfalls unterbrochen durch ein oder mehrere Heteroatome, darstellen;
- R₅ unabhängig eine kovalente Bindung oder einen gesättigten zweiwertigen Rest auf Kohlenwasserstoffbasis, gegebenenfalls unterbrochen durch ein oder mehrere Heteroatome, darstellt;
- R₆ unabhängig ein Wasserstoffatom oder einen Rest auf Kohlenwasserstoffbasis, gegebenenfalls unterbrochen durch ein oder mehrere Heteroatome, darstellt.

4. Verfahren nach Anspruch 3, **dadurch gekennzeichnet, dass** die (Poly)carbodiimidverbindung(en) ausgewählt sind aus den Verbindungen der Formel (II), wobei:
- X₁ und X₂ unabhängig ein Sauerstoffatom darstellen;
- R₁ und R₂ unabhängig ausgewählt sind aus Dialkylaminoalkoholen, Hydroxycarbonsäurealkylestern und Monoalkylethern von (Poly)alkylenglycol, bei denen eine Hydroxygruppe entfernt worden ist, und Gemischen davon;
- n und z eine ganze Zahl in dem Bereich von 1 bis 20 bezeichnen, wobei n+z ≥ 2 und w gleich 1 ist;
- L1 ausgewählt ist aus einem zweiwertigen aliphatischen C₁-C₁₈-Rest auf Kohlenwasserstoffbasis, einem C₃-C₁₅-Cycloalkylenrest, einer C₃-C₁₂-Heterocycloalkylengruppe oder einer C₆-C₁₄-Arylengruppe und Gemischen davon;
- E unabhängig eine Gruppe darstellt, die ausgewählt ist aus:
-O-R₃-O-; -S-R₄-S-; -R₅-N(R₆)-R₄-N(R₆)-R₅-;
wobei R3 und R4 unabhängig ausgewählt sind aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon;
- wenn R₅ keine kovalente Bindung ist, R₅ ausgewählt ist aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon; und
- R₆ ausgewählt ist aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon.

5. Verfahren nach Anspruch 3 oder 4, **dadurch gekennzeichnet, dass** die (Poly)carbodiimidverbindung(en) ausgewählt sind aus den Verbindungen der Formel (II), wobei:
- X₁ und X₂ unabhängig ein Sauerstoffatom darstellen;
- R₁ und R₂ unabhängig Monoalkylether von (Poly)alkylenglycol, bei denen eine Hydroxygruppe entfernt worden ist, sind;
- n und z eine ganze Zahl in dem Bereich von 1 bis 20 bezeichnen, wobei n+z ≥ 2 und w gleich 1 ist;
- L₁ ein C₃-C₁₅-Cycloalkylenrest ist;
- E unabhängig eine Gruppe darstellt, die ausgewählt ist aus:
-O-R₃-O-; -S-R₄-S-; -R₅-N(R₆)-R₄-N(R₆)-R₅-;
wobei R3 und R4 unabhängig ausgewählt sind aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon;
- wenn R₅ keine kovalente Bindung ist, R₅ ausgewählt ist aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon; und
- R₆ ausgewählt ist aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon.

6. Verfahren nach einem der Ansprüche 3 bis 5, **dadurch gekennzeichnet, dass** die (Poly)carbodiimidverbindung(en) ausgewählt sind aus den Verbindungen der Formel (II), wobei:
- X₁ und X₂ unabhängig ein Sauerstoffatom darstellen;
- R₁ und R₂ unabhängig die Verbindung der folgenden Formel (VI) darstellen:
R₁₃-[O-CH₂-C(H)(R₁₄)]_{q}- (VI),
wobei R₁₃ eine C₁-C₄-Alkylgruppe oder ein Phenyl, vorzugsweise eine C₁-C₄-Alkylgruppe, bevorzugter Methyl, darstellt, R₁₄ ein Wasserstoffatom oder eine C₁-C₄-Alkylgruppe, vorzugsweise ein Wasserstoffatom, darstellt und q eine ganze Zahl in dem Bereich von 4 bis 30 bezeichnet,
- n und z eine ganze Zahl in dem Bereich von 1 bis 20 bezeichnen, wobei n+z in dem Bereich von 4 bis 10 liegt und w gleich 1 ist;
- L₁ ein C₃-C₁₅-Cycloalkylenrest ist, wie z.B. Cyclopentylen, Cycloheptylen, Cyclohexylen und 4,4-Dicyclohexylenmethan; und
- E eine Gruppe -O-R₃-O- darstellt, wobei R₃ ausgewählt ist aus einem C₆-C₁₄-Arylenrest, einem C₃-C₁₂-Cycloalkylenrest, einem linearen oder verzweigten C₁-C₁₈-Alkylenrest, gegebenenfalls unterbrochen durch ein oder mehrere Heteroatome, und Gemischen davon.

7. Verfahren nach einem der Ansprüche 3 bis 6, **dadurch gekennzeichnet, dass** die (Poly)carbodiimidverbindung(en) ausgewählt sind aus den Verbindungen der Formel (II), wobei:
- X₁ und X₂ unabhängig ein Sauerstoffatom darstellen;
- R₁ und R₂ unabhängig die Verbindung der folgenden Formel (VI) darstellen:
R₁₃-[O-CH₂-C(H)(R₁₄)]_{q}- (VI),
wobei R₁₃ eine C₁-C₄-Alkylgruppe oder ein Phenyl, vorzugsweise eine C₁-C₄-Alkylgruppe, bevorzugter Methyl, darstellt, R₁₄ ein Wasserstoffatom oder eine C₁-C₄-Alkylgruppe, vorzugsweise ein Wasserstoffatom, darstellt und q eine ganze Zahl in dem Bereich von 4 bis 30 bezeichnet,
- n und z eine ganze Zahl in dem Bereich von 1 bis 20 bezeichnen, wobei mit n+z in dem Bereich von 4 bis 10 liegt und w gleich 1 ist;
- L₁ ein C₃-C₁₅-Cycloalkylenrest ist, wie z.B. Cyclopentylen, Cycloheptylen, Cyclohexylen und 4,4-Dicyclohexylenmethan, vorzugsweise 4,4-Dicyclohexylenmethan; und
- E eine Gruppe -O-R₃-O- darstellt, wobei R₃ einen linearen oder verzweigten C₁-C₁₈-Alkylenrest darstellt, wie z.B. Methylen, Propylen, Butylen oder Ethylen, gegebenenfalls unterbrochen durch ein oder mehrere Heteroatome.

8. Verfahren nach einem der Ansprüche 3 bis 7, **dadurch gekennzeichnet, dass** die (Poly)carbodiimidverbindung(en) ausgewählt sind aus den Verbindungen der folgenden Formel (XII): 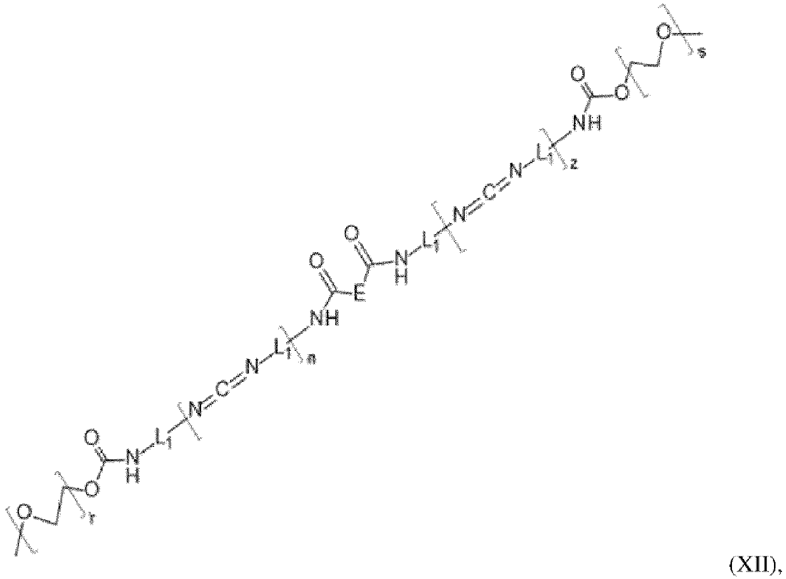 wobei L₁ 4,4-Dicyclohexylenmethan ist, n und z eine ganze Zahl in dem Bereich von 1 bis 20 mit n+z in dem Bereich von 4 bis 10 bezeichnen, E eine Gruppe -O-R₃-O- darstellt, wobei R₃ einen geradkettigen oder verzweigten C₁-C₁₈-Alkylenrest darstellt, wie z.B. Methylen, Propylen, Butylen oder Ethylen, gegebenenfalls unterbrochen durch ein oder mehrere Heteroatome, und r und s eine ganze Zahl in dem Bereich von 4 bis 30 bezeichnen.

9. Verfahren nach einem der vorstehenden Ansprüche, **dadurch gekennzeichnet, dass** die Gesamtmenge der (Poly)carbodiimidverbindung(en) in dem Bereich von 0,01 Gew.-% bis 20 Gew.-%, vorzugsweise von 0,1 Gew.-% bis 15 Gew.-%, bevorzugter von 0,2 Gew.-% bis 10 Gew.-%, noch bevorzugter von 0,5 Gew.-% bis 8 Gew.-% und noch besser von 1 Gew.-% bis 6 Gew.-%, bezogen auf das Gesamtgewicht der Zusammensetzung C, liegt.

10. Verfahren nach einem der vorstehenden Ansprüche, **dadurch gekennzeichnet, dass** die Verbindung(en), die wenigstens eine Carbonsäuregruppe enthalten, ausgewählt sind aus Siliconverbindungen, die wenigstens eine Carboxylgruppe umfassen, Polyurethanen, Acrylpolymeren und Gemischen davon, vorzugsweise aus Polyurethanen, Acrylpolymeren und Gemischen davon.

11. Verfahren nach einem der vorstehenden Ansprüche, **dadurch gekennzeichnet, dass** die Verbindung(en), die wenigstens eine Carbonsäuregruppe enthalten, in der Form von wässrigen Dispersionen von Partikeln von Polymer(en) ausgewählt aus Polyurethanen, Acrylpolymeren und Gemischen davon, vorzugsweise in der Form von wässrigen Dispersionen von Acrylpolymerpartikeln, bevorzugter in der Form von wässrigen Dispersionen von filmbildenden Acrylpolymerenpartikeln, vorliegen.

12. Verfahren nach einem der vorstehenden Ansprüche, **dadurch gekennzeichnet, dass** die Gesamtmenge der Verbindung(en), die wenigstens eine Carbonsäuregruppe enthalten, in dem Bereich von 0,1 Gew.-% bis 30 Gew.-%, bevorzugter von 0,5 Gew.-% bis 20 Gew.-%, noch besser von 0,5 Gew.-% bis 15 Gew.-% und noch bevorzugter von 1 Gew.-% bis 10 Gew.-%, bezogen auf das Gesamtgewicht von Zusammensetzung C, liegt.

13. Verfahren nach einem der vorstehenden Ansprüche, **dadurch gekennzeichnet, dass** die Aminosilicon(e) der Formel (XIII) so sind, dass:
- R1 unabhängig ein Wasserstoffatom oder eine Alkylgruppe mit von 1 bis 10 Kohlenstoffatomen, vorzugsweise von 1 bis 4 Kohlenstoffatomen, darstellt;
- R2 unabhängig einen einwertigen Rest der Formel -C_{q}H_{2q}L darstellt, wobei q eine Zahl in dem Bereich von 2 bis 8 ist und L eine gegebenenfalls quaternisierte Amingruppe ausgewählt aus den folgenden Gruppen ist:
-N(R3)₂;
-N(R3)-CH₂-CH₂-N(R3)₂;
wobei R3, das gleich oder verschieden sein kann, ein Wasserstoffatom, eine Phenylgruppe, eine Benzylgruppe oder einen gesättigten einwertigen Rest auf Kohlenwasserstoffbasis, beispielsweise eine C₁-C₂₀-Alkylgruppe, darstellt;
- X einen Rest der folgenden Formel (XIV) darstellt: 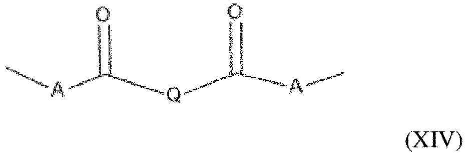 wobei:
- A unabhängig eine lineare oder verzweigte Alkylengruppe mit von 1 bis 6 Kohlenstoffatomen darstellt, die von wenigstens einem Heteroatom, ausgewählt aus O, S, NH oder einer Carbonylgruppe (CO), vorzugsweise NH, unterbrochen ist; und
- Q eine Alkylengruppe mit von 1 bis 10 Kohlenstoffatomen darstellt, die gegebenenfalls mit wenigstens einer Alkoxygruppe mit von 1 bis 10 Kohlenstoffatomen, einer Hydroxygruppe oder einer Aminoalkylgruppe mit von 1 bis 20 Kohlenstoffatomen substituiert ist, und
- n eine ganze Zahl in dem Bereich von 0 bis 500 bezeichnet, m eine ganze Zahl in dem Bereich von 1 bis 500 bezeichnet und o eine ganze Zahl in dem Bereich von 0 bis 500 bezeichnet, wobei n+m+o in dem Bereich von 250 bis 500 liegt.

14. Verfahren nach einem der vorstehenden Ansprüche, **dadurch gekennzeichnet, dass** die Aminosilicon(e) der Formel (XIII) so sind, dass:
- R1 unabhängig ein Wasserstoffatom oder eine Alkylgruppe mit von 1 bis 10 Kohlenstoffatomen, vorzugsweise von 1 bis 4 Kohlenstoffatomen, darstellt;
- R2 unabhängig einen einwertigen Rest der Formel -C_{q}H_{2q}L darstellt, wobei q eine Zahl in dem Bereich von 2 bis 8 ist und L eine Amingruppe mit der folgenden Formel ist:
-N(R3)-CH₂-CH₂-N(R3)₂;
wobei R3 ein Wasserstoffatom darstellt,
- X einen Rest der folgenden Formel (XV) darstellt: 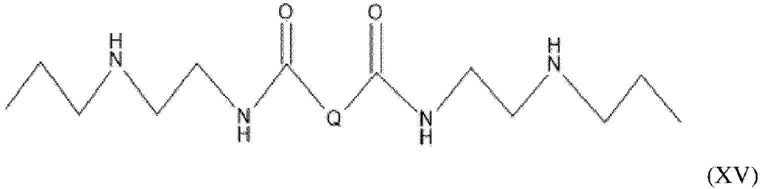 wobei:
- Q eine Alkylengruppe mit von 1 bis 10 Kohlenstoffatomen darstellt, die gegebenenfalls mit wenigstens einer Alkoxygruppe mit von 1 bis 10 Kohlenstoffatomen, einer Hydroxygruppe oder einer Aminoalkylgruppe mit von 1 bis 20 Kohlenstoffatomen substituiert ist, und
- n eine ganze Zahl in dem Bereich von 0 bis 500 bezeichnet, m eine ganze Zahl in dem Bereich von 1 bis 500 bezeichnet und o eine ganze Zahl in dem Bereich von 0 bis 500 bezeichnet, wobei n+m+o in dem Bereich von 250 bis 500 liegt.

15. Verfahren nach einem der vorstehenden Ansprüche, **dadurch gekennzeichnet, dass** das Aminosilicon der Formel (XIII) die nachstehende Formel (XVI) aufweist: 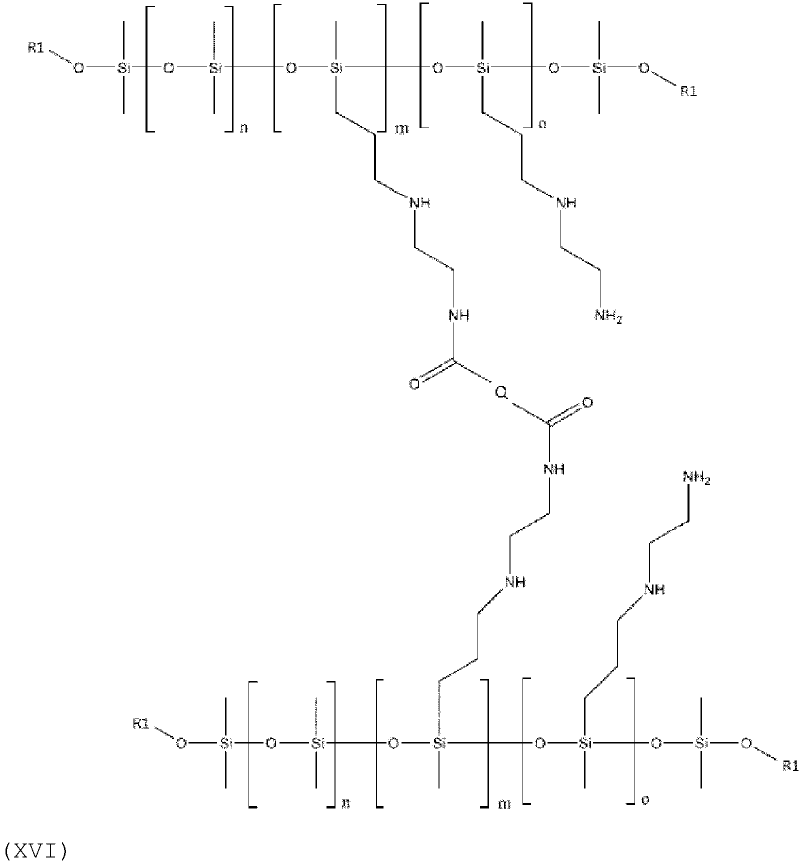 wobei:
- R1 unabhängig ein Wasserstoffatom oder eine Alkylgruppe mit von 1 bis 10 Kohlenstoffatomen, vorzugsweise von 1 bis 4 Kohlenstoffatomen, darstellt;
- Q eine Alkylengruppe mit von 1 bis 10 Kohlenstoffatomen darstellt, die gegebenenfalls mit wenigstens einer Alkoxygruppe mit von 1 bis 10 Kohlenstoffatomen, einer Hydroxygruppe oder einer Aminoalkylgruppe mit von 1 bis 20 Kohlenstoffatomen substituiert ist, und
- n eine ganze Zahl in dem Bereich von 0 bis 500 bezeichnet, m eine ganze Zahl in dem Bereich von 1 bis 500 bezeichnet und o eine ganze Zahl in dem Bereich von 0 bis 500 bezeichnet, wobei n+m+o in dem Bereich von 250 bis 500 liegt.

16. Verfahren nach einem der vorstehenden Ansprüche, **dadurch gekennzeichnet, dass** die Aminosilicon(e) der Formel (XIII) in einer Gesamtmenge in dem Bereich von 0,01 Gew.-% bis 20 Gew.-%, vorzugsweise von 0,05 Gew.-% bis 15 Gew.-%, bevorzugter von 0,1 Gew.-% bis 10 Gew.-% und noch bevorzugter von 0,25 Gew.-% bis 8 Gew.-%, bezogen auf das Gesamtgewicht der Zusammensetzung C, vorliegen.

17. Verfahren nach einem der vorstehenden Ansprüche, **dadurch gekennzeichnet, dass** die Zusammensetzung ein oder mehrere Blocksilikoncopolymere, vorzugsweise erhalten aus Dimethylvinylsiloxy-Polydimethylsiloxan (oder Divinyldimethicon), und die Verbindung der Formel (XVIII) umfasst: 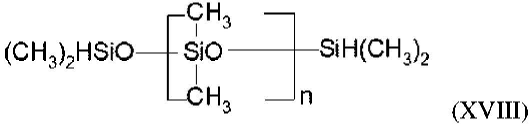 wobei n eine ganze Zahl größer als 1 und vorzugsweise größer als 10 ist, beispielsweise in dem Bereich von 10 bis 30, vorzugsweise mit n = 20, vorzugsweise in der Form einer Partikeldispersion erhalten in Gegenwart von C₁₂-C₁₃-Pareth-3 und C₁₂-C₁₃-Pareth-23 als Emulgatoren.

18. Verfahren nach Anspruch 17, **dadurch gekennzeichnet, dass** die Blocksiliconpolymer(e) in einer Gesamtmenge in dem Bereich von 0,01 Gew.-% bis 15 Gew.-%, vorzugsweise von 0,05 Gew.-% bis 10 Gew.-%, bevorzugter von 0,1 Gew.-% bis 8 Gew.-% und noch bevorzugter von 0,25 Gew.-% bis 5 Gew.-%, bezogen auf das Gesamtgewicht von Zusammensetzung C, vorliegen.

19. Verfahren nach einem der vorstehenden Ansprüche, **dadurch gekennzeichnet, dass** es ferner einen Schritt des Aufbringens einer Zusammensetzung D, die wenigstens eine Siliconverbindung umfassend wenigstens eine Carboxylgruppe umfasst, auf die Keratin-Haarfasern umfasst.

20. Verfahren nach Anspruch 19, **dadurch gekennzeichnet, dass** die Gesamtmenge der Siliconverbindung(en), die wenigstens eine Carboxylgruppe umfassen, in dem Bereich von 0,01 Gew.-% bis 20 Gew.-%, vorzugsweise von 0,1 Gew.-% bis 15 Gew.-%, bevorzugter von 0,5 Gew.-% bis 10 Gew.-% und besser noch von 1 Gew.-% bis 5 Gew.-%, bezogen auf das Gesamtgewicht der Zusammensetzung D, liegt.

21. Verfahren nach Anspruch 19 oder 20, **dadurch gekennzeichnet, dass** die Zusammensetzung D ferner ein oder mehrere Öl(e) umfasst, vorzugsweise ausgewählt aus C₈-C₁₆-Alkanen, bevorzugter aus Isododecan, Isohexadecan, Tetradecan und/oder Gemischen davon.

22. Verfahren nach einem der Ansprüche 1 bis 18, **dadurch gekennzeichnet, dass** es aus unvorbereitetem Mischen zum Zeitpunkt der Verwendung von wenigstens zwei Zusammensetzungen A und B, um eine Zusammensetzung C zu erhalten, und Aufbringen der Zusammensetzung C auf die Keratin-Haarfasern besteht, wobei:
- Zusammensetzung A wenigstens eine (Poly)carbodiimidverbindung nach einem der Ansprüche 1 bis 8 umfasst;
- Zusammensetzung B wenigstens eine Verbindung umfasst, die wenigstens eine Carbonsäuregruppe nach einem der Ansprüche 1, 10 und 11 enthält,
Zusammensetzung A und/oder Zusammensetzung B wenigstens ein Aminosilicon der Formel (XIII) nach einem der Ansprüche 1 und 13 bis 15 umfassen;
Zusammensetzung A und/oder Zusammensetzung B wenigstens ein Farbmittel ausgewählt aus Pigmenten, Direktfarbstoffen und Gemischen davon umfassen.

23. Vorrichtung zum Färben der Keratin-Haarfasern, umfassend mehrere Kompartimente, die enthalten:
- in einem ersten Kompartiment eine Zusammensetzung A umfassend:
a) wenigstens eine (Poly)carbodiimidverbindung nach einem der Ansprüche 1 bis 8,
- in einem zweiten Kompartiment eine Zusammensetzung B umfassend:
b) wenigstens eine Verbindung, die wenigstens eine Carbonsäuregruppe nach einem der Ansprüche 1, 10 und 11 enthält,
wobei Zusammensetzung A und/oder Zusammensetzung B umfassen:
c) wenigstens ein Aminosilicon der Formel (XIII) nach einem der Ansprüche 1 und 13 bis 15; und
wobei Zusammensetzung A und/oder Zusammensetzung B umfassen:
d) wenigstens ein Farbmittel ausgewählt aus Pigmenten, Direktfarbstoffen und Gemischen davon, und
- gegebenenfalls in einem dritten Kompartiment eine Zusammensetzung D umfassend wenigstens eine Siliconverbindung, die wenigstens eine Carbonsäuregruppe nach einem der Ansprüche 19 bis 21 umfasst.

## Revendications

1. Procédé de coloration des cheveux comprenant l'application sur les cheveux d'au moins une composition C comprenant :
a) au moins un composé (poly)carbodiimide ;
b) au moins un composé ayant au moins un groupement acide carboxylique,
c) au moins une silicone aminée de formule (XIII) suivante : 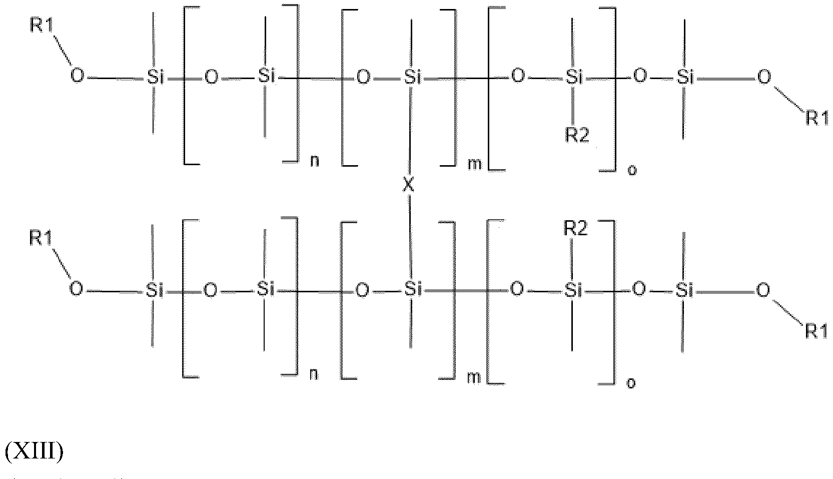 dans laquelle :
- **R1** représente indépendamment un atome d'hydrogène ou un groupe alkyle ayant de 1 à 10 atomes de carbone, de préférence de 1 à 4 atomes de carbone ;
- **R2** représente indépendamment un radical monovalent de formule -C_{q}H_{2q}L dans laquelle q est un nombre allant de 2 à 8, et L est un groupement aminé éventuellement quaternisé choisi parmi les groupements :
-N(R3)₂ ; -N⁺(R3)₃A⁻ ; -NR3-Y-N(R3)₂ et -NR-Y-N⁺(R3)₃A⁻,
dans lesquels **R3**, identique ou différent, représente un atome d'hydrogène, un groupement phényle, un groupement benzyle, ou un radical hydrocarboné saturé monovalent, par exemple un groupement alkyle en C₁-C₂₀; **Y** désigne un groupement de formule CᵣH₂ᵣ, linéaire ou ramifié, r étant un entier allant de 2 à 6, de préférence de 2 à 4; et **A**⁻ représente un anion cosmétiquement acceptable, notamment halogénure tel que fluorure, chlorure, bromure ou iodure ;
- **X** représente un radical répondant à la formule (XIV) suivante : 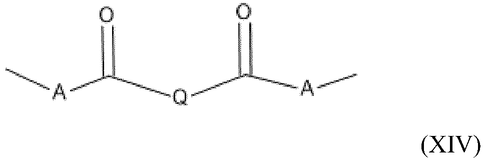 dans laquelle :
- **A** représente indépendamment un groupe alkylène ayant de 1 à 10 atomes de carbone linéaire ou ramifié, pouvant être interrompu par au moins un hétéroatome choisi parmi O, S, NH ou un groupement carbonyle (CO), de préférence NH, et
- **Q** représente un groupe alkylène ayant de 1 à 20 atomes de carbone, ledit groupe alkylène étant éventuellement substitué par au moins un groupe alcoxy ayant de 1 à 10 atomes de carbone, un groupement hydroxy ou un groupement aminoalkyle ayant de 1 à 20 atomes de carbone, et
- **n** désigne un nombre entier allant de 0 à 500, **m** désigne un nombre entier allant de 1 à 500 et **o** désigne un nombre entier allant de 0 à 500 avec n+m+o variant de 250 à 500., et d) au moins un agent colorant choisi parmi les pigments, les colorants directs, et leurs mélanges.

2. Procédé selon la revendication 1, **caractérisée en ce que** le ou les composé(s) (poly)carbodiimide est (sont) choisi(s) parmi les composés de formule (I) suivante : 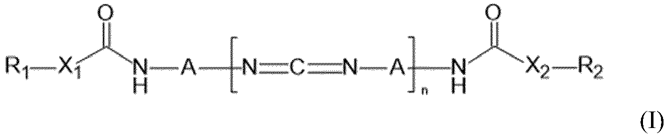 dans laquelle :
- X₁ et X₂ représentent, indépendamment, un atome d'oxygène O, un atome de soufre S ou un groupement NH ;
- R₁ et R₂ représentent indépendamment un groupement choisi parmi un radical hydrocarboné, de préférence alkyle, éventuellement interrompu par un ou plusieurs hétéroatome(s), un groupement choisi parmi les groupements alkoxysilyle, hydroxysilyle, acétoxysilyle, vinylsilyle, acrylalkylsilyle, méthacrylalkylsilyle, crotonylalkylsilyle, carboxyanhydridoalkylsilyle, carboxyalkylsilyle, hydroxyalkylsilyle, aldéhydoalkylsilyle, mercaptoalkylsilyle, norbornénylsilyle, acylpentadiénylalkylsilyle, maléimidoalkylsilyle, sulphonylalkylsilyle, (méth)acrylalkyle, crotonylalkyle, alkylépoxyde tel que propylépoxyde ou butylépoxyde, et azacyclopropane, et un radical hydrocarboné, de préférence alkyle, éventuellement interrompu par un ou plusieurs hétéroatome(s) et par un ou plusieurs groupements choisis parmi les groupements alkoxysilyle, hydroxysilyle, acétoxysilyle, vinylsilyle, acrylalkylsilyle, méthacrylalkylsilyle, crotonylalkylsilyle, carboxyanhydridoalkylsilyle, carboxyalkylsilyle, hydroxyalkylsilyle, aldéhydoalkylsilyle, mercaptoalkylsilyle, norbornénylsilyle, acylpentadiénylalkylsilyle, maléimidoalkylsilyle, sulphonylalkylsilyle, (méth)acrylalkyle, crotonylalkyle, alkylépoxyde tels que propylépoxyde ou butylépoxyde, et azacyclopropane ;
- n désigne un nombre entier allant de 1 à 1000 ; et
- A est un monomère choisi parmi les composés ci-dessous :

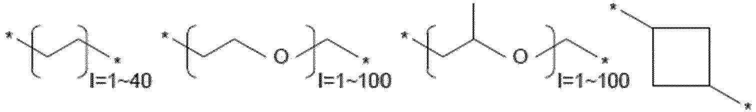

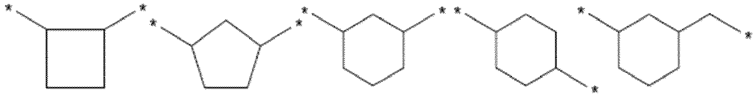

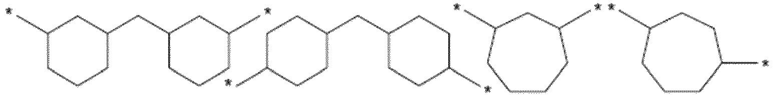

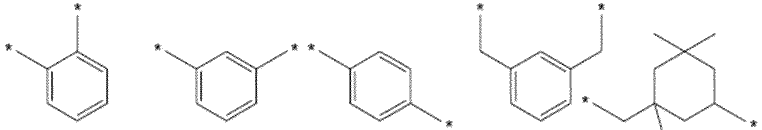

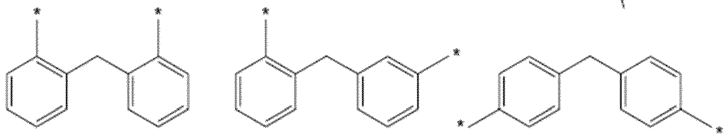

3. Procédé selon la revendication 1, **caractérisé en ce que** le ou les composé(s) (poly)carbodiimide est (sont) choisi(s) parmi les composés de formule (II) suivante : 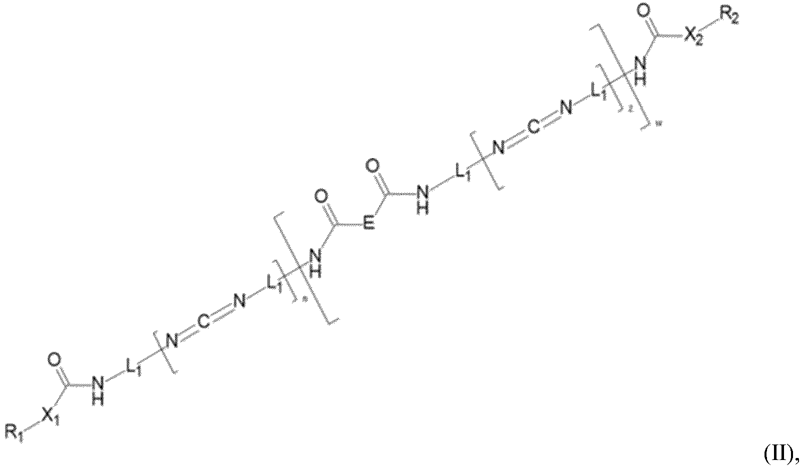 dans laquelle
- X₁ et X₂ représentent, indépendamment, un atome d'oxygène O, un atome de soufre S ou un groupement NH ;
- R₁ et R₂ représentent indépendamment un radical hydrocarboné éventuellement interrompu par un ou plusieurs hétéroatome(s) ;
- n et z désignent un nombre entier allant de 1 à 20, avec n+z ≥ 2 et w désigne un nombre entier allant de 1 à 3 ;
- L₁ représente indépendamment un radical hydrocarboné aliphatique divalent en C₁-C₁₈, un radical cycloalkylène en C₃-C₁₅, un groupement hétérocycloalkylène en C₃-C₁₂, ou un groupement arylène en C₆-C₁₄, et leurs mélanges ;
- E représente indépendamment un groupement choisi parmi :
-O-R₃-O-; -S-R₄-S-; -R₅-N(R₆)-R₄-N(R₆)-R₅-,
dans lequel R₃ et R₄ représentent indépendamment un radical hydrocarboné divalent éventuellement interrompu par un ou plusieurs hétéroatome(s) ;
- R₅ représente indépendamment une liaison covalente ou un radical hydrocarboné saturé divalent, éventuellement interrompu par un ou plusieurs hétéroatome(s) ;
- R₆ représente indépendamment un atome d'hydrogène, ou un radical hydrocarboné éventuellement interrompu par un ou plusieurs hétéroatome(s).

4. Procédé selon la revendication 3, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, sont choisis indépendamment parmi les dialkylaminoalcools, les esters alkyliques d'acide hydroxycarboxylique et les éthers monoalkyliques de (poly)alkylèneglycol, dans lesquels un groupement hydroxy a été retiré, et leurs mélanges;
- n et z désignent un nombre entier allant de 1 à 20, avec n+z ≥ 2 et w est égal à 1 ;
- L1 est choisi parmi un radical hydrocarboné aliphatique divalent en C₁-C₁₈, un radical cycloalkylène en C₃-C₁₅, un groupement hétérocycloalkylène en C₃-C₁₂, ou un groupement arylène en C₆-C₁₄, et leurs mélanges ;
- E représente indépendamment un groupement choisi parmi :
- -O-R₃-O-; -S-R₄-S-; -R₅-N(R₆)-R₄-N(R₆)-R₅-;
dans lequel R3 et R4 sont choisis indépendamment parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ;
- lorsque R₅ n'est pas une liaison covalente, R₅ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ; et
- R₆ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges.

5. Procédé selon la revendication 3 ou 4, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, sont indépendamment des éthers monoalkyliques de (poly)alkylèneglycol, dans lesquels un groupement hydroxy a été retiré ;
- n et z désignent un nombre entier allant de 1 à 20, avec n+z ≥ 2 et w est égal à 1 ;
- L₁ est un radical cycloalkylène en C₃-C₁₅ ;
- E représente indépendamment un groupement choisi parmi :
-O-R₃-O-; -S-R₄-S-; -R₅-N(R₆)-R₄-N(R₆)-R₅- ;
dans lequel R₃ et R₄ sont choisis indépendamment parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ;
- lorsque R₅ n'est pas une liaison covalente, R₅ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ; et
- R₆ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges.

6. Procédé selon l'une quelconque des revendications 3 à 5, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, représentent indépendamment le composé de formule (VI) suivante :
R₁₃-[O-CH₂-C(H)(R₁₄)]_{q}- (VI),
dans laquelle R₁₃ représente un groupe alkyle en C₁-C₄ ou un phényle, de préférence un groupe alkyle en C₁-C₄, plus préférentiellement un méthyle, R₁₄ représente un atome d'hydrogène ou un groupe alkyl en C₁-C₄, de préférence un atome d'hydrogène et q désigne un nombre entier allant de 4 à 30 ;
- n et z désignent un nombre entier allant de 1 à 20, avec n+z allant de 4 à 10 et w est égal à 1 ;
- L₁ est un radical cycloalkylène en C₃-C₁₅ tel que cyclopentylène, cycloheptylène, cyclohexylène et le 4,4-dicyclohexylène méthane, et
- E représente un groupement -O-R₃-O- dans lequel R₃ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges.

7. Procédé selon l'une quelconque des revendications 3 à 6, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, représentent indépendamment le composé de formule (VI) suivante :
R₁₃-[O-CH₂-C(H)(R₁₄)]_{q}- (VI),
dans laquelle R₁₃ représente un groupe alkyle en C₁-C₄ ou un phényle, de préférence un groupe alkyle en C₁-C₄, plus préférentiellement un méthyle, R₁₄ représente un atome d'hydrogène ou un groupe alkyle en C₁-C₄, de préférence un atome d'hydrogène et q désigne un nombre entier allant de 4 à 30 ;
- n et z désignent un nombre entier allant de 1 à 20, avec n+z allant de 4 à 10, et w est égal à 1 ;
- L₁ est un radical cycloalkylène en C₃-C₁₅ tel que le cyclopentylène, le cycloheptylène, le cyclohexylène et le 4,4-dicyclohexylène méthane, de préférence le 4,4-dicyclohexylène méthane ; et
- E représente un groupement- -O-R₃-O- dans lequel R₃ représente un radical alkylène linéaire ou ramifié en C₁-C₁₈ tel que le méthylène, propylène, butylène, éthylène, éventuellement interrompu par un ou plusieurs hétéroatome(s).

8. Procédé selon l'une quelconque des revendications 3 à 7, **caractérisé en ce que** le ou les composé(s) (poly)carbodiimide est(sont) choisi(s) parmi les composés de formule (XII) suivante : 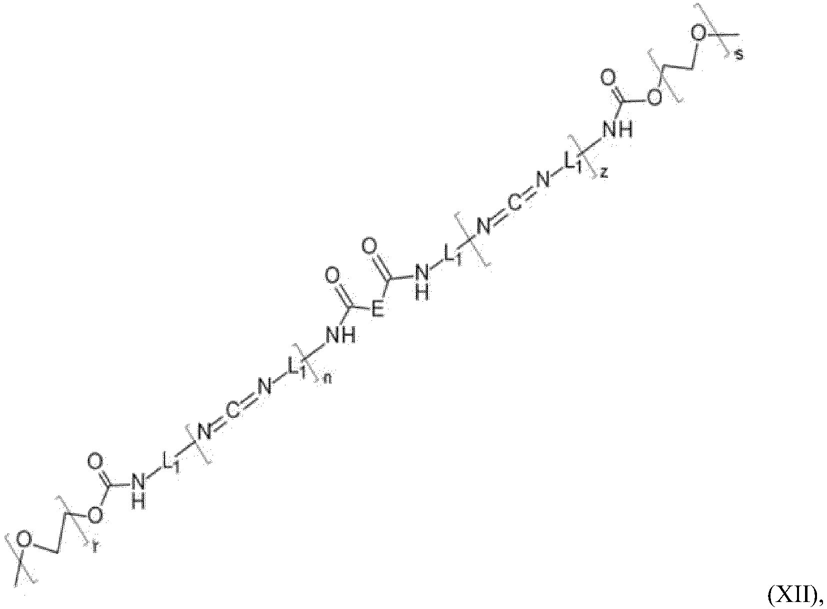 dans laquelle L₁ est le 4,4-dicyclohexylène méthane, n et z désignent un nombre entier allant de 1 à 20, avec n+z allant de 4 à 10, E représente un groupement- -O-R₃-O- dans lequel R₃ représente un radical alkylène linéaire ou ramifié en C₁-C₁₈ tel que méthylène, propylène, butylène éthylène, éventuellement interrompu par un ou plusieurs hétéroatome(s), et r et s désignent un nombre entier allant de 4 à 30.

9. Procédé selon l'une quelconque des revendications précédentes, **caractérisé en ce que** la quantité totale du ou des composé(s) (poly)carbodiimide va de 0,01 à 20% en poids, de préférence de 0,1 à 15% en poids, plus préférentiellement de 0,2 à 10% en poids, encore plus préférentiellement de 0,5 à 8% en poids, mieux de 1 à 6% en poids par rapport au poids total de la composition C.

10. Procédé selon l'une quelconque des revendications précédentes, **caractérisé en ce que** le ou les composés ayant au moins un groupement acide carboxylique sont choisis parmi les composés siliconés comprenant au moins un groupement carboxylique, les polyuréthanes, les polymères acryliques et leurs mélanges, de préférence parmi les polyuréthanes, les polymères acryliques et leurs mélanges.

11. Procédé selon l'une quelconque des revendications précédentes, **caractérisé en ce que** le ou les composés ayant au moins un groupement acide carboxylique sont sous forme de dispersions aqueuses de particules de polymère(s) choisis parmi les polyuréthanes, les polymères acryliques et leurs mélanges, de préférence sous forme de dispersions aqueuses de particules de polymères acryliques, plus préférentiellement sous forme de dispersions aqueuses de particules de polymères acryliques filmogènes.

12. Procédé selon l'une quelconque des revendications précédentes, **caractérisé en ce que** la quantité totale du ou des composés ayant au moins un groupement acide carboxylique va de 0,1 à 30% en poids, plus préférentiellement de 0,5 à 20% en poids, mieux encore de 0,5 à 15% en poids, et encore plus préférentiellement de 1 à 10% en poids par rapport au poids total de la composition C.

13. Procédé l'une quelconque des revendications précédentes, **caractérisé en ce que** la ou les silicones aminées de formule (XIII) sont telles que :
- **R1** représente indépendamment un atome d'hydrogène ou un groupe alkyle ayant de 1 à 10 atomes de carbone, de préférence de 1 à 4 atomes de carbone
- **R2** représente indépendamment un radical monovalent de formule -C_{q}H_{2q}L dans laquelle q est un nombre allant de 2 à 8, et L est un groupement aminé éventuellement quaternisé choisi parmi les groupements :
-N(R3)₂ ;
- N(R3)-CH₂-CH₂-N(R3)₂ ;
dans lesquels **R3**, identique ou différent, représente un atome d'hydrogène, un groupement phényle, un groupement benzyle, ou un radical hydrocarboné saturé monovalent, par exemple un groupement alkyle en C₁-C₂₀;
- **X** représente un radical répondant à la formule (XIV) suivante 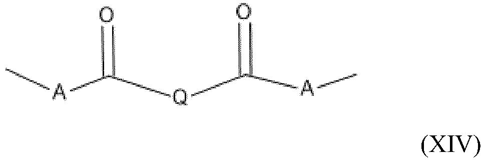 dans laquelle :
- **A** représente indépendamment un groupe alkylène ayant de 1 à 6 atomes de carbone linéaire ou ramifié, étant interrompu par au moins un hétéroatome choisi parmi O, S, NH ou un groupement carbonyle (CO), de préférence NH, et
- **Q** représente un groupe alkylène ayant de 1 à 10 atomes de carbone, ledit groupe alkylène étant éventuellement substitué par au moins un groupe alcoxy ayant de 1 à 10 atomes de carbone, un groupement hydroxy ou un groupement aminoalkyle ayant de 1 à 20 atomes de carbone, et
- **n** désigne un nombre entier allant de 0 à 500, **m** désigne un nombre entier allant de 1 à 500 et **o** désigne un nombre entier allant de 0 à 500 avec n+m+o variant de 250 à 500.

14. Procédé selon l'une quelconque des revendications précédentes, **caractérisé en ce que** la ou les silicones aminées de formule (XIII) sont telles que :
- **R1** représente indépendamment un atome d'hydrogène ou un groupe alkyle ayant de 1 à 10 atomes de carbone, de préférence de 1 à 4 atomes de carbone;
- **R2** représente indépendamment un radical monovalent de formule -C_{q}H_{2q}L dans laquelle q est un nombre allant de 2 à 8, et L est un groupement aminé de formule suivante : - N(R3)-CH₂-CH₂-N(R3)₂ ;
dans laquelle **R3**, représente un atome d'hydrogène,
- **X** représente un radical répondant à la formule (XV) suivante 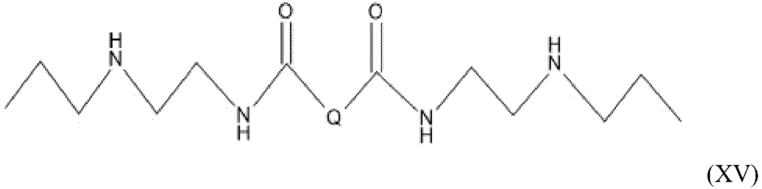 dans laquelle :
- **Q** représente un groupe alkylène ayant de 1 à 10 atomes de carbone, ledit groupe alkylène étant éventuellement substitué par au moins un groupe alcoxy ayant de 1 à 10 atomes de carbone, un groupement hydroxy ou un groupement aminoalkyle ayant de 1 à 20 atomes de carbone, et,
- **n** désigne un nombre entier allant de 0 à 500, **m** désigne un nombre entier allant de 1 à 500 et **o** désigne un nombre entier allant de 0 à 500 avec n+m+o variant de 250 à 500.

15. Procédé selon l'une quelconque des revendications précédentes, **caractérisé en ce que** la silicones aminée de formule (XIII) est de formule (XVI) suivante : 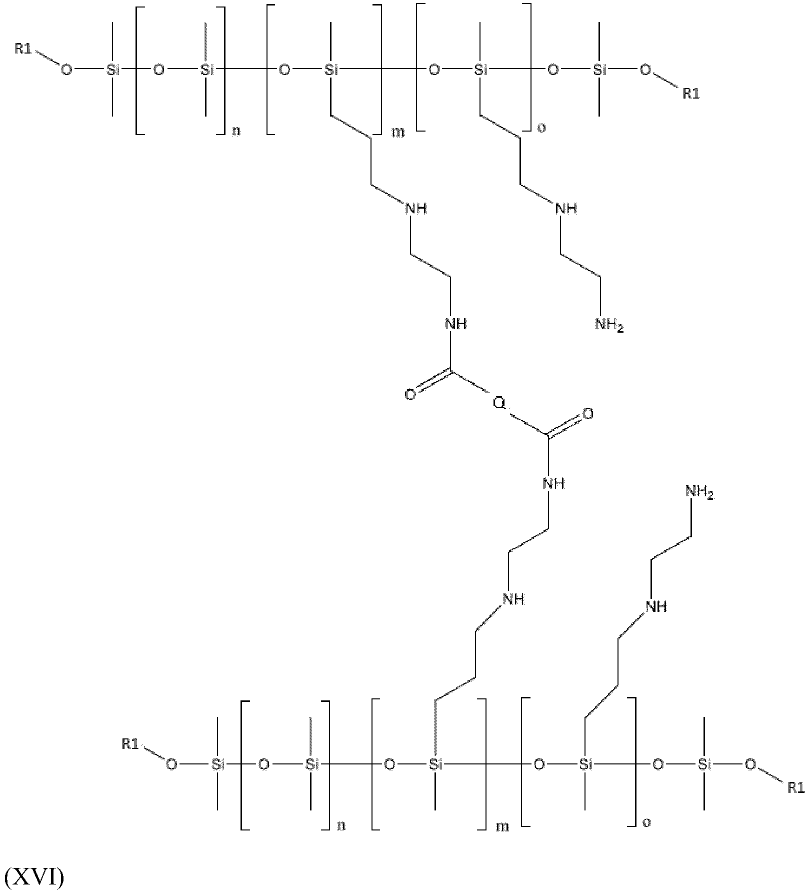 dans laquelle :
- **R1** représente indépendamment un atome d'hydrogène ou un groupe alkyle ayant de 1 à 10 atomes de carbone, de préférence de 1 à 4 atomes de carbone ;
- **Q** représente un groupe alkylène ayant de 1 à 10 atomes de carbone, ledit groupe alkylène étant éventuellement substitué par au moins un groupe alcoxy ayant de 1 à 10 atomes de carbone, un groupement hydroxy ou un groupement aminoalkyle ayant de 1 à 20 atomes de carbone, et,
- **n** désigne un nombre entier allant de 0 à 500, **m** désigne un nombre entier allant de 1 à 500 et **o** désigne un nombre entier allant de 0 à 500 avec n+m+o variant de 250 à 500.

16. Procédé selon l'une quelconque des revendications précédentes, **caractérisé en ce que** la ou (les) silicone(s) aminées(s) de formule (XIII) est (sont) présente(s) en une quantité totale variant de 0,01% à 20%, de préférence de 0,05 à 15%, plus préférentiellement de 0,1 à 10%, plus préférentiellement encore de 0,25 à 8 % en poids par rapport au poids total de la composition C.

17. Procédé selon l'une quelconque des revendications précédentes, **caractérisé en ce que** la composition comprend un ou plusieurs copolymères siliconés blocs, de préférence obtenus à partir de diméthylvinyl-siloxy-polydiméthylsiloxane (ou divinyldimethicone, et du composé de formule (XVIII) : 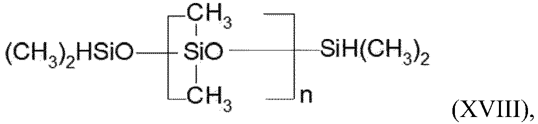 où n est un nombre entier supérieur à 1 et de préférence supérieur à 10, et par exemple allant de 10 à 30, avec de préférence n=20, de préférence sous forme de dispersion de particules obtenue en présence de C₁₂-C₁₃ Pareth-3 et C₁₂-C₁₃ Pareth-23 comme émulsifiants.

18. Procédé selon la revendication 17, **caractérisé en ce que** le ou (les) copolymères siliconés blocs est(sont) présente(s) en une quantité totale variant de 0,01% à 15%, de préférence de 0,05 à 10%, plus préférentiellement de 0,1 à 8%, plus préférentiellement encore de 0,25 à 5 % en poids par rapport au poids total de la composition C.

19. Procédé selon l'une quelconque des revendications précédentes, **caractérisé en ce qu'**il comprend en outre une étape d'application sur les cheveux d'une composition D comprenant au moins un composé siliconé comprenant au moins un groupement carboxylique.

20. Procédé selon la revendication 19, **caractérisé en ce que** la quantité totale en composé(s) siliconé(s) comprenant au moins un groupement carboxylique va de 0,01 à 20% en poids, de préférence de 0,1 à 15% en poids, plus préférentiellement de 0,5 à 10% en poids, mieux de 1 à 5% en poids par rapport au poids total de la composition D.

21. Procédé selon la revendication 19 ou 20, **caractérisé en ce que** la composition D comprend en outre une ou plusieurs huile(s), de préférence choisie(s) parmi les alcanes en C₈-C₁₆, plus préférentiellement parmi l'isododécane, l'isohexadécane, le tétradécane et/ou leurs mélanges.

22. Procédé selon l'une quelconque des revendications 1 à 18, **caractérisé en ce qu'**il consiste à mélanger extemporanément au moment de l'emploi au moins deux compositions A et B pour obtenir une composition C et à appliquer la composition C sur les cheveux, avec :
- la composition A comprenant au moins un composé (poly)carbodiimide tel(s) que défini(s) selon l'une quelconque des revendications 1 à 8 ;
- la composition B comprenant au moins un composé ayant au moins un groupement acide carboxylique tel(s) que défini(s) selon l'une quelconque des revendications 1, 10 à 11, la composition A et/ou la composition B comprenant au moins une silicone aminée de formule (XIII) telle(s) que définie(s) selon l'une quelconque des revendications 1, 13 à 15 ;
la composition A et/ou la composition B comprenant au moins un agent colorant choisi parmi les pigments, les colorants directs, et leurs mélanges.

23. Dispositif pour la coloration des cheveux comprenant plusieurs compartiments contenant :
- dans un premier compartiment, une composition A comprenant :
a) au moins un composé (poly)carbodiimide tel(s) que défini(s) selon l'une quelconque des revendications 1 à 8,
- dans un deuxième compartiment, une composition B comprenant :
b) au moins un composé ayant au moins un groupement acide carboxylique tel(s) que défini(s) selon l'une quelconque des revendications 1, 10 à 11,
la composition A et/ou la composition B comprenant :
c) au moins une silicone aminée de formule (XIII) telle(s) que définie(s) selon l'une quelconque des revendications 1, 13 à 15 ; et
la composition A et/ou la composition B comprenant :
d) au moins un agent colorant choisi parmi les pigments, les colorants directs, et leurs mélanges, et
- optionnellement dans un troisième compartiment, une composition D comprenant au moins un composé siliconé comprenant au moins un groupement carboxylique tel que défini selon l'une quelconque des revendications 19 à 21.
